(19) Europäisches Patentamt
European Patent Office
Office européen des brevets

(11) EP 4 405 051 B1

(12) EUROPEAN PATENT SPECIFICATION

(45) Date of publication and mention of the grant of the patent:
**15.07.2026 Bulletin 2026/29**

(21) Application number: **22786353.7**

(22) Date of filing: **22.09.2022**

(51) International Patent Classification (IPC):
***A61P 37/00*** *(2006.01)* ***C07D 471/04*** *(2006.01)*

(52) Cooperative Patent Classification (CPC):
**C07D 471/04; A61P 37/00**

(86) International application number:
**PCT/EP2022/076306**

(87) International publication number:
**WO 2023/046806 (30.03.2023 Gazette 2023/13)**

# (54) PYRAZOLO[3,4-B]PYRIDINE COMPOUNDS FOR THE TREATMENT OF AUTOIMMUNE DISEASE

PYRAZOLO[3,4-B PYRIDIN-VERBINDUNGEN ZUR BEHANDLUNG VON AUTOIMMUNKRANKHEITEN

COMPOSÉS DE PYRAZOLO[3,4-B]PYRIDINE POUR LE TRAITEMENT D’UNE MALADIE AUTO-IMMUNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **24.09.2021 PCT/CN2021/120406**
**25.04.2022 PCT/CN2022/089033**

(43) Date of publication of application:
**31.07.2024 Bulletin 2024/31**

(73) Proprietor: **F. Hoffmann-La Roche AG**
**4070 Basel (CH)**

(72) Inventors:
- **CHEN, Dongdong**
  **Shanghai 201203 (CN)**
- **DEY, Fabian**
  **4070 Basel (CH)**
- **HONG, Xin**
  **Shanghai 201203 (CN)**
- **WANG, Xiaoqing**
  **Shanghai 201203 (CN)**
- **ZHANG, Zhisen**
  **Shanghai 201203 (CN)**
- **ZHU, Wei**
  **Shanghai 201203 (CN)**
- **ZOU, Ge**
  **Shanghai 201203 (CN)**

(74) Representative: **Schirlin, Julien**
**F. Hoffmann-La Roche AG**
**CLP - Patent Department**
**Grenzacherstrasse 124**
**4070 Basel (CH)**

(56) References cited:
**WO-A1-2015/088045**
**WO-A1-2018/047081**
**WO-A1-2019/220390**
**WO-A1-2022/013136**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 405 051 B1

**Description**

The present invention relates to organic compounds useful for therapy and/or prophylaxis in a mammal, and in particular to antagonist of TLR7 and TLR8 and TLR9 useful for treating systemic lupus erythematosus or lupus nephritis.

## FIELD OF THE INVENTION

Autoimmune connective tissue disease (CTD) include prototypical autoimmune syndromes such as Systemic Lupus Erythematosus (SLE), primary Sjögren's syndrome (pSjS), mixed connective tissue disease (MCTD), Dermatomyositis/Polymyositis (DM/PM), Rheumatoid Arthritis (RA), and systemic sclerosis (SSc). With the exception of RA, no really effective and safe therapies are available to patients. SLE represents the prototypical CTD with a prevalence of 20-150 per 100,000 and causes broad inflammation and tissue damage in distinct organs, from commonly observed symptoms in the skin and joints to renal, lung, or heart failure. Traditionally, SLE has been treated with nonspecific anti-inflammatory or immunosuppressive drugs. However, long-term usage of immunosuppressive drug, e.g. corticosteroids is only partially effective, and is associated with undesirable toxicity and side effects. Belimumab is the only FDA-approved drug for lupus in the last 50 years, despite its modest and delayed efficacy in only a fraction of SLE patients (Navarra, S. V. et al Lancet 2011, 377, 721.). Other biologics, such as anti-CD20 mAbs, mAbs against or soluble receptors of specific cytokines, have failed in most clinical studies. Thus, novel therapies are required that provide sustained improvement in a greater proportion of patient groups and are safer for chronic use in many autoimmune as well as autoinflammation diseases.

Toll like Receptors (TLR) are an important family of pattern recognition receptors (PRR) which can initiate broad immune responses in a wide variety of immune cells. As natural host defense sensors, endosomal TLRs 7, 8 and 9 recognize nucleic acids derived from viruses, bacteria; specifically, TLR7/8 and TLR9 recognize single-stranded RNA (ssRNA) and single-stranded CpG-DNA, respectively. However, aberrant nucleic acid sensing of TRL7, 8, 9 is considered as a key node in a broad of autoimmune and auto-inflammatory diseases (Krieg, A. M. et al. Immunol. Rev. 2007, 220, 251. Jiménez-Dalmaroni, M. J. et al Autoimmun Rev. 2016, 15, 1. Chen, J. Q., et al. Clinical Reviews in Allergy & Immunology 2016, 50, 1.). Anti-RNA and anti-DNA antibodies are well-established diagnostic markers of SLE, and these antibodies can deliver both self-RNA and self-DNA to endosomes. While self-RNA complexes can be recognized by TLR7 and TLR8, self-DNA complexes can trigger TLR9 activation. Indeed, defective clearance of self-RNA and self-DNA from blood and/or tissues is evident in SLE (Systemic Lupus Erythematosus) patients. TLR7 and TLR9 have been reported to be upregulated in SLE tissues, and correlate with chronicity and activity of lupus nephritis, respectively. In B cells of SLE patients, TLR7 expression correlates with anti-RNP antibody production, while TLR9 expression with IL-6 and anti-dsDNA antibody levels. Consistently, in lupus mouse models, TLR7 is required for anti-RNA antibodies, and TLR9 is required for anti-nucleosome antibody. On the other hand, overexpression of TLR7 or human TLR8 in mice promotes autoimmunity and autoinflammation. Moreover, activation of TLR8 specifically contributes to inflammatory cytokine secretion of mDC/macrophages, neutrophil NETosis, induction of Th17 cells, and suppression of Treg cells. In addition to the described role of TLR9 in promoting autoantibody production of B cells, activation of TLR9 by self-DNA in pDC also leads to induction of type I IFNs and other inflammatory cytokines. Given these roles of TLR9 in both pDC and B cells, both as key contributors to the pathogenesis of autoimmune diseases, and the extensive presence of self-DNA complexes that could readily activate TLR9 in many patients with autoimmune diseases, it may have extra benefit to further block self-DNA mediated TLR9 pathways on top of inhibition of TLR7 and TLR8 pathways. Taken together, TLR7, 8 and 9 pathways represent new therapeutic targets for the treatment of autoimmune and auto-inflammatory diseases, for which no effective steroid-free and non-cytotoxic oral drugs exist, and inhibition of all these pathways from the very upstream may deliver satisfying therapeutic effects. As such, we invented oral compounds that target and suppress TLR7, TLR8 and TLR9 for the treatment of autoimmune and auto-inflammatory diseases.

## SUMMARY OF THE INVENTION

Any references to methods of treatment in the subsequent paragraphs of this description are to be interpreted as references to the compounds, pharmaceutical compositions and medicaments of the present invention for use in a method for treatment of the human (or animal) body by therapy (or for diagnosis). The present invention relates to novel compounds of formula (I),

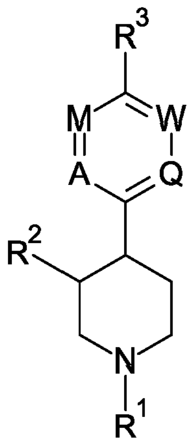

(I),

wherein

$R^1$ is

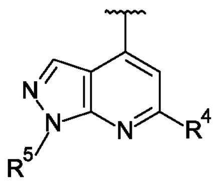

;

wherein $R^4$ is H or $C_{1-6}$alkyl; $R^5$ is $C_{1-6}$alkyl;
$R^2$ is H or $C_{1-6}$alkyl;
$R^3$ is piperazinyl, ($C_{1-6}$alkoxy$C_{1-6}$alkyl)piperazinyl, 3,4,4a,5,7,7a-hexahydro-2H-pyrrolo[3,4-b][1,4]oxazinyl, 4,7-diazaspiro[2.5]octanyl, 5-oxa-2,8-diazaspiro[3.5]nonanyl, amino-1,4-oxazepanyl, amino($C_{1-6}$alkoxy)piperidinyl, amino($C_{1-6}$alkoxy)pyrrolidinyl, amino($C_{1-6}$alkyl)azetidinyl or aminohalopiperidinyl;
A is N or $CR^6$; wherein $R^6$ is H, $C_{1-6}$alkyl or $C_{1-6}$alkoxy;
M is N or $CR^7$; wherein $R^7$ is H or $C_{1-6}$alkyl;
W is N or CH;
Q is N or CH;
with the condition that no more than two of A, M, W and Q are N simultaneously;
or a pharmaceutically acceptable salt thereof.

Another object of the present invention is related to novel compounds of formula (I) or (Ia). Their manufacture, medicaments based on a compound in accordance with the invention and their production as well as the use of compounds of formula (I) or (Ia) as TLR7 and TLR8 and TLR9 antagonist, and for the treatment or prophylaxis of systemic lupus erythematosus or lupus nephritis. The compounds of formula (I) or (Ia) show superior TLR7 and TLR8 and TLR9 antagonism activity. In addition, the compounds of formula (I) or (Ia) also show good cytotoxicity, phototoxicity, solubility, hPBMC, human microsome stability and SDPK profiles, as well as low CYP inhibition.

Novartis patent WO2018047081 disclosed compounds with same pyrazolo[3,4-b]pyridinyl moiety as the compounds of this invention, however the central bicyclic core of 4,5,6,7-tetrahydro-1H-pyrazolo[4,3-c]pyridine and the terminal substitution with bicyclo[2,2,2]octane/bicyclo[1,1,1]pentane moieties were essential for the TLR7/8/9 activity based on the information disclosed in Novartis patent, which also were considered as the major structural differentiation compared with the compounds of current invention. On the other hand, most of the compounds in WO2018047081 showed poor TLR9 activity. Unfortunately few compounds with relatively improved TLR9 activity, such as N79 (as Example 79) with best TLR9 activity, still suffer from poor human liver microsome stability (see Table 5) and therefore have unsatisfactory PK profile.

Another Novartis patent WO2019220390 disclosed the polymorph of compound N8 (as Example 8 in WO2018047081), which considered as the lead compound of its series and turned out to have much lower TLR9 activity and similar poor human liver microsome stability (see Table 5).

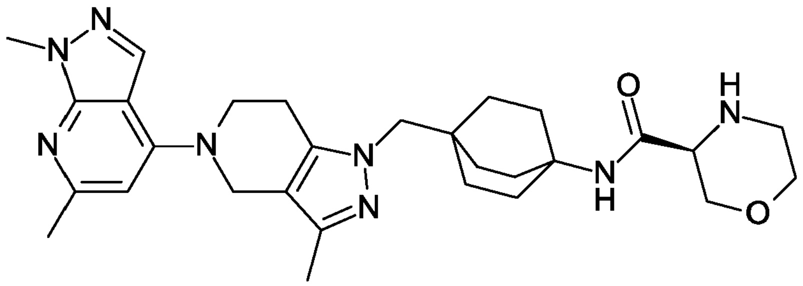

**Compound N8** (hPBMC TLR7/8/9 antagonist IFNα Assay $IC_{50}$ (μM): 0.004/0.166/4.28)

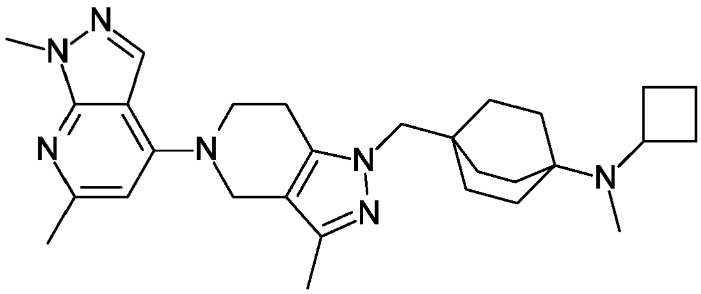

**Compound N79** (hPBMC TLR7/8/9 antagonist IFNα Assay $IC_{50}$ (μM): 0.004/0.136/0.064)

## DETAILED DESCRIPTION OF THE INVENTION

### DEFINITIONS

The term "$C_{1-6}$alkyl" denotes a saturated, linear or branched chain alkyl group containing 1 to 6, particularly 1 to 4 carbon atoms, for example methyl, ethyl, n-propyl, isopropyl, n-butyl, isobutyl, *tert*-butyl and the like. Particular "$C_{1-6}$alkyl" groups are methyl, ethyl and n-propyl.

The term "$C_{1-6}$alkoxy" denotes $C_{1-6}$alkyl-O-.

The term "halogen" and "halo" are used interchangeably herein and denote fluoro, chloro, bromo, or iodo.

The term "halopiperidinyl" denotes a piperidinyl group wherein at least one of the hydrogen atoms of the piperidinyl group has been replaced by same or different halogen atoms, particularly fluoro atoms. Examples of halopiperidinyl include fluoropyrrolidinyl and difluoropiperidinyl.

The term *"cis"* and *"trans"* denote the relative stereochemistry of the molecule or moiety. For example:

Example 18 as the cis-isomer, refers to a mixture of

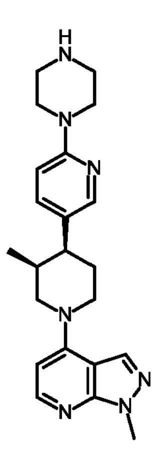

and

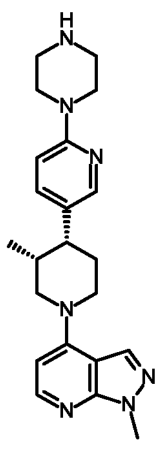

;

similarly, Example 19 as the *trans*-isomer, refers to a mixture of

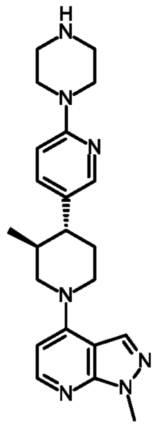

and

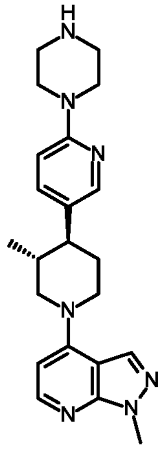

.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, *p*-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, *N*-ethylpiperidine, and polyamine resins.

The term "A pharmaceutically active metabolite" (not part of the invention) denotes a pharmacologically active product produced through metabolism in the body of a specified compound or salt thereof. After entry into the body, most drugs are substrates for chemical reactions that may change their physical properties and biologic effects. These metabolic conversions, which usually affect the polarity of the compounds of the invention, alter the way in which drugs are distributed in and excreted from the body. However, in some cases, metabolism of a drug is required for therapeutic effect.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

**ANTAGONIST OF TLR7 AND TLR8 AND TLR9**

The present invention relates to (i) a compound of formula (I),

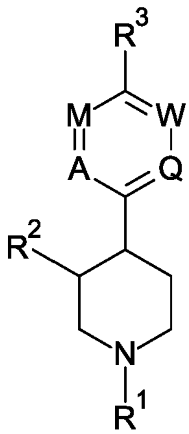

(I),

wherein

$R^1$ is

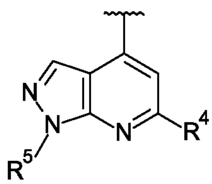

;

wherein $R^4$ is H or $C_{1-6}$alkyl; $R^5$ is $C_{1-6}$alkyl;
$R^2$ is H or $C_{1-6}$alkyl;
$R^3$ is piperazinyl, ($C_{1-6}$alkoxy$C_{1-6}$alkyl)piperazinyl, 3,4,4a,5,7,7a-hexahydro-2H-pyrrolo[3,4-b][1,4]oxazinyl, 4,7-diazaspiro[2.5]octanyl, 5-oxa-2,8-diazaspiro[3.5]nonanyl, amino-1,4-oxazepanyl, amino($C_{1-6}$alkoxy)piperidinyl, amino($C_{1-6}$alkoxy)pyrrolidinyl, amino($C_{1-6}$alkyl)azetidinyl or aminohalopiperidinyl;
A is N or $CR^6$; wherein $R^6$ is H, $C_{1-6}$alkyl or $C_{1-6}$alkoxy;
M is N or $CR^7$; wherein $R^7$ is H or $C_{1-6}$alkyl;
W is N or CH;
Q is N or CH;
with the condition that no more than two of A, M, W and Q are N simultaneously;
or a pharmaceutically acceptable salt thereof.

Another embodiment of present invention is (ii) a compound of formula (Ia),

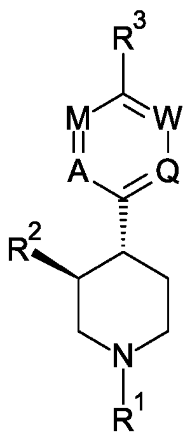

(Ia),

wherein

$R^1$ is

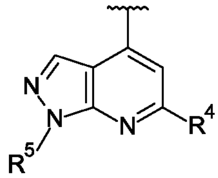

;'

wherein $R^4$ is H or $C_{1-6}$alkyl; $R^5$ is $C_{1-6}$alkyl;

$R^2$ is H or $C_{1-6}$alkyl;

$R^3$ is piperazinyl, ($C_{1-6}$alkoxy$C_{1-6}$alkyl)piperazinyl, 3,4,4a,5,7,7a-hexahydro-2H-pyrrolo[3,4-b][1,4]oxazinyl, 4,7-diazaspiro[2.5]octanyl, 5-oxa-2,8-diazaspiro[3.5]nonanyl, amino-1,4-oxazepanyl, amino($C_{1-6}$alkoxy)piperidinyl, amino($C_{1-6}$alkoxy)pyrrolidinyl, amino($C_{1-6}$alkyl)azetidinyl or aminohalopiperidinyl;

A is N or $CR^6$; wherein $R^6$ is H, $C_{1-6}$alkyl or $C_{1-6}$alkoxy;

M is N or $CR^7$; wherein $R^7$ is H or $C_{1-6}$alkyl;

W is N or CH;

Q is N or CH;

with the condition that no more than two of A, M, W and Q are N simultaneously;

or a pharmaceutically acceptable salt thereof.

A further embodiment of present invention is (iii) a compound of formula (I) or (Ia) according to (i) or (ii), or a pharmaceutically acceptable salt thereof, wherein $R^1$ is

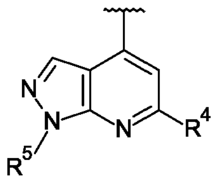

;

wherein $R^4$ is $C_{1-6}$alkyl; $R^5$ is $C_{1-6}$alkyl.

A further embodiment of present invention is (iv) a compound of formula (I) or (Ia), according to any one of (i) to (iii), or a pharmaceutically acceptable salt thereof, wherein $R^4$ is methyl; $R^5$ is methyl.

A further embodiment of present invention is (v) a compound of formula (I) or (Ia) according to any one of (i) to (iv), wherein A is $CR^6$; wherein $R^6$ is H or $C_{1-6}$alkyl.

A further embodiment of present invention is (vi) a compound of formula (I) or (Ia), or a pharmaceutically acceptable salt thereof, according to any one of (i) to (v), wherein A is $CR^6$; wherein $R^6$ is H or methyl.

A further embodiment of present invention is (vii) a compound of formula (I) or (Ia), or a pharmaceutically acceptable salt thereof, according to any one of (i) to (vi), wherein M is $CR^7$; wherein $R^7$ is H.

A further embodiment of present invention is (viii) a compound of formula (I) or (Ia), or a pharmaceutically acceptable salt thereof, according to any one of (i) to (vii), wherein W is CH.

A further embodiment of present invention is (ix) a compound of formula (I) or (Ia), or a pharmaceutically acceptable salt thereof, according to any one of (i) to (viii), wherein Q is N.

A further embodiment of present invention is (x) a compound of formula (I) or (Ia), or a pharmaceutically acceptable salt thereof, according to any one of (i) to (ix), wherein $R^3$ is amino-1,4-oxazepanyl, amino($C_{1-6}$alkoxy) pyrrolidinyl or piperazinyl.

A further embodiment of present invention is (xi) a compound of formula (I) or (Ia), or a pharmaceutically acceptable salt thereof, according to any one of (i) to (x), wherein $R^3$ is 6-amino-1,4-oxazepan-4-yl, 3-amino-4-methoxy-pyrrolidin-1-yl or piperazin-1-yl.

A further embodiment of present invention is (xii) a compound of formula (I) or (Ia), according to any one of (i) to (xi), wherein

$R^1$ is

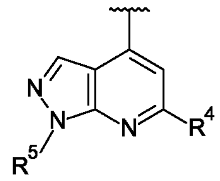

;

wherein $R^4$ is $C_{1-6}$alkyl; $R^5$ is $C_{1-6}$alkyl;
$R^2$ is H or $C_{1-6}$alkyl;
$R^3$ is amino-1,4-oxazepanyl, amino($C_{1-6}$alkoxy)pyrrolidinyl or piperazinyl;
A is $CR^6$; wherein $R^6$ is H or $C_{1-6}$alkyl;
M is $CR^7$; wherein $R^7$ is H;
W is CH;
Q is N;
or a pharmaceutically acceptable salt thereof.

A further embodiment of present invention is (xiii) a compound of formula (I) or (Ia), according to any one of (i) to (xii), wherein

$R^1$ is

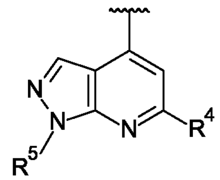

;

wherein $R^4$ is methyl; $R^5$ is methyl;
$R^2$ is H or methyl;
$R^3$ is 6-amino-1,4-oxazepan-4-yl, 3-amino-4-methoxy-pyrrolidin-1-yl or piperazin-1-yl;
A is $CR^6$; wherein $R^6$ is H or methyl;
M is $CR^7$; wherein $R^7$ is H;
W is CH;
Q is N;
or a pharmaceutically acceptable salt thereof.

Another embodiment of present invention is (xiv) a compound of formula compound of formula (Ib),

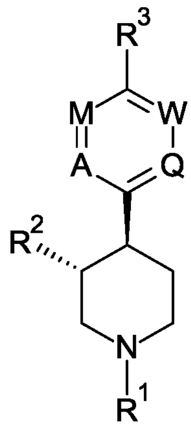

(Ib),

wherein

$R^1$ is

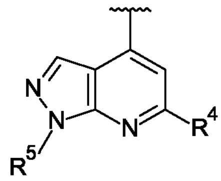

;wherein $R^4$ is $C_{1-6}$alkyl; $R^5$ is $C_{1-6}$alkyl;
$R^2$ is $C_{1-6}$alkyl;
$R^3$ is piperazinyl, ($C_{1-6}$alkoxy$C_{1-6}$alkyl)piperazinyl, 4,7-diazaspiro[2.5]octanyl, 5-oxa-2,8-diazaspiro[3.5]nonanyl, amino-1,4-oxazepanyl, amino($C_{1-6}$alkoxy)piperidinyl, amino($C_{1-6}$alkoxy)pyrrolidinyl, amino($C_{1-6}$alkyl)azetidinyl or aminohalopiperidinyl;
A is CH;
M is CH;
W is CH;
Q is N;
or a pharmaceutically acceptable salt thereof.

A further embodiment of present invention is (xv) a compound of formula (Ib), according to (xiv), wherein $R^4$ is methyl; $R^5$ is methyl; $R^2$ is methyl.

A further embodiment of present invention is (xvi) a compound of formula (Ib), according to (xiv) or (xv), wherein $R^3$ is amino-1,4-oxazepanyl.

A further embodiment of present invention is (xvii) a compound of formula (Ib), according to any one of (xiv) to (xvi), wherein $R^3$ is 6-amino-1,4-oxazepan-4-yl.

A further embodiment of present invention is (xviii) a compound of formula (Ib), according to any one of (xiv) to (xvii), wherein

$R^1$ is

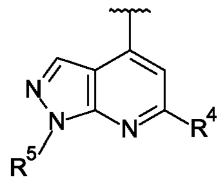

;'

wherein $R^4$ is $C_{1-6}$alkyl; $R^5$ is $C_{1-6}$alkyl;
$R^2$ is $C_{1-6}$alkyl;
$R^3$ is amino-1,4-oxazepanyl;
A is CH;
M is CH;
W is CH;
Q is N;
or a pharmaceutically acceptable salt thereof.

A further embodiment of present invention is (xix) a compound of formula (Ib), according to any one of (xiv) to (xviii), wherein

$R^1$ is

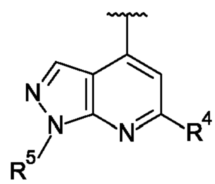

;

wherein $R^4$ is methyl; $R^5$ is methyl;

$R^2$ is methyl;
$R^3$ is 6-amino-1,4-oxazepan-4-yl;
A is CH;
M is CH;
W is CH;
Q is N;
or a pharmaceutically acceptable salt thereof.

Another embodiment of present invention is a compound of formula (I) or (Ia) or (Ib) selected from the following:

1,6-dimethyl-4-[4-(4-piperazin-1-ylphenyl)-1-piperidyl]pyrazolo[3,4-b]pyridine;
1,6-dimethyl-4-[4-(5-piperazin-1-yl-2-pyridyl)-1-piperidyl]pyrazolo[3,4-b]pyridine;
1,6-dimethyl-4-[4-(5-piperazin-1-ylpyrazin-2-yl)-1-piperidyl]pyrazolo[3,4-b]pyridine;
1,6-dimethyl-4-[4-(5-piperazin- 1-ylpyrimidin-2-yl)-1-piperidyl]pyrazolo[3,4-b]pyridine;
1-methyl-4-[4-(3-methyl-5-piperazin-1-yl-2-pyridyl)-1-piperidyl]pyrazolo[3,4-b]pyridine;
1-methyl-4-[4-(4-methyl-6-piperazin-1-yl-3-pyridyl)-1-piperidyl]pyrazolo[3,4-b]pyridine;
1,6-dimethyl-4-[4-(3-methyl-5-piperazin-1-yl-2-pyridyl)-1-piperidyl]pyrazolo[3,4-b]pyridine;
1,6-dimethyl-4-[4-(4-methyl-6-piperazin-1-yl-3-pyridyl)-1-piperidyl]pyrazolo[3,4-b]pyridine;
1,6-dimethyl-4-[4-(4-methyl-5-piperazin-1-yl-2-pyridyl)-1-piperidyl]pyrazolo[3,4-b]pyridine;
1,6-dimethyl-4-[4-(2-methyl-6-piperazin-1-yl-3-pyridyl)-1-piperidyl]pyrazolo[3,4-b]pyridine;
1,6-dimethyl-4-[4-(4-methyl-2-piperazin-1-yl-pyrimidin-5-yl)- 1-piperidyl]pyrazolo[3,4-b]pyridine;
1,6-dimethyl-4-[4-(3-methyl-5-piperazin-1-yl-pyrazin-2-yl)-1-piperidyl]pyrazolo[3,4-b]pyridine;
4-[4-(3-ethyl-5-piperazin-1-yl-2-pyridyl)-1-piperidyl]-1,6-dimethyl-pyrazolo[3,4-b]pyridine;
4-[4-(3-methoxy-5-piperazin-1-yl-2-pyridyl)-1-piperidyl]-1,6-dimethyl-pyrazolo[3,4-b]pyridine;
(3*S*,4*R*)-1-[6-[1-(1,6-dimethylpyrazolo[3,4-b]pyridin-4-yl)-4-piperidyl]-5-methyl-3-pyridyl]-3-fluoro-piperidin-4-amine;
4-[4-[5-[(3*R*)-3-(methoxymethyl)piperazin-1-yl]-3-methyl-2-pyridyl]-1-piperidyl]-1,6-dimethyl-pyrazolo[3,4-b]pyridine;
(3*S*,4*S*)-1-[6-[1-(1,6-dimethylpyrazolo[3,4-b]pyridin-4-yl)-4-piperidyl]-5-methyl-3-pyridyl]-3-methoxy-piperidin-4-amine;
1-methyl-4-[*cis*-3-methyl-4-(6-piperazin-1-yl-3-pyridyl)-1-piperidyl]pyrazolo[3,4-b]pyridine;
1-methyl-4-[*trans*-3-methyl-4-(6-piperazin-1-yl-3-pyridyl)-1-piperidyl]pyrazolo[3,4-b]pyridine;
1,6-dimethyl-4-[*cis*-3-methyl-4-(4-methyl-6-piperazin-1-yl-3-pyridyl)-1-piperidyl]pyrazolo[3,4-b]pyridine;
1,6-dimethyl-4-[*trans*-3-methyl-4-(4-methyl-6-piperazin-1-yl-3-pyridyl)-1-piperidyl]pyrazolo[3,4-b]pyridine;
1,6-dimethyl-4-[*cis*-3-methyl-4-(3-methyl-5-piperazin-1-yl-pyrazin-2-yl)-1-piperidyl]pyrazolo[3,4-b]pyridine;
1,6-dimethyl-4-[*trans*-3-methyl-4-(3-methyl-5-piperazin-1-yl-pyrazin-2-yl)-1-piperidyl]pyrazolo[3,4-b]pyridine;
1,6-dimethyl-4-[*cis*-3-methyl-4-(3-methyl-5-piperazin-1-yl-2-pyridyl)-1-piperidyl]pyrazolo[3,4-b]pyridine;
1,6-dimethyl-4-[*trans*-3-methyl-4-(3-methyl-5-piperazin-1-yl-2-pyridyl)-1-piperidyl]pyrazolo[3,4-b]pyridine;
(3*R*,4*R*)-1-[6-[(3*S*,4*R*)-1-(1,6-dimethylpyrazolo[3,4-b]pyridin-4-yl)-3-methyl-4-piperidyl]-5-methyl-3-pyridyl]-4-methoxy-pyrrolidin-3-amine;
2-[6-[(3*S*,4*R*)-1-(1,6-dimethylpyrazolo[3,4-b]pyridin-4-yl)-3-methyl-4-piperidyl]-5-methyl-3-pyridyl]-5-oxa-2,8-diazaspiro[3.5]nonane;
(6*S*)-4-[6-[(3*S*,4*R*)-1-(1,6-dimethylpyrazolo[3,4-b]pyridin-4-yl)-3-methyl-4-piperidyl]-5-methyl-3-pyridyl]-1,4-oxazepan-6-amine;
4-[(3*S*,4*R*)-4-[5-[(3*R*)-3-(methoxymethyl)piperazin-1-yl]-3-methyl-2-pyridyl]-3-methyl-1-piperidyl]-1,6-dimethyl-pyrazolo[3,4-b]pyridine;
4-[(3*S*,4*R*)-4-[5-(4,7-diazaspiro[2.5]octan-7-yl)-3-methyl-2-pyridyl]-3-methyl-1-piperidyl]-1,6-dimethyl-pyrazolo [3,4-b]pyridine;
(3*R*,4*R*)-1-[6-[(3*S*,4*R*)-1-(1,6-dimethylpyrazolo[3,4-b]pyridin-4-yl)-3-methyl-4-piperidyl]-5-methyl-3-pyridyl]-3-fluoro-piperidin-4-amine;
1-[6-[(3*S*,4*R*)-1-(1,6-dimethylpyrazolo[3,4-b]pyridin-4-yl)-3-methyl-4-piperidyl]-5-methyl-3-pyridyl]-3-methyl-azetidin-3-amine;
(4*aR*,7*aR*)-6-[6-[(3*S*,4*R*)-1-(1,6-dimethylpyrazolo[3,4-b]pyridin-4-yl)-3-methyl-4-piperidyl]-5-methyl-3-pyridyl]-3,4,4a,5,7,7a-hexahydro-2H-pyrrolo[3,4-b][1,4]oxazine;
1,6-dimethyl-4-[(3*R*,4*S*)-3-methyl-4-(5-piperazin-1-yl-2-pyridyl)-1-piperidyl]pyrazolo[3,4-b]pyridine;
1,6-dimethyl-4-[(3*S*,4*R*)-3-methyl-4-(5-piperazin-1-yl-2-pyridyl)-1-piperidyl]pyrazolo[3,4-b]pyridine;
(3*R*,4*R*)-1-[6-[(3*R*,4*S*)-1-(1,6-dimethylpyrazolo[3,4-b]pyridin-4-yl)-3-methyl-4-piperidyl]-3-pyridyl]-4-methoxy-pyrrolidin-3-amine;

4-[(3*R*,4*S*)-4-[5-[(3*R*)-3-(methoxymethyl)piperazin-1-yl]-2-pyridyl]-3-methyl-1-piperidyl]-1,6-dimethyl-pyrazolo[3,4-b]pyridine;
2-[6-[(3*R*,4*S*)-1-(1,6-dimethylpyrazolo[3,4-b]pyridin-4-yl)-3-methyl-4-piperidyl]-3-pyridyl]-5-oxa-2,8-diazaspiro[3.5]nonane;
(3*S*,4*S*)-1-[6-[(3*R*,4*S*)-1-(1,6-dimethylpyrazolo[3,4-b]pyridin-4-yl)-3-methyl-4-piperidyl]-3-pyridyl]-3-methoxy-piperidin-4-amine;
(6*S*)-4-[6-[(3*R*,4*S*)-1-(1,6-dimethylpyrazolo[3,4-b]pyridin-4-yl)-3-methyl-4-piperidyl]-3-pyridyl]-1,4-oxazepan-6-amine;
(3*R*,4*R*)-1-[6-[(3*R*,4*S*)-1-(1,6-dimethylpyrazolo[3,4-b]pyridin-4-yl)-3-methyl-4-piperidyl]-3-pyridyl]-3-fluoro-piperidin-4-amine;
4-[(3*R*,4*S*)-4-[5-(4,7-diazaspiro[2.5]octan-7-yl)-2-pyridyl]-3-methyl-1-piperidyl]-1,6-dimethyl-pyrazolo[3,4-b]pyridine; and
1-[6-[(3*R*,4*S*)-1-(1,6-dimethylpyrazolo[3,4-b]pyridin-4-yl)-3-methyl-4-piperidyl]-3-pyridyl]-3-methyl-azetidin-3-amine;
or a pharmaceutically acceptable salt thereof.

## PHARMACEUTICAL COMPOSITIONS AND ADMINISTRATION

Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compounds of formula (I) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to inhibit TLR7, 8, 9 interaction with respective stimulatory ligands, downstream signaling mediated by MYD88, IRF7, IRF5, NFκB etc. leading to production of type I interferons and proinflammatory cytokines (e.g. TNFα, IL-6), and activation of all kind of immune cells. For example, such amount may be below the amount that is toxic to normal cells, or the mammal as a whole.

In one example, the pharmaceutically effective amount of the compound of the invention administered parenterally per dose will be in the range of about 0.001 to 1000 mg/kg, alternatively about 0.001 to 1000 mg/kg of patient body weight per day, with the typical initial range of compound used being 0.3 to 15 mg/kg/day. In another embodiment, oral unit dosage forms, such as tablets and capsules, preferably contain from about 0.1 to about 1000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of

the pharmaceutical product (i.e., medicament).

An example of a suitable oral dosage form is a tablet containing about 0.1 to 1000 mg of the compound of the invention compounded with about 0.1 to 1000 mg anhydrous lactose, about 0.1 to 1000 mg sodium croscarmellose, about 0.1 to 1000 mg polyvinylpyrrolidone (PVP) K30, and about 0.1 to 1000 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 0.1 to 1000 mg, of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of Formula (I), or a stereoisomer or pharmaceutically acceptable salt thereof. In a further embodiment includes a pharmaceutical composition comprising a compound of Formula (I), or a stereoisomer or pharmaceutically acceptable salt thereof, together with a pharmaceutically acceptable carrier or excipient.

Another embodiment includes a pharmaceutical composition comprising a compound of Formula (I) for use in the treatment of an autoimmune disease. Another embodiment includes a pharmaceutical composition comprising a compound of Formula (I) for use in the treatment of autoimmune disease.

The following embodiments illustrate typical compositions of the present invention, but serve merely as representative thereof.

**Composition A**

A compound of the present invention can be used in a manner known per se as the active ingredient for the production of tablets of the following composition:

Per tablet

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

**Composition B**

A compound of the present invention can be used in a manner known per se as the active ingredient for the production of capsules of the following composition:

Per capsule

| | |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

**SYNTHESIS**

The compounds of the present invention can be prepared by any conventional means. Suitable processes for synthesizing these compounds as well as their starting materials are provided in the schemes below and in the examples.

All substituents, in particular, $R^1$ to $R^5$, A, M, W and Q are as defined above unless otherwise indicated. Furthermore, and unless explicitly otherwise stated, all reactions, reaction conditions, abbreviations and symbols have the meanings well known to a person of ordinary skill in organic chemistry.

General synthetic routes for preparing the compound of formula (I) or (Ia) or (Ib) are shown below.

## Scheme 1

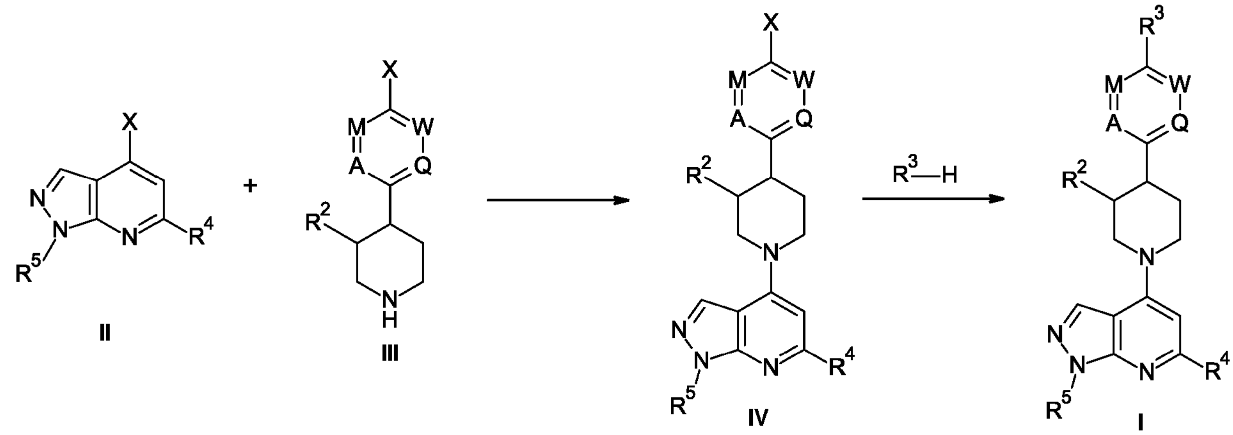

II III IV I

Wherein X is halogen.

The coupling of compound of formula (II) with (III) can be achieved by direct substitution at elevated temperature in the presence of a base, such as DIPEA or CsF, or via coupling reaction of Buchwald-Hartwig C-N bond formation (ref: Acc. Chem. Res. 1998, 31, 805-818; Chem. Rev. 2016, 116, 12564-12649; Topics in Current Chemistry, 2002, 219, 131-209; and references cited therein) with a catalyst, such as RuPhos Pd G2, $Pd_2(dba)_3$/XantPhos and a base, such as $Cs_2CO_3$ or *t*-BuONa, to provide compound of formula (IV). Subsequently, the coupling of compound of formula (IV) with $R^3$-H can be achieved by direct coupling under Buchwald-Hartwig C-N bond formation conditions with a catalyst, such as RuPhos Pd G2, $Pd_2(dba)_3$/XantPhos and a base, such as $Cs_2CO_3$ or *t*-BuONa, to provide compound of formula (I). In some embodiments, the coupling of compound of formula (IV) with $R^3$-H may give a product containing a protecting group, e.g. Boc or Cbz, originated from $R^3$-H, which will be removed before affording the final compound of formula (I).

## Scheme 2

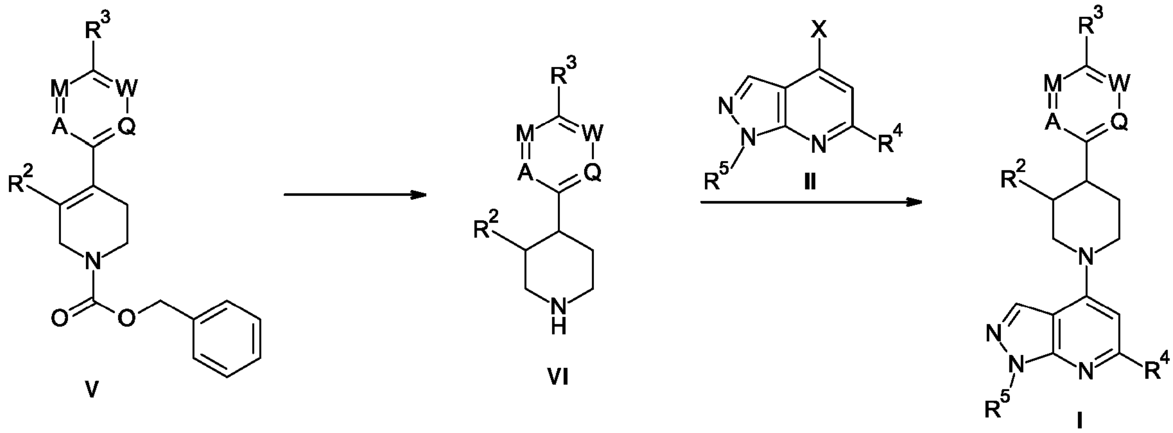
V VI II I

Wherein X is halogen.

Compound of formula (VI) can be achieved by hydrogenation reaction from compound of formula (V) in the presence of a catalyst, such as $Pd(OH)_2$/C, Pd/C or Wilkinson catalyst under hydrogen atmosphere. The Cbz group could also be de-protected under the same condition. Subsequently, the coupling of compound of formula (VI) with (II) can be achieved by direct substitution at elevated temperature in the presence of a base, such as DIPEA or CsF, or by Buchwald-Hartwig C-N bond formation at conditions with a catalyst, such as RuPhos Pd G2, $Pd_2(dba)_3$/XantPhos and a base, such as $Cs_2CO_3$ or *t*-BuONa, to provide compound of formula (I). In some embodiments, the coupling of compound of formula (VI) with (II) may give a product containing a protecting group, e.g. Boc, originated from (VI), which will be removed before

affording the final compound of formula (I).

Compounds of this invention can be obtained as mixtures of diastereomers or enantiomers, which can be separated by methods well known in the art, e.g. (chiral) HPLC or SFC.

This invention also relates to a process for the preparation of a compound of formula (I) or (Ia) comprising the following steps:

a) the Buchwald-Hartwig C-N bond formation reaction between compound of formula (IV),

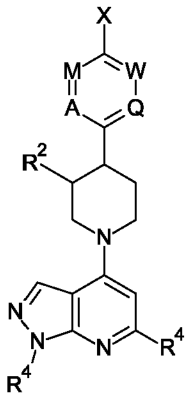

(IV),

and $R^3$-H , in the presence of a catalyst and a base;

b) the Buchwald-Hartwig C-N bond formation reaction between compound of formula (VI),

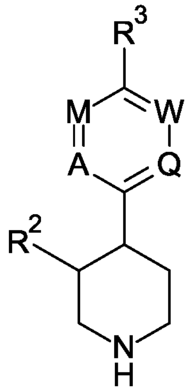

(VI),

and compound of formula (II),

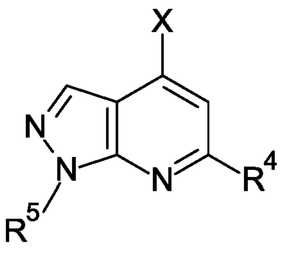

(II),

in the presence of a catalyst and a base;

c) the direct substation reaction between compound of formula (VI) and compound of formula (II) at in the presence of a base;

wherein

in step a) and b), the catalyst can be, for example, RuPhos Pd G2, $Pd_2(dba)_3$/XantPhos; the base can be, for example,

$Cs_2CO_3$ or *t*-BuONa;
in step c), the base can be DIPEA or CsF.

A compound of formula (I) or (Ia) when manufactured according to the above process is also an object of the invention.

**INDICATIONS AND METHODS OF TREATMENT**

The present invention provides compounds that can be used as TLR7 and TLR8 and TLR9 antagonist, which inhibits pathway activation through TLR7 and/or TLR8 and/or TLR9 as well as respective downstream biological events including, but not limited to, innate and adaptive immune responses mediated through the production of all types of cytokines and all forms of auto-antibodies. Accordingly, the compounds of the invention are useful for blocking TLR7 and/or TLR8 and/or TLR9 in all types of cells that express such receptor(s) including, but not limited to, plasmacytoid dendritic cell, B cell, T cell, macrophage, monocyte, neutrophil, keratinocyte, epithelial cell. As such, the compounds can be used as a therapeutic or prophylactic agent for systemic lupus erythematosus and lupus nephritis.

The present invention provides methods for treatment or prophylaxis of systemic lupus erythematosus and lupus nephritis in a patient in need thereof.

Another embodiment includes a method of treating or preventing systemic lupus erythematosus and lupus nephritis in a mammal in need of such treatment, wherein the method comprises administering to said mammal a therapeutically effective amount of a compound of formula (I), a stereoisomer, tautomer, or pharmaceutically acceptable salt thereof.

**EXAMPLES**

The invention will be more fully understood by reference to the following examples. They should not, however, be construed as limiting the scope of the invention.

**ABBREVIATIONS**

The invention will be more fully understood by reference to the following examples. They should not, however, be construed as limiting the scope of the invention.

Abbreviations used herein are as follows:

| | |
|---|---|
| ACN: | acetonitrile |
| AIBN: | azobisisobutyronitrile |
| $Boc_2O$: | di-*tert* butyl dicarbonate |
| CbzCl: | benzylchloroformate |
| CyPF-*t*-Bu: | [(*R*)-1-[(*S*)-2-(dicyclohexylphosphino)ferrocenyl]ethyl]di-*tert*-butylphosphine |
| DCM: | dichloromethane |
| DEA: | diethylamine |
| DIPEA: | *N,N*-diisopropylethylamine |
| DMA: | dimethylacetamide |
| DMF: | *N,N*-dimethylformamide |
| DMFDMA: | *N,N*-dimethylformamide dimethyl acetal |
| dtbbpy: | 4,4'-Di-tert-butyl-2,2'-dipyridyl |
| EtOAc or EA: | ethyl acetate |
| FA: | formic acid |
| HLM | human liver microsome |
| $IC_{50}$: | half inhibition concentration |
| $Ir[dF(CF_3)ppy]_2(dtbpy)(PF_6)$: | [4,4'-Bis(1,1-dimethylethyl)-2,2'-bipyridine-N1,N1']bis[3,5-difluoro-2-[5-(trifluoromethyl)-2-pyridinyl-N]phenyl-C]Iridium(III) hexafluorophosphate |
| JackiePhos: | Bis(3,5-bis(trifluoromethyl)phenyl)(2',4',6'- triisopropyl-3,6-dimethoxybiphenyl-2-yl) phosphine |
| LCMS | liquid chromatography-mass spectrometry |
| MS: | mass spectrometry |
| $[Pd(allyl)Cl]_2$: | allylpalladium(II) chloride dimer |
| $Pd_2(dba)_3$: | Tris(dibenzylideneacetone)dipalladium |
| $Pd[P(o\text{-}tol)_3]_2$: | bis(tri-*o*-tolylphosphine)palladium |

| | |
|---|---|
| PE: | petroleum ether |
| prep-HPLC: | preparative high performance liquid chromatography |
| rt: | room temperature |
| RuPhos Pd G2: | chloro(2-dicyclohexylphosphino-2',6'-diisopropoxy-1,1'-biphenyl)[2-(2'-amino-1,1'-biphenyl)]palladium(II) 2nd generation |
| SFC: | supercritical fluid chromatography |
| TCDI: | 1,1'-thiocarbonyldiimidazole |
| TEA: | trimethylamine |
| TFA: | trifluoroacetic acid |
| $Tf_2O$: | trifluoromethanesulfonic anhydride |
| THF: | tetrahydrofuran |
| TTMSS: | 1,1,1,3,3,3-Hexamethyl-2-trimethylsilyl-trisilane |
| v/v: | volume ratio |
| XantPhos: | 4,5-Bis(diphenylphosphino)-9,9-dimethylxanthene |

## GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel brand and pore size: i) KP-SIL 60 Å, particle size: 40-60 μm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge™ Prep-C18 (5 μm, OBDTM 30 × 100 mm) column, SunFire™ Prep-C18 (5 μm, OBD™ 30 × 100 mm) column, Phenomenex Synergi-C18 (10 μm, 25 × 150 mm) or Phenomenex Gemini-C18 (10 μm, 25 × 150 mm). Waters AutoP purification System (Sample Manager 2767, Pump 2525, Detector: Micromass ZQ and UV 2487, solvent system: acetonitrile and 0.1% ammonium hydroxide in water; acetonitrile and 0.1% FA in water or acetonitrile and 0.1% TFA in water). Or Gilson-281 purification System (Pump 322, Detector: UV 156, solvent system: acetonitrile and 0.05% ammonium hydroxide in water; acetonitrile and 0.225% FA in water; acetonitrile and 0.05% HCl in water; acetonitrile and 0.075% TFA in water; or acetonitrile and water).

For SFC chiral separation, intermediates were separated by chiral column (Daicel chiralpak IC, 5 μm, 30 × 250 mm), AS (10 μm, 30 × 250 mm) or AD (10 μm, 30 × 250 mm) using Mettler Toledo Multigram III system SFC, Waters 80Q preparative SFC or Thar 80 preparative SFC, solvent system: $CO_2$ and IPA (0.5% TEA in IPA) or $CO_2$ and MeOH (0.1% $NH_3 \cdot H_2O$ in MeOH), back pressure 100bar, detection UV@ 254 or 220 nm.

LC/MS spectra of compounds were obtained using a LC/MS (Waters™ Alliance 2795-Micromass ZQ, Shimadzu Alliance 2020-Micromass ZQ or Agilent Alliance 6110-Micromass ZQ), LC/MS conditions were as follows (running time 3 or 1.5 mins):

Acidic condition I: A: 0.1% TFA in $H_2O$; B: 0.1% TFA in acetonitrile;
Acidic condition II: A: 0.0375% TFA in $H_2O$; B: 0.01875% TFA in acetonitrile;
Basic condition I: A: 0.1% $NH_3 \cdot H_2O$ in $H_2O$; B: acetonitrile;
Basic condition II: A: 0.025% $NH_3 \cdot H_2O$ in $H_2O$; B: acetonitrile;
Neutral condition: A: $H_2O$; B: acetonitrile.
Mass spectra (MS): generally only ions which indicate the parent mass are reported, and unless otherwise stated the mass ion quoted is the positive mass ion $(MH)^+$.
NMR Spectra were obtained using Bruker Avance 400 MHz.

The microwave assisted reactions were carried out in a Biotage Initiator Sixty microwave synthesizer. All reactions involving air-sensitive reagents were performed under an argon or nitrogen atmosphere. Reagents were used as received from commercial suppliers without further purification unless otherwise noted.

## PREPARATIVE EXAMPLES

The following examples are intended to illustrate the meaning of the present invention but should by no means represent a limitation within the meaning of the present invention:

### Intermediate A

**4-[(3*S*,4*R*)-4-(5-chloro-2-pyridyl)-3-methyl-1-piperidyl]-1,6-dimethyl-pyrazolo[3,4-b]pyridine**

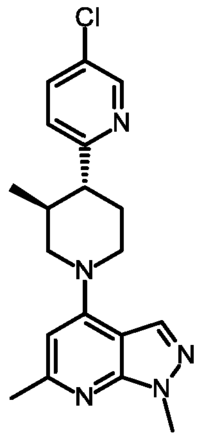

The title compound was prepared according to the following scheme:

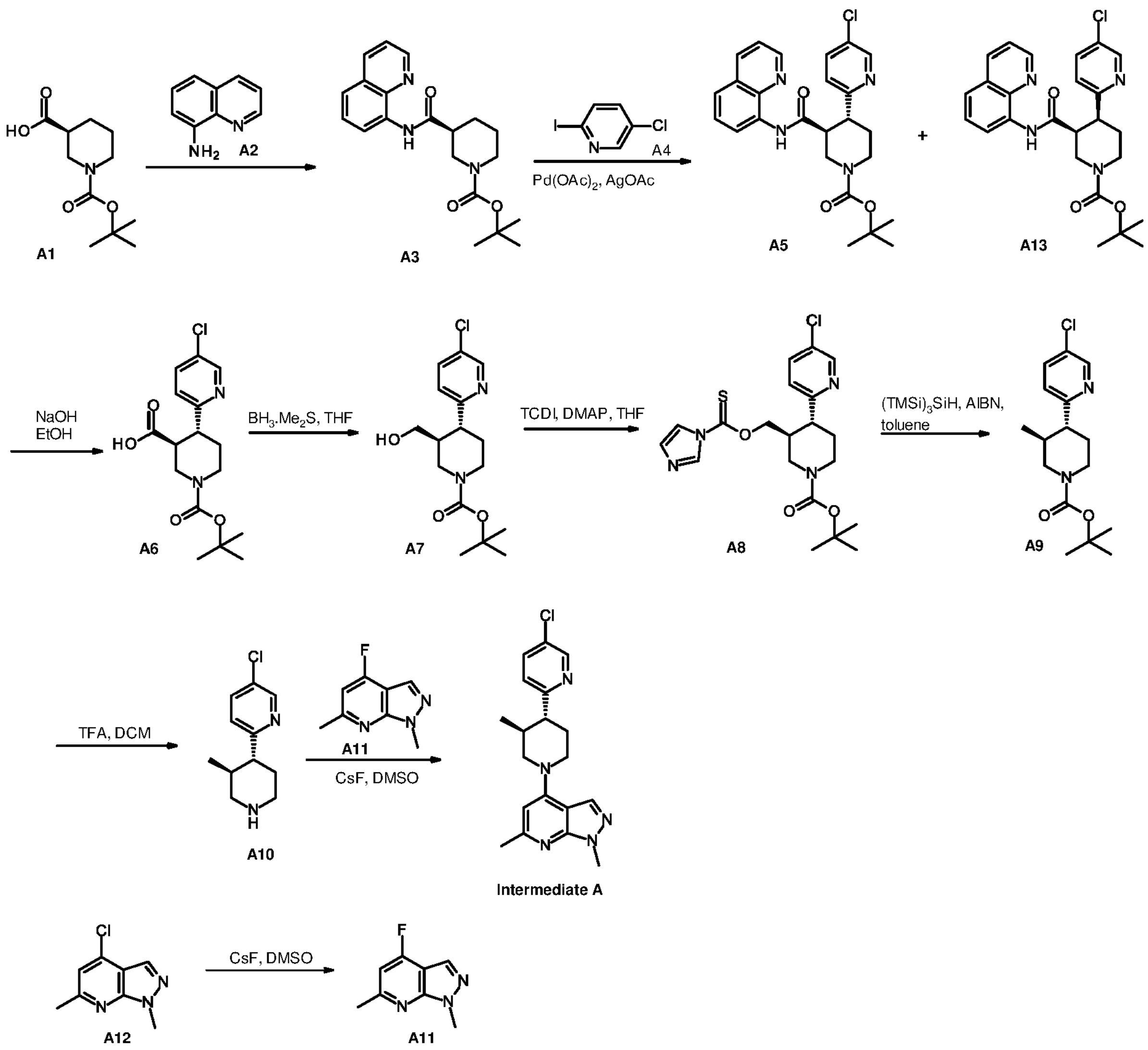

**Step 1: preparation of *tert*-butyl (3*S*)-3-(8-quinolylcarbamoyl)piperidine-1-carboxylate (compound A3)**

To a solution of 8-aminoquinoline (compound **A2,** CAS: 578-66-5, Vendor: Accela, 69.17 g, 479.78 mmol) in DMF (1000 mL) was added HATU (190.60 g, 501.59 mmol) and DIPEA (233 mL, 1309 mmol). The reaction was stirred at 0 °C for 1h. Then (3*S*)-1-*tert*-butoxycarbonylpiperidine-3-carboxylic acid (compound **A1,** CAS: 88495-54-9, Vendor: Accela, 100.00 g, 436.17 mmol) was added. The reaction was stirred at 0 °C for 15h. EtOAc (200 mL) and ice-water (50 mL) were added and separated. The aqueous phase was extracted with EtOAc (200 mL) twice. The combined organic layer was washed with brine (300 mL) for 4 times, dried over $Na_2SO_4$, filtered, and concentrated under vacuum. The residue was purified by flash column eluting with a gradient of EA/PE (1:5) to give compound **A3** (120 g) as a yellow solid. MS: calc'd 356 ($MH^+$), measured 356 ($MH^+$).

**Step 2: preparation of *tert*-butyl (3*S*,4*R*)-4-(5-chloro-2-pyridyl)-3-(8-quinolylcarbamoyl)piperidine-1-carboxylate (compound A5) and *tert*-butyl (3S,4S)-4-(5-chloro-2-pyridyl)-3-(8-quinolylcarbamoyl)piperidine-1-carboxylate (compound A13)**

The mixture of *tert*-butyl (3*S*)-3-(8-quinolylcarbamoyl)piperidine-1-carboxylate (compound **A3,** 50.00 g, 140.67 mmol), 5-chloro-2-iodo-pyridine (compound **A4,** CAS: 244221-57-6, Vendor: Bide Pharmatech, 77.00 g, 322.18 mmol) and $Pd(OAc)_2$ (3.15 g, 14.07 mmol), AgOAc(46.70 g, 281.35 mmol) was degassed with $N_2$, and then stirred at 110 °C for 48 h under $N_2$ atmosphere. After being cooled to room temperature, the mixture was filtrated. The filtrate was concentrated under vacuum. The residue was further purified by flash column eluting with a gradient of EA/PE (1/10 to 1/1) to give compound **A5** (20.00 g) and compound **A13** (15.00 g) as yellow oil. MS: calc'd 467 ($MH^+$), measured 467 ($MH^+$).

**Step 3: preparation of (3*S*,4*R*)-1-*tert*-butoxycarbonyl-4-(5-chloro-2-pyridyl)piperidine-3-carboxylic acid (compound A6)**

To a solution of *tert*-butyl (3*S*,4*R*)-4-(5-chloro-2-pyridyl)-3-(8-quinolylcarbamoyl)piperidine-1-carboxylate (compound **A5**, 17.00 g, 36.41 mmol) in ethanol (200 mL) was added sodium hydroxide (14.57 g, 364.22 mmol) and then stirred at 85 °C for 16 h. After being cooled to room temperature, the reaction mixture was concentrated under reduced pressure. The residue was neutralized with HCl (3 M) to pH 6. The mixture was extracted with DCM (300mL) three times. The organic phase was concentrated in vacuum. The residue was purified by flash column eluting with a gradient of PE/EA (5/1 to 1/1) to give compound **A6** (11.50 g) as yellow oil. MS: calc'd 341 ($MH^+$), measured 285 ($M-C_4H_8+H^+$).

**Step 4: preparation of *tert*-butyl (3*S*,4*R*)-4-(5-chloro-2-pyridyl)-3-(hydroxymethyl)piperidine-1-carboxylate (compound A7)**

To a solution of (3*S*,4*R*)-1-*tert*-butoxycarbonyl-4-(5-chloro-2-pyridyl)piperidine-3-carboxylic acid (compound **A6,** 11.50 g, 33.74 mmol) in THF (50 mL) was added $BH_3.Me_2S$ (13.5 mL, 134.98 mmol) at 0 °C. After addition, the reaction mixture was further stirred at 25 °C under $N_2$ atmosphere for 16h. The volatiles were removed to give a white solid residue and then methanol (50 mL) was added. After being stirred for 30 min, the reaction mixture was concentrated and methanol (50 mL) was added again. The residue was then treated with dropwise addition of 10% citric acid. The mixture was extracted with DCM (200 mL) for three times. The combined organic layer was dried with $Na_2SO_4$, filtered and concentrated in vacuum. The residue was purified by flash column eluting with a gradient of PE/EA (3/1) to give compound **A7** (8.00 g) as colorless gum. MS: calc'd 327 ($MH^+$), measured 271 ($M-C_4H_8+H^+$).

**Step 5: preparation of *tert*-butyl (3*S*,4*R*)-4-(5-chloro-2-pyridyl)-3-(imidazole-1-carbothioyloxymethyl)piperidine-1-carboxylate (compound A8)**

To a solution of *tert*-butyl (3S,4R)-4-(5-chloro-2-pyridyl)-3-(hydroxymethyl)piperidine-1-carboxylate (compound **A7,** 4.00 g, 12.24 mmol) and TCDI (4.36 g, 24.48 mmol) in THF (60 mL) was added DMAP (299 mg, 2.45 mmol). The mixture was stirred at 25 °C for 3h. Then the reaction mixture was concentrated and the residue was purified by flash column eluting with a gradient of PE/EA (1/1) to give compound **A8** (5.00 g) as yellow oil. MS: calc'd 437 ($MH^+$), measured 437 ($MH^+$).

**Step 6: preparation of *tert*-butyl (3*S*,4*R*)-4-(5-chloro-2-pyridyl)-3-methyl-piperidine-1-carboxylate (compound A9)**

To a mixture of *tert*-butyl (3*S*,4*R*)-4-(5-chloro-2-pyridyl)-3-(imidazole-1-carbothioyloxymethyl)piperidine-1-carboxylate (compound **A8, 5**.00 g, 11.44 mmol) in toluene (250 mL) was added tris(trimethylsilyl)silane (17 mL, 47.56 mmol)

and AIBN (1877 mg, 11.44 mmol). The mixture was degassed with $N_2$ for 10 min and stirred at 85 °C for 5h. After being cooled to room temperature, the mixture was concentrated under vacuum. The residue was purified by flash column eluting with a gradient of PE/EA (5/1) and pre-HPLC to give compound **A9** (combination of two batches, 4.00 g) as yellow oil. MS: calc'd 311 ($MH^+$), measured 255 ($M-C_4H_8+H^+$).

**Step 7: preparation of 5-chloro-2-[(3*S*,4*R*)-3-methyl-4-piperidyl]pyridine (compound A10)**

To a solution of *tert*-butyl (3*S*,4*R*)-4-(5-chloro-2-pyridyl)-3-methyl-piperidine-1-carboxylate (compound **A9,** 4.00 g, 12.87 mmol) in DCM (20 mL) was added TFA (31 mL, 123.08 mmol) at 0 °C. The mixture was stirred at 25 °C for 16 h. The reaction mixture was concentrated under vacuum. The residue was purified by pre-HPLC to give compound **A10** (2.70 g) as a white solid. MS: calc'd 211 ($MH^+$), measured 211 ($MH^+$).

**Step 8: preparation of 4-fluoro-1,6-dimethyl-pyrazolo[3,4-b]pyridine (compound A11)**

The mixture of 4-chloro-1,6-dimethyl-pyrazolo[3,4-b]pyridine (compound **A12,** CAS: 19867-78-8, Vendor: PharmaBlock, 4.60 g, 25.33 mmol) and cesium fluoride (19.24 mg, 126.64 mmol) in DMSO (60 mL) was stirred at 120°C for 18 hrs. After being cooled to rt, the reaction mixture was added to water (100 mL), extracted with EA(100 mL) three times. The combined organic layer was washed with brine (200 mL) twice, dried over $Na_2SO_4$, filtered and concentrated. The residue was purified by flash column to give compound **A11** (4.00 g, contained some compound **A12**) as a white solid. MS: calc'd 166 ($MH^+$), measured 166 ($MH^+$).

**Step 9: preparation of 4-[(3*S*,4*R*)-4-(5-chloro-2-pyridyl)-3-methyl-1-piperidyl]-1,6-dimethyl-pyrazolo[3,4-b]pyridine (Intermediate A)**

To a mixture of 4-fluoro-1,6-dimethyl-pyrazolo[3,4-b]pyridine (compound **A11**, 2.12 g, 12.81 mmol) and 5-chloro-2-[(3*S*,4*R*)-3-methyl-4-piperidyl]pyridine (compound **A10,** 2.70 g, 12.81 mmol) in DMSO (50 mL) was added CsF (3.72 mg, 64.07 mmol). The reaction was stirred at 120 °C for 12 h under $N_2$ atmosphere. After being cooled to room temperature, the reaction mixture was poured into ice-water (200 mL), and extracted with EA (250 mL) three times. The combined organic layer was dried over $Na_2SO_4$, filtered and concentrated under vacuum. The residue was purified by flash column eluting with a gradient of PE/EA (1/1) to give a crude product which was further purified by SFC (Gradient: 55% MeOH (0.05% DEA) in $CO_2$, Column: Daicel Chiralpak IC, 250×30 mm, 10μm) to give **Intermediate A** (2.52 g, slower eluting) as a white solid. MS: calc'd 356 ($MH^+$), measured 356 ($MH^+$).

**Intermediate B**

**4-[(3*S*,4*R*)-4-(5-chloro-3-methyl-2-pyridyl)-3-methyl-1-piperidyl]-1,6-dimethyl-pyrazolo[3,4-b]pyridine**

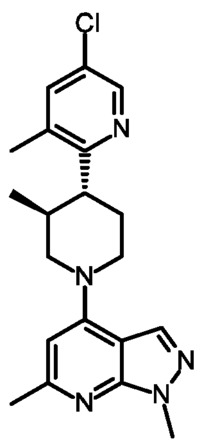

The title compound was prepared according to the following scheme:

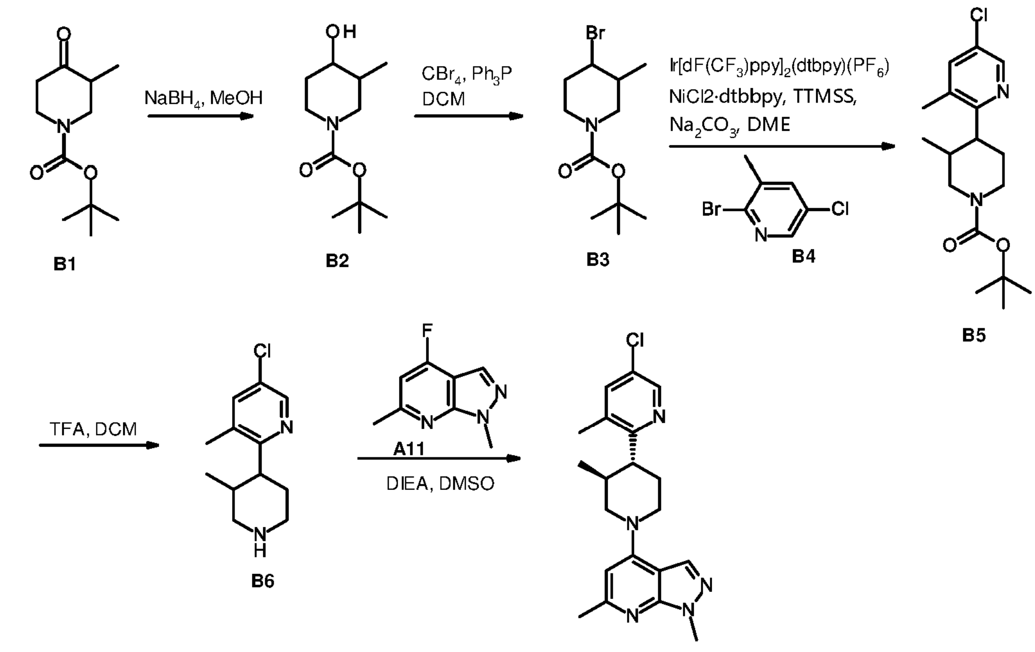

B1 B2 B3 B4 B5 B6

Intermediate B

**Step 1: preparation of *tert*-butyl 4-hydroxy-3-methyl-piperidine-1-carboxylate (compound B2)**

To a solution of *tert*-butyl 3-methyl-4-oxopiperidine-1-carboxylate (compound **B1**, CAS: 181269-69-2, Vendor: PharmaBlock, 50.00 g, 234.44 mmol) in methanol (500 mL) was added $NaBH_4$ (13.30 g, 351.67 mmol) in portions at 0° C. After being stirred at 0° C for 1h and then at 20 °C for 2.5h, the reaction mixture was quenched with saturated ammonium chloride aqueous solution and concentrated under reduced pressure in order to remove the methanol. Then the mixture was extracted with dichloromethane, the combined organic layer was dried over sodium sulfate, filtered and concentrated to afford compound **B2** (50.40 g) as light yellow oil.

**Step 2: preparation of *tert*-butyl 4-bromo-3-methyl-piperidine-1-carboxylate (compound B3)**

To a mixture of *tert*-butyl 4-hydroxy-3-methyl-piperidine-1-carboxylate (compound **B2,** 45.00 g, 209.02 mmol) in DCE (500 mL) was added $Ph_3P$ (71.27 g, 271.73 mmol) at 20°C, followed by addition of $CBr_4$ (110.91 g, 334.43 mmol) at 20°C. The flask was degassed and purged with $N_2$ for four times. was After being stirred at 25 °C for 15 h under $N_2$ atmosphere, the reaction mixture was concentrated, and the residue was purified by flash column eluting with a gradient of PE/EA (100/0 to 100/5) to give compound **B3** (35.00 g) as light yellow oil.

**Step 3: preparation of *tert*-butyl-4-(5-chloro-3-methyl-2-pyridyl)-3-methyl-piperidine-1-carboxylate (compound B5)**

To a 1000 mL vial equipped with a stir bar was added 2-bromo-5-chloro-3-methyl-pyridine (compound **B4,** CAS: 65550-77-8, Vendor: Accela, 22.00 g, 106.55 mmol), *tert*-butyl 4-bromo-3-methyl-piperidine-1-carboxylate (compound **B3,** 38.53 g, 138.52 mmol), $Ir[dF(CF_3)ppy]_2(dtbpy)(PF_6)$ (0.85 g, 1.07 mmol), $NiCl_2$·dtbbpy (0.21 g, 0.530 mmol), TTMSS (26.50 g, 106.55 mmol), $Na_2CO_3$ (22.59 g, 213.11 mmol) and DME (500 mL). The vial was sealed and purged with nitrogen. The mixture was stirred and irradiated with a 34 W blue LED lamp (7 cm away), with cooling fan to keep the reaction temperature at 25 °C for 14 h. The reaction was filtered, and washed with a mixture of solvent (DCM/MeOH=10/1, 5 mL) for three times. The filtrate was concentrated and the residue was purified by flash column eluting with a gradient of PE/EA (5/1) to afford compound **B5** (7.00 g) as light yellow oil. MS: calc'd 325 ($MH^+$), measured 269 ($M-C_4H_8+H^+$).

**Step 4: preparation of 5-chloro-3-methyl-2-(3-methyl-4-piperidyl)pyridine (compound B6)**

To a solution of *tert*-butyl-4-(5-chloro-3-methyl-2-pyridyl)-3-methyl-piperidine-1-carboxylate (compound **B5,** 7.00 g, 21.55 mmol) in DCM (30 mL) was added TFA (10 mL) dropwise at 0 °C. was After being stirred at 20 °C for 2 h, the reaction mixture was concentrated and the residue was dissolved in DCM (50 mL) and basified by saturated $NaHCO_3$ solution. The mixture was extracted with DCM (40 mL) for three times. The combined organic layer was washed

with 50 mL brine, dried over $Na_2SO_4$ and concentrated to give compound **B6** (4.80 g) as light yellow oil. MS: calc'd 225 ($MH^+$), measured 225 ($MH^+$).

**Step 5: preparation of 4-[(3*S*,4*R*)-4-(5-chloro-3-methyl-2-pyridyl)-3-methyl-1-piperidyl]-1,6-dimethyl-pyrazolo [3,4-b]pyridine (Intermediate B)**

To a mixture of 5-chloro-3-methyl-2-(3-methyl-4-piperidyl)pyridine (compound **B6,** 4.60 g, 20.47 mmol) and 4-fluoro-1,6-dimethyl-pyrazolo[3,4-b]pyridine (compound **A11,** 3.72 g, 22.52 mmol) in DMSO (40 mL) was added DIPEA (2.37 g, 40.94 mmol). The reaction was stirred at 120 °C for 3 h under $N_2$ atmosphere. After being cooled to rt, The reaction mixture was poured into ice-water (400 mL), extracted with EA (80 mL) for 3 times. The combined organic layer was dried over $Na_2SO_4$, filtered and concentrated under vacuum. The residue was purified by flash column eluting with a gradient of EA/PE (1/1) to give a mixture (5.40 g) as a light brown solid. The mixture was continued to be purified by SFC (Gradient: 40% EtOH (0.1% $NH_3H_2O$) in $CO_2$, Column: Daicel Chiralpak AD, 250×30 mm, 10μm) to afford **Intermediate B** (2.10 g) as a light yellow solid. MS: calc'd 370 ($MH^+$), measured 370 ($MH^+$).

**Intermediate C**

**4-[4-(6-bromo-3-pyridyl)-3-methyl-1-piperidyl]-1-methyl-pyrazolo[3,4-b]pyridine**

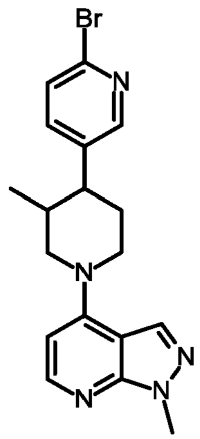

The title compound was prepared according to the following scheme:

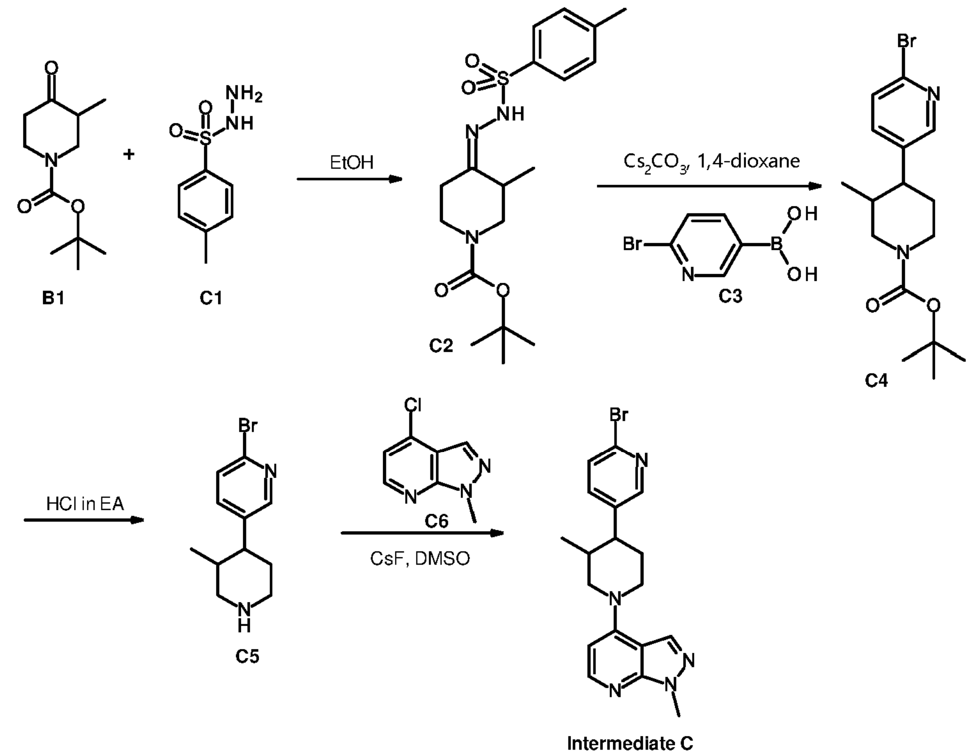


B1 C1 C2 C3 C4 C5 C6

Intermediate C

**Step 1: preparation of *tert*-butyl-3-methyl-4-(p-tolylsulfonylhydrazono)piperidine-1-carboxylate (compound C2)**

To a solution of *tert*-butyl-3-methyl-4-oxopiperidine-1-carboxylate (compound **B1,** CAS: 181269-69-2, Vendor: PharmaBlock, 300 mg, 1.41 mmol) in Ethanol (10 mL) was added 4-methylbenzenesulfonohydrazide (compound **C1,** 341 mg, 1.83 mmol). The mixture was stirred at 70 °C for 3 h. After being cooled to rt, the mixture was concentrated to afford crude product compound **C2** (537 mg) which was used in next step without further purification. MS: calc'd 382 ($MH^+$), measured 382 ($MH^+$).

**Step 2: preparation of *tert*-butyl 4-(6-bromo-3-pyridyl)-3-methyl-piperidine-1-carboxylate (compound C4)**

To a solution of *tert*-butyl-3-methyl-4-(p-tolylsulfonylhydrazono)piperidine-1-carboxylate (compound **C2,** 537 mg, 1.41 mmol) in 1,4-dioxane (5 mL) was added (6-bromopyridin-3-yl)boronic acid (compound **C3,** CAS: 223463-14-7, Vendor: Accela, 369 mg, 1.83 mmol) and $Cs_2CO_3$ (1.38 g, 4.22 mmol). The mixture was stirred at 120°C under $N_2$ for 16h. After being cooled to rt, the mixture was filtered and the solid was washed with EA (10 mL) twice. The combined organic layer was concentrated and purified by flash column eluting with a gradient of EA/PE (0% to 70%) to give the desired produc**t C4** (50 mg) as a colorless oil. MS: calc'd 355 ($MH^+$), measured 355 ($MH^+$).

**Step 3: preparation of 2-bromo-5-(3-methyl-4-piperidyl)pyridine (compound C5)**

The mixture of *tert*-butyl 4-(6-bromo-3-pyridyl)-3-methyl-piperidine-1-carboxylate (compound **C4,** 50 mg, 141 μmol) in HCl (1M in EA, 10 ml, 10 mmol) was stirred at rt for 2h. The mixture was concentrated to give the desired product **C5** (41 mg) as a white solid. MS: calc'd 255 ($MH^+$), measured 255 ($MH^+$).

**Step 4: preparation of 4-[4-(6-bromo-3-pyridyl)-3-methyl-1-piperidyl]-1-methyl-pyrazolo[3,4-b]pyridine (Intermediate C)**

To a solution of 4-chloro-1-methyl-pyrazolo[3,4-b]pyridine (compound **C6,** CAS: 1268520-92-8, Vendor: PharmaBlock, 35 mg, 211 μmol) and 2-bromo-5-(3-methyl-4-piperidyl)pyridine (compound **C5,** 41 mg, 141 μmol) in DMSO (15 mL) was added CsF (107 mg, 703 μmol). The reaction mixture was stirred at 130 °C overnight. After being cooled to room temperature, the reaction was diluted with EA, washed with water (30 mL) four times, dried over $Na_2SO_4$, filtered and concentrated under vacuum. The residue was purified by flash column eluting with a gradient of EA(with 10% MeOH)/PE (0% to 70%) to give the **Intermediate C** (42 mg). MS: calc'd 386 ($MH^+$), measured 386 ($MH^+$).

**Intermediate D**

**4-[(3*R*,4*S*)-4-(5-chloro-2-pyridyl)-3-methyl-1-piperidyl]-1,6-dimethyl-pyrazolo[3,4-b]pyridine**

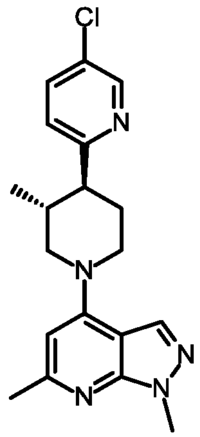

The title compound was prepared in analogy to the preparation of **Intermediate A** by using compound **A13** instead of compound **A5. Intermediate D** was obtained as a white solid. MS: calc'd 356 ($MH^+$), measured 356 ($MH^+$). (Note: The stereo center of the 3-position on the piperidine ring is epimerized from *S* configuration to *R* configuration, when intermediate A13 was treated with sodium hydroxide in ethanol, to afford (3*R*,4*S*)-1-tert-butoxycarbonyl-4-(5-chloro-2-pyridyl)piperidine-3-carboxylic acid.)

**Intermediate E**

**4-[4-(5-chloro-3-methyl-2-pyridyl)-1-piperidyl]-1,6-dimethyl-pyrazolo[3,4-b]pyridine**

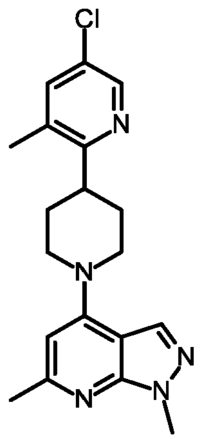

The title compound was prepared according to the following scheme:

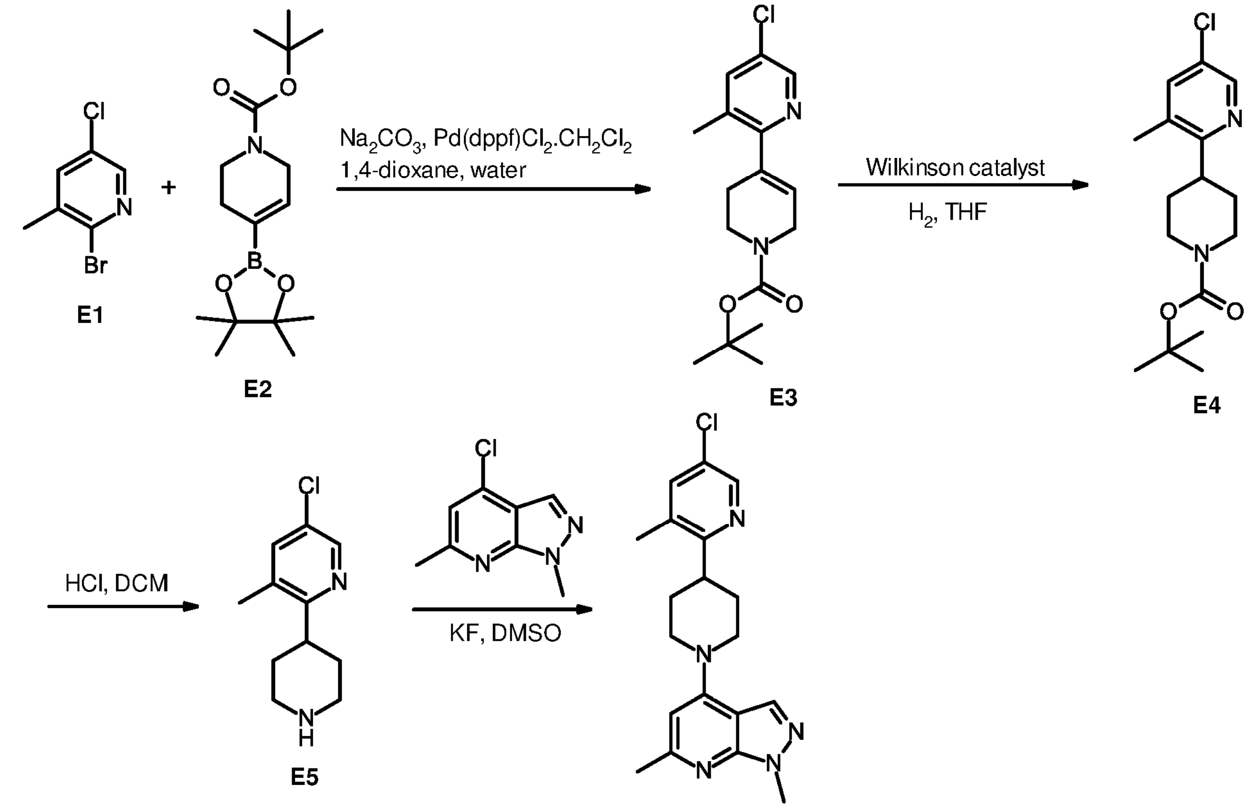


E1 E2 E3 E4 E5

Intermediate E

**Step 1: preparation of *tert*-butyl 4-(5-chloro-3-methyl-2-pyridyl)-3,6-dihydro-2*H*-pyridine-1-carboxylate (compound E3)**

To a mixture of 2-bromo-5-chloro-3-methyl-pyridine (compound **E1,** 15.00 g, 72.65 mmol) and *tert*-butyl 4-(4,4,5,5-tetramethyl-1,3,2-dioxaborolan-2-yl)-3,6-dihydro-2H-pyridine-1-carboxylate (compound **E2,** 24.71 g, 79.91 mmol) in 1,4-dioxane (400 mL) was added sodium carbonate (15.40 g, 145.30 mmol) in water (40 mL) and Pd(dppf) $Cl_2.CH_2Cl_2$ (5.93 g, 7.26 mmol). The mixture was stirred at 95°C for 16 h under $N_2$ atmosphere. After being cooled to room temperature, the mixture was filtered and washed with 1,4-dioxane (10 mL) for three times. The filtrate was concentrated, and the crude was purified by flash column eluting with a gradient of PE/EA (5/1 to 3/1) to give compound **E3** (9.40 g) as a yellow oil. MS: calc'd 309 ($MH^+$), measured 309 ($MH^+$).

**Step 2: preparation of *tert*-butyl 4-(5-chloro-3-methyl-2-pyridyl)piperidine-1-carboxylate (compound E4)**

To the solution of *tert*-butyl 4-(5-chloro-3-methyl-2-pyridyl)-3,6-dihydro-2*H*-pyridine-1-carboxylate (compound **E3,** 6.00 g, 19.43 mmol) in tetrahydrofuran (120 mL) was added tris(triphenylphosphine)rhodium(I) chloride (4.49 g, 4.86 mmol). The flask was degassed and purged with $H_2$ for four times. The mixture was stirred at 60 °C for 36 h under hydrogen atmosphere (45 psi). The mixture was filtered on celite and the solid was washed with methanol (5 mL). The solvent was removed under vacuum and the crude was purified by flash column eluting with a gradient of PE/EA (5/1 to 3/1) to give compound **E4** (4.50 g) as an off-white solid. MS: calc'd 311 ($MH^+$), measured 311 ($MH^+$).

**Step 3: preparation of 5-chloro-3-methyl-2-(4-piperidyl)pyridine (compound E5)**

To a solution of *tert*-butyl 4-(5-chloro-3-methyl-2-pyridyl) piperidine-1-carboxylate (compound **E4,** 4.50 g, 14.48 mmol) in DCM (50 mL) was added HCl in dioxane (10 mL) at 0°C. After being stirred at 25 °C for 16 h, the reaction mixture was concentrated under reduced pressure to give compound **E5** (3.56 g) as a white solid. MS: calc'd 211 ($MH^+$), measured 211 ($MH^+$).

**Step 4: preparation of 4-[4-(5-chloro-3-methyl-2-pyridyl)-1-piperidyl]-1,6-dimethyl-pyrazolo[3,4-b]pyridine (Intermediate E)**

To a mixture of 4-chloro-1,6-dimethyl-pyrazolo[3,4-b]pyridine (1.15 g, 6.33 mmol) and 5-chloro-3-methyl-2-(4-piperidyl)pyridine (compound **E5,** 1.72 g, 6.97 mmol) in DMSO (30 mL) was added KF (3.67 g, 63.32 mmol). The reaction was stirred at 120 °C for 15 h under $N_2$ atmosphere. After being cooled to rt, the reaction mixture was poured into ice-water (200 mL) and aq. $NaHCO_3$ (50 mL), extracted with EA (250 mL) three times. The combined organic layer was dried over $Na_2SO_4$, filtered and concentrated under vacuum. The crude was purified by flash column eluting with a gradient of PE/EA (5/1 to 0/1) to give **Intermediate E** (819 mg) as a light yellow solid. MS: calc'd 356 ($MH^+$), measured 356 ($MH^+$).

**Example 1**

**1,6-dimethyl-4-[4-(4-piperazin-1-ylphenyl)-1-piperidyl]pyrazolo[3,4-b]pyridine**

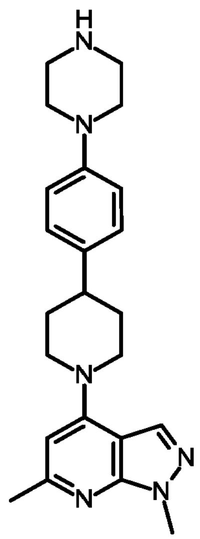

The title compound was prepared according to the following scheme:

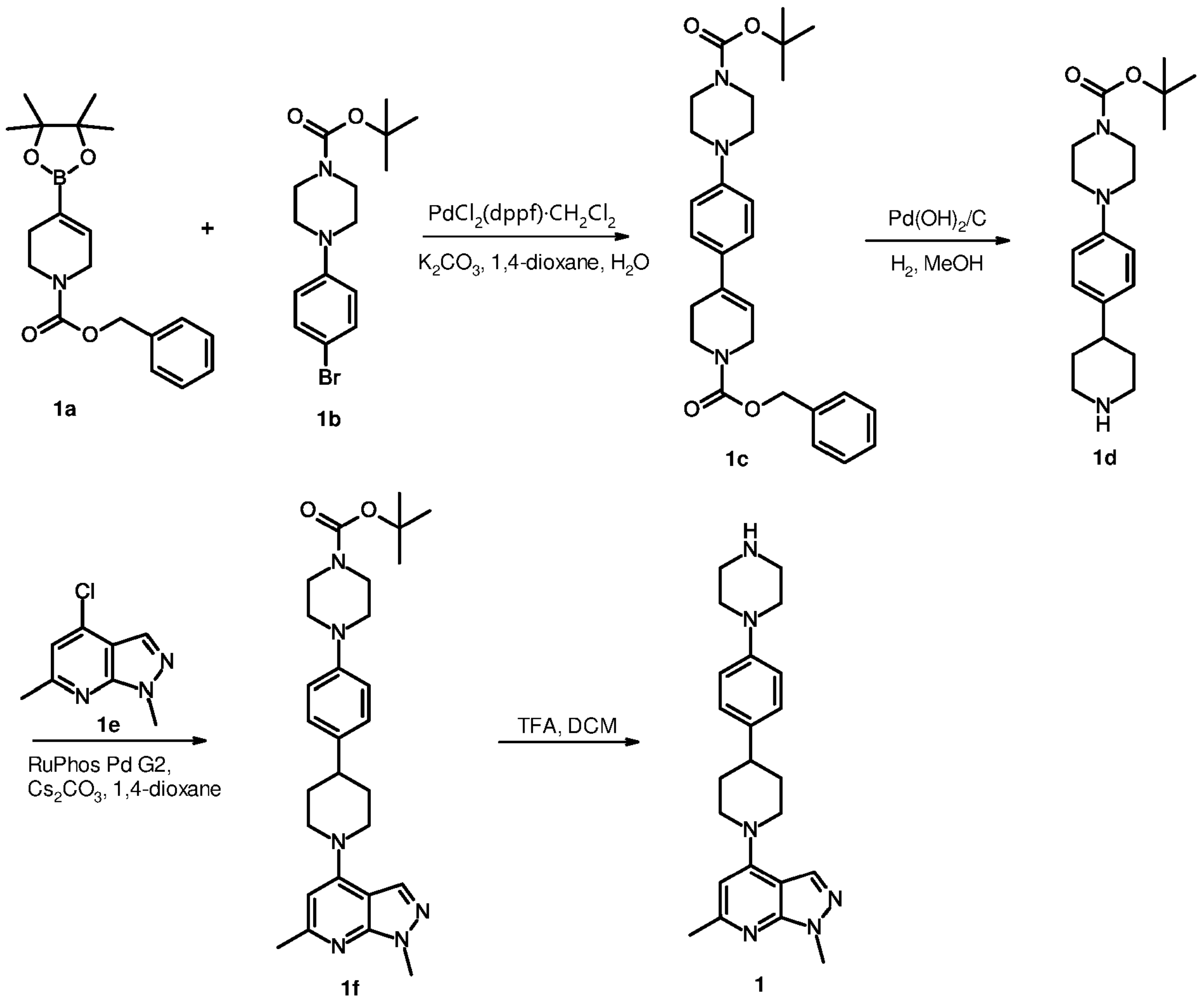


1a 1b 1c 1d 1e 1f 1

**Step 1: preparation of *tert*-butyl 4-[4-(1-benzyloxycarbonyl-3,6-dihydro-2*H*-pyridin-4-yl)phenyl]piperazine-1-carboxylate (compound 1c)**

To the mixture of benzyl 4-(4,4,5,5-tetramethyl-1,3,2-dioxaborolan-2-yl)-3,6-dihydropyridine-1(2*H*)-carboxylate (compound **1a,** CAS: 286961-15-7, Vendor: Accela, 100 mg, 291 μmol), *tert*-butyl 4-(4-bromophenyl)piperazine-1-carboxylate (compound **1b,** CAS: 352437-09-3, Vendor: Accela, 99.4 mg, 291 μmol) in 1,4-dioxane (5 mL) and water (1 mL) was added $K_2CO_3$ (121 mg, 874 μmol) and $PdCl_2$(dppf)·$CH_2Cl_2$ (24 mg, 29 μmol). The mixture was charged with $N_2$, and stirred at 100°C overnight. After being cooled to room temperature, the mixture was dried over $Na_2SO_4$, filtered and the solid was washed with EA (10 mL) twice. The combined organic phase was concentrated and purified by flash column eluting with a gradient of EA/PE (0% to 60%) to afford compound **1c** (83 mg). MS: calc'd 478 ($MH^+$), measured 478 ($MH^+$).

**Step 2: preparation of *tert*-butyl 4-[4-(4-piperidyl)phenyl]piperazine-1-carboxylate (compound 1d)**

To the mixture of *tert*-butyl 4-[4-(1-benzyloxycarbonyl-3,6-dihydro-2*H*-pyridin-4-yl)phenyl]piperazine-1-carboxylate (compound **1c,** 83 mg, 174 μmol) in MeOH (10 mL) was added $Pd(OH)_2$ (10 wt.% in carbon, 12 mg, 87 μmol). The suspension was purged with $H_2$ for 3 times, then stirred under hydrogen balloon at rt overnight. The mixture was filtered and concentrated to give the crude product compound **1d** (60 mg) which was used in the next step without further purification. MS: calc'd 346 ($MH^+$), measured 346 ($MH^+$).

**Step 3: preparation of *tert*-butyl 4-[4-[1-(1,6-dimethylpyrazolo[3,4-b]pyridin-4-yl)-4-piperidyl]phenyl]piperazine-1-carboxylate (compound 1f)**

To the mixture of 4-chloro-1,6-dimethyl-pyrazolo[3,4-b]pyridine (compound **1e,** CAS: 19867-78-8, Vendor:

PharmaBlock, 24 mg, 130 μmol) and *tert*-butyl 4-[4-(4-piperidyl)phenyl]piperazine-1-carboxylate (compound **1d,** 30 mg, 87 μmol) in 1,4-dioxane (2 mL) was added RuPhos Pd G2 (7 mg, 9 μmol) and $Cs_2CO_3$ (85 mg, 261 μmol). The mixture was charged with $N_2$, and stirred at 110 °C overnight. After being cooled to room temperature, the mixture was filtered and the solid was washed with EA (10 mL) twice. The combined organic phase was concentrated and purified by flash column eluting with a gradient of EA (with 10% MeOH)/PE (0% to 60%) to give the desired product **1f**. MS: calc'd 491 ($MH^+$), measured 491 ($MH^+$).

**Step 4: preparation of 1,6-dimethyl-4-[4-(4-piperazin-1-ylphenyl)-1-piperidyl]pyrazolo[3,4-b]pyridine**

The compound **1f** was dissolved in DCM (10 mL) and TFA (2 mL) and stirred at rt for 2 h. The mixture was concentrated and purified by reversed flash column eluting with a gradient of ACN/Water (with 0.5% TFA) (0% to 30%) to give **Example 1** (36 mg) as a yellow solid. MS: calc'd 391 ($MH^+$), measured 391 ($MH^+$). $^1H$ NMR (400 MHz, METHANOL-$d_4$) δ = $^1H$ NMR (400 MHz, METHANOL-$d_4$) δ = 8.36 (s, 1H), 7.23 - 7.18 (m, 2H), 7.01 - 6.96 (m, 2H), 6.70 (s, 1H), 4.60 (br d, *J* = 13.3 Hz, 2H), 4.07 (s, 3H), 3.56 (br t, *J* = 12.7 Hz, 2H), 3.39 - 3.33 (m, 8H), 3.01 (tt, *J* = 3.8, 11.9 Hz, 1H), 2.62 (s, 3H), 2.14 - 2.07 (m, 2H), 1.84 (dq, *J* = 3.9, 12.7 Hz, 2H).

**Example 2**

**1,6-dimethyl-4-[4-(5-piperazin-1-yl-2-pyridyl)-1-piperidyl]pyrazolo[3,4-b]pyridine**

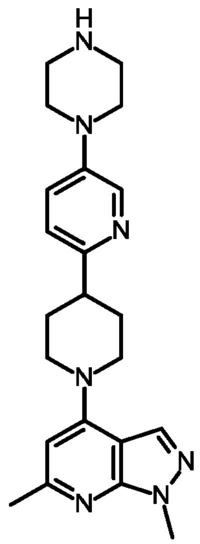

The title compound was prepared in analogy to the preparation of **Example 1** by using *tert*-butyl 4-[6-(1-benzyloxycarbonyl-3,6-dihydro-2*H*-pyridin-4-yl)-3-pyridyl]piperazine-1-carboxylate (compound **2b**) instead of compound **1c. Example 2** (44 mg) was obtained as a white solid. MS: calc'd 392 ($MH^+$), measured 392 ($MH^+$). $^1H$ NMR (400 MHz, CDCl3) δ = 8.22 (d, *J* = 2.4 Hz, 1H), 7.90 (s, 1H), 7.15 (dd, *J* = 2.6, 8.6 Hz, 1H), 7.04 (d, *J* = 8.6 Hz, 1H), 6.21 (s, 1H), 4.23 (br d, *J* = 13.1 Hz, 2H), 4.04 (s, 3H), 3.25 - 3.09 (m, 6H), 3.05 - 2.89 (m, 5H), 2.53 (s, 3H), 2.09 - 2.01 (m, 2H), 1.98 - 1.86 (m, 3H).

The compound **2b** was prepared according to the following scheme:

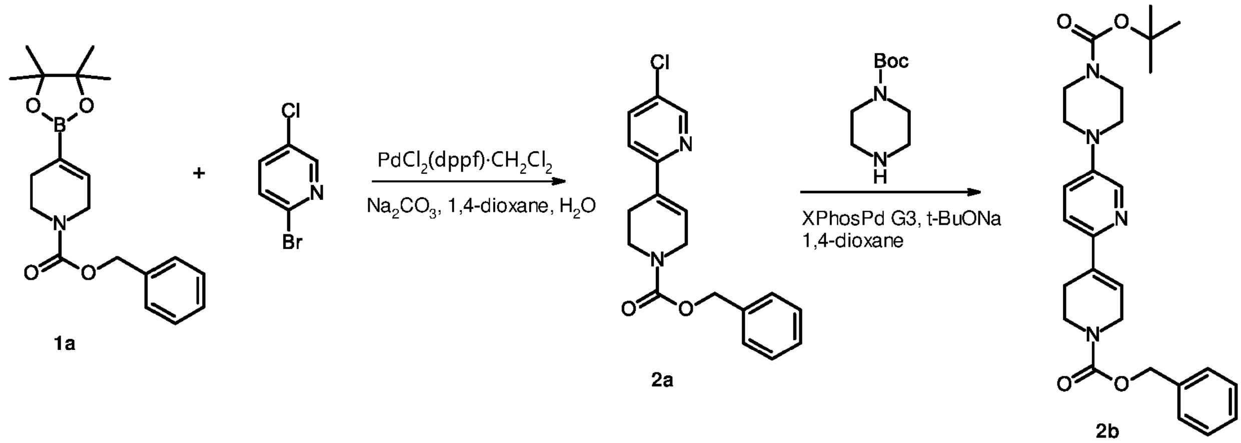


1a 2a 2b

**Step 1: preparation of benzyl 4-(5-chloro-2-pyridyl)-3,6-dihydro-2*H*-pyridine-1-carboxylate (compound 2a)**

To a solution of 2-bromo-5-chloropyridine (CAS: 40473-01-6, Vendor: Accela, 1.00 g, 5.2 mmol), benzyl 4-(4,4,5,5-tetramethyl-1,3,2-dioxaborolan-2-yl)-3,6-dihydro-2*H*-pyridine-1-carboxylate (compound **1a,** 1.80 g, 5.24 mmol) and sodium carbonate (1.20 g, 11.32 mmol) in 1,4-dioxane (10 mL) and water (1mL) was added $PdCl_2$ (dppf)·$CH_2Cl_2$ (380 mg, 0.52 mmol) under $N_2$. The mixture was stirred at 80°C for 16 h under $N_2$. After being cooled to rt, the reaction mixture was filtered and the filtrate was concentrated under reduced pressure to remove the solvent, and the residue was purified by flash column eluting with a gradient of EA/PE (1/5 to 1/0) to give compound **2a** (1.50 g) as a brown oil. MS: calc'd 329 ($MH^+$), measured 329 ($MH^+$).

**Step 2: preparation of *tert*-butyl 4-[6-(1-benzyloxycarbonyl-3,6-dihydro-2*H*-pyridin-4-yl)-3-pyridyl]piperazine-1-carboxylate (compound 2b)**

To a solution of benzyl 4-(5-chloro-2-pyridyl)-3,6-dihydro-2*H*-pyridine-1-carboxylate (compound **2a** , 500 mg, 1.52 mmol), *tert*-butyl piperazine-1-carboxylate (566 mg, 3.04 mmol) and sodium *tert*-butoxide (225 mg, 2.34 mmol) in 1,4-dioxane (5 mL) was added Xphos-Pd-G3 (129 mg, 0.15 mmol) under $N_2$. The mixture was stirred at 100 °C for 3 h under $N_2$. After being cooled to rt, the reaction mixture was filtered on celite and the filtrate was concentrated under reduced pressure to remove the solvent, and the crude was purified by flash column eluting with a gradient of EA/PE (1/5 to 1/0) to give compound **2b** (223 mg) as a yellow solid. MS: calc'd 479 ($MH^+$), measured 479 ($MH^+$).

**Example 3**

**1,6-dimethyl-4-[4-(5-piperazin-1-ylpyrazin-2-yl)-1-piperidyl]pyrazolo[3,4-b]pyridine**

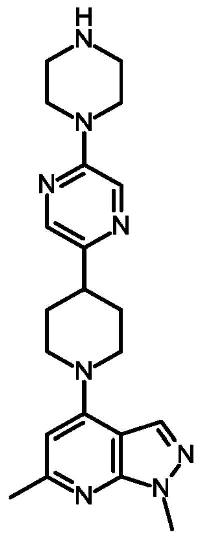

The title compound was prepared in analogy to the preparation of **Example 1** by using *tert*-butyl 4-[5-(1-benzyloxycarbonyl-3,6-dihydro-2*H*-pyridin-4-yl)pyrazin-2-yl]piperazine-1-carboxylate (compound **3b**) instead of compound **1c. Example 3** (23 mg) was obtained as a white solid. MS: calc'd 393 ($MH^+$), measured 393 ($MH^+$). $^1H$ NMR (400 MHz, CDCl3) $\delta$ = 8.09 - 8.06 (m, 1H), 8.01 - 7.98 (m, 1H), 7.93 - 7.91 (m, 1H), 6.24 - 6.22 (m, 1H), 4.29 - 4.21 (m, 2H), 4.07 - 4.04 (m, 3H), 3.55 - 3.50 (m, 4H), 3.27 - 3.18 (m, 2H), 3.02 - 2.97 (m, 4H), 2.97 - 2.89 (m, 1H), 2.57 - 2.54 (m, 3H), 2.07 - 1.93 (m, 4H).

The compound **3b** was prepared according to the following scheme:

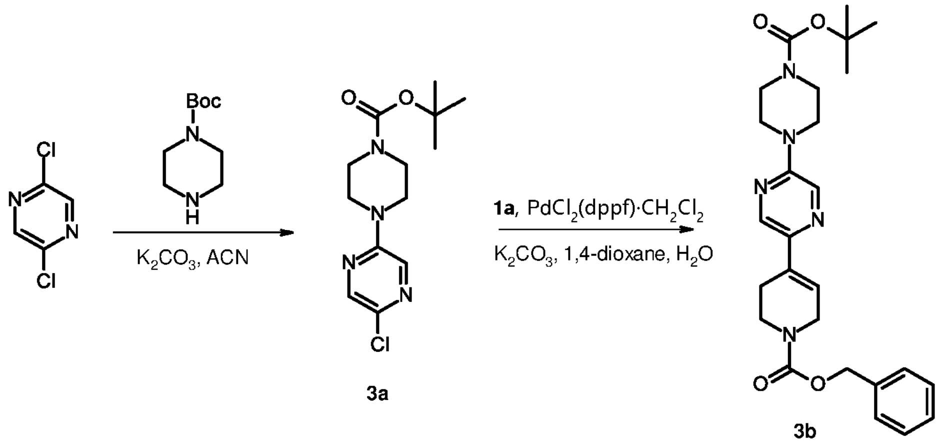


**Step 1: preparation of *tert*-butyl 4-(5-chloropyrazin-2-yl)piperazine-1-carboxylate (compound 3a)**

To a solution of 2,5-dichloropyrazine (CAS: 19745-07-4, Vendor: Accela, 2.00 g, 13.42 mmol) and *tert*-butyl piperazine-1-carboxylate (2.75 g, 14.77 mmol) in ACN (20 mL) was added potassium carbonate (2.78 g, 20.14 mmol). The mixture was stirred at 80 °C for 16 h. After being cooled to rt, the reaction mixture was filtered and the filtrate was concentrated under reduced pressure to remove the solvent, and the residue was purified by flash column eluting with a gradient of PE/EA (10/1 to 3/1) to give compound **3a** (1.50 g, 5.02 mmol) as a light yellow solid. MS: calc'd 299 ($MH^+$), measured 299 ($MH^+$).

**Step 2: preparation of *tert*-butyl 4-[5-(1-benzyloxycarbonyl-3,6-dihydro-2*H*-pyridin-4-yl)pyrazin-2-yl]piperazine-1-carboxylate (compound 3b)**

To a mixture of *tert*-butyl 4-(5-chloropyrazin-2-yl)piperazine-1-carboxylate (compound **3a,** 1.45 g, 4.85 mmol) and benzyl 4-(4,4,5,5-tetramethyl-1,3,2-dioxaborolan-2-yl)-3,6-dihydro-2*H*-pyridine-1-carboxylate (compound **1a,** 1.83 g, 5.34 mmol) in 1,4-dioxane (10 mL) and water (1 mL) was added $K_2CO_3$ (1.34 g, 9.71 mmol) and $PdCl_2(dppf)\cdot CH_2Cl_2$ (198 mg, 0.24 mmol). The flask was degassed and purged with $N_2$ for four times. The mixture was stirred at 90°C for 16 h

under $N_2$ atmosphere. After being cooled to rt, the mixture was diluted by water (10 mL), extracted by EA (20 mL) three times. The combined organic layer was washed by brine (50 mL), dried over $Na_2SO_4$, filtered and concentrated to give the crude product which was purified by flash column eluting with a gradient of PE/EA (5/1 to 3/1) to give compound **3b** (1.50 g) as a yellow solid. MS: calc'd 480 ($MH^+$), measured 480 ($MH^+$).

**Example 4**

**1,6-dimethyl-4-[4-(5-piperazin-1-ylpyrimidin-2-yl)-1-piperidyl]pyrazolo[3,4-b]pyridine**

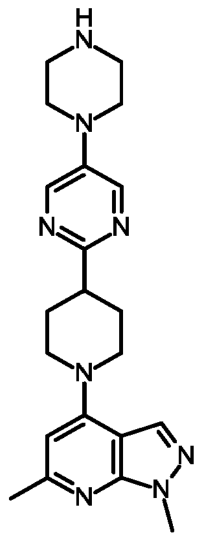

The title compound was prepared in analogy to the preparation of **Example 2** by using 5-bromo-2-iodopyrimidine (CAS: 183438-24-6, Vendor: Accela) instead of 2-bromo-5-chloropyridine. **Example 4** (3.5 mg) was obtained as a white solid. MS: calc'd 393 ($MH^+$), measured 393 ($MH^+$). $^1H$ NMR (400 MHz, METHANOL-d4) $\delta$ = 8.40 (s, 2H), 8.08 (s, 1H), 6.40 (s, 1H), 5.02 - 4.90 (m, 2H), 4.33 (br d, *J* = 13.2 Hz, 2H), 3.99 (s, 3H), 3.26 - 3.20 (m, 4H), 3.18 - 3.11 (m, 1H), 3.03 - 2.95 (m, 4H), 2.52 (s, 3H), 2.13 - 1.94 (m, 4H).

**Example 5**

**1-methyl-4-[4-(3-methyl-5-piperazin-1-yl-2-pyridyl)-1-piperidyl]pyrazolo[3,4-b]pyridine**

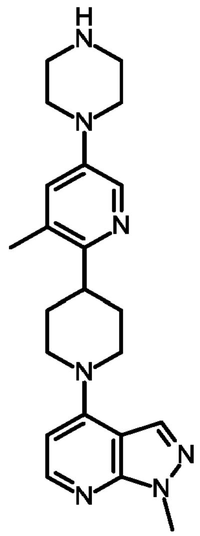

The title compound was prepared in analogy to the preparation of **Example 2** by using 2-bromo-5-chloro-3-methyl-pyridine (CAS: 65550-77-8, Vendor: Accela) and 4-chloro-1-methyl-pyrazolo[3,4-b]pyridine (CAS: 1268520-92-8, Vendor: PharmaBlock) instead of 2-bromo-5-chloropyridine and compound **1e. Example 5** (29 mg) was obtained as a white solid. MS: calc'd 392 ($MH^+$), measured 392 ($MH^+$). $^1H$ NMR (400 MHz, METHANOL-d4) $\delta$ = 8.16 (s, 1H), 8.10 (d, *J* = 5.9 Hz, 1H), 7.96 (d, *J* = 2.7 Hz, 1H), 7.21 (d, *J* = 2.7 Hz, 1H), 6.50 (d, *J* = 5.9 Hz, 1H), 4.41 (br d, *J* = 13.7 Hz, 2H), 4.00 (s, 3H), 3.41 - 3.33 (m, 2H), 3.29 - 3.22 (m, 1H), 3.18 - 3.10 (m, 4H), 3.04 - 2.93 (m, 4H), 2.40 (s, 3H), 2.10 - 1.95 (m, 2H), 1.88 (br dd, *J* = 1.5, 12.5 Hz, 2H).

**Example 6**

**1-methyl-4-[4-(4-methyl-6-piperazin-1-yl-3-pyridyl)-1-piperidyl]pyrazolo[3,4-b]pyridine**

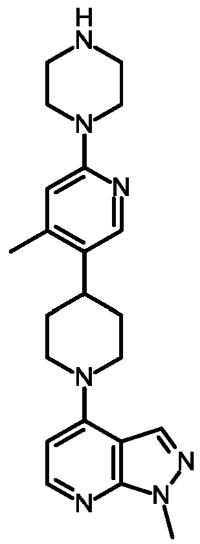

The title compound was prepared in analogy to the preparation of **Example 1** by using 4-chloro-1-methyl-pyrazolo[3,4-b]pyridine (CAS: 1268520-92-8, Vendor: PharmaBlock) and *tert*-butyl 4-(5-bromo-4-methyl-2-pyridyl)piperazine-1-carboxylate (CAS: 944582-92-7, Vendor: Bide Pharmatech) instead of compound **1e** and compound **1b. Example 6** (35 mg) was obtained as a light yellow solid. MS: calc'd 392 ($MH^+$), measured 392 ($MH^+$). $^1H$ NMR (400 MHz, METHANOL-$d_4$) $\delta$ = 8.45 (s, 1H), 8.06 (d, *J* = 7.3 Hz, 1H), 7.91 (s, 1H), 7.21 (s, 1H), 6.86 (d, *J* = 7.5 Hz, 1H), 4.70 - 4.62 (m, 2H), 4.08 (s, 3H), 3.94 - 3.84 (m, 4H), 3.65 (br t, *J* = 12.1 Hz, 2H), 3.44 - 3.36 (m, 4H), 3.35 - 3.24 (m, 1H), 2.56 (s, 3H), 2.14 (br d, *J* = 12.3 Hz, 2H), 1.90 (dq, *J* = 3.5, 12.6 Hz, 2H).

**Example 7**

**1,6-dimethyl-4-[4-(3-methyl-5-piperazin-1-yl-2-pyridyl)-1-piperidyl]pyrazolo[3,4-b]pyridine**

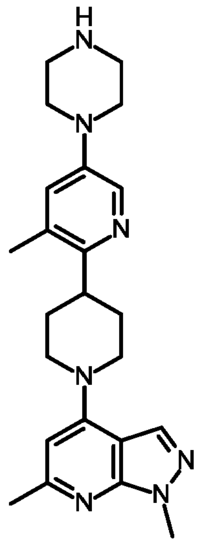

The title compound was prepared in analogy to the preparation of **Example 2** by using 2-bromo-5-chloro-3-methyl-pyridine (CAS: 65550-77-8, Vendor: Accela) instead of 2-bromo-5-chloropyridine. **Example 7** (61 mg) was obtained as a white solid. MS: calc'd 406 ($MH^+$), measured 406 ($MH^+$). $^1H$ NMR (400 MHz, METHANOL-d4) $\delta$ = 8.07 (s, 1H), 7.96 (d, *J* = 2.9 Hz, 1H), 7.21 (d, *J* = 2.7 Hz, 1H), 6.40 (s, 1H), 4.37 (br d, *J* = 13.4 Hz, 2H), 3.99 (s, 3H), 3.35 (br d, *J* = 2.0 Hz, 1H), 3.29 - 3.21 (m, 2H), 3.15 (br dd, *J* = 3.9, 6.1 Hz, 4H), 3.02 - 2.94 (m, 4H), 2.52 (s, 3H), 2.40 (s, 3H), 2.08 - 1.96 (m, 2H), 1.89 - 1.80 (m, 2H).

**Example 8**

**1,6-dimethyl-4-[4-(4-methyl-6-piperazin-1-yl-3-pyridyl)-1-piperidyl]pyrazolo[3,4-b]pyridine**

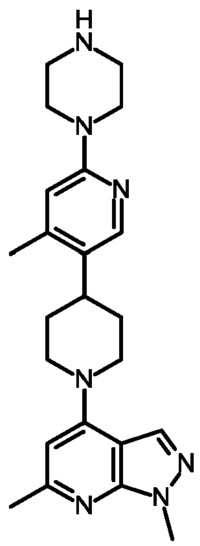

The title compound was prepared in analogy to the preparation of **Example 1** by using 4-bromo-1,6-dimethyl-pyrazolo[3,4-b]pyridine (CAS: 1783407-55-5, Vendor: Accela) and *tert*-butyl 4-(5-bromo-4-methyl-2-pyridyl)piperazine-1-carboxylate (CAS: 944582-92-7, Vendor: Bide Pharmatech) instead of compound **1e** and compound **1b**. **Example 8** (32 mg) was obtained as a yellow solid. MS: calc'd 406 ($MH^+$), measured 406 ($MH^+$). $^1H$ NMR (400 MHz, METHANOL-$d_4$) $\delta$ = 8.27 (s, 1H), 7.81 (s, 1H), 7.07 (s, 1H), 6.62 (s, 1H), 4.54 (br d, *J* = 13.4 Hz, 2H), 3.98 (s, 3H), 3.82 - 3.73 (m, 4H), 3.51 (br t, *J* = 12.6 Hz, 2H), 3.35 - 3.25 (m, 4H), 3.19 - 3.12 (m, 1H), 2.53 (s, 3H), 2.45 (s, 3H), 2.03 (br d, *J* = 12.3 Hz, 2H), 1.77 (dq, *J* = 3.5, 12.6 Hz, 2H).

**Example 9**

**1,6-dimethyl-4-[4-(4-methyl-5-piperazin-1-yl-2-pyridyl)-1-piperidyl]pyrazolo[3,4-b]pyridine**

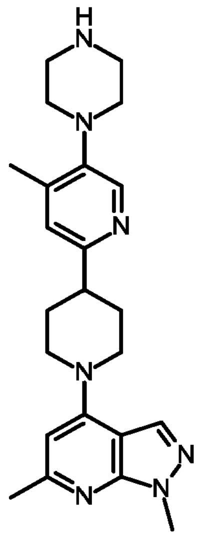

The title compound was prepared in analogy to the preparation of **Example** 3 by using 5-bromo-2-chloro-4-methylpyridine (CAS: 778611-64-6, Vendor: Accela) instead of 2,5-dichloropyrazine, and replacing $S_NAr$ reaction ($K_2CO_3$, CAN) with Buchwald coupling reaction (XantPhos, $Pd_2(dba)_3$, t-BuONa, toluene) in the step 1. **Example 9** (61 mg) was obtained as a white solid. MS: calc'd 406 ($MH^+$), measured 406 ($MH^+$). $^1H$ NMR (400 MHz, METHANOL-d4) $\delta$ = 8.09 (d, *J* = 3.4 Hz, 2H), 7.18 (s, 1H), 6.41 (s, 1H), 4.37 (br d, *J* = 13.2 Hz, 2H), 3.99 (s, 3H), 3.30 - 3.23 (m, 2H), 3.06 - 3.01 (m, 4H), 3.02 - 2.99 (m, 1H), 3.00 - 2.93 (m, 4H), 2.52 (s, 3H), 2.33 (s, 3H), 2.05 - 1.97 (m, 2H), 1.97 - 1.85 (m, 2H).

**Example 10**

**1,6-dimethyl-4-[4-(2-methyl-6-piperazin-1-yl-3-pyridyl)-1-piperidyl]pyrazolo[3,4-b]pyridine**

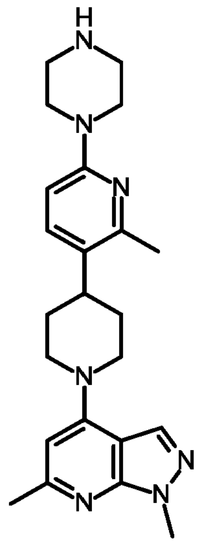

The title compound was prepared in analogy to the preparation of **Example 3** by using 3-bromo-6-fluoro-2-methylpyridine (CAS: 375368-83-5, Vendor: Accela) instead of 2,5-dichloropyrazine, and replacing $S_NAr$ reaction condition ($K_2CO_3$, CAN) with (DIPEA, DMF) in the step 1. **Example 10** (7 mg) was obtained as a white solid. MS: calc'd 406 ($MH^+$), measured 406 ($MH^+$). $^1H$ NMR (400MHz, METHANOL-d4) $\delta$ = 8.10 (s, 1H), 7.42 (d, *J* = 8.8 Hz, 1H), 6.61 (d, *J* = 8.6 Hz, 1H), 6.43 (s, 1H), 4.38 (br d, *J* = 13.1 Hz, 2H), 4.01 (s, 3H), 3.49 - 3.43 (m, 4H), 3.32 - 3.26 (m, 2H), 3.11 - 3.01 (m, 1H), 2.99 - 2.92 (m, 4H), 2.54 (s, 3H), 2.50 (s, 3H), 1.97 - 1.89 (m, 2H), 1.86 - 1.75 (m, 2H).

**Example 11**

**1,6-dimethyl-4-[4-(4-methyl-2-piperazin-1-yl-pyrimidin-5-yl)-1-piperidyl]pyrazolo[3,4-b]pyridine**

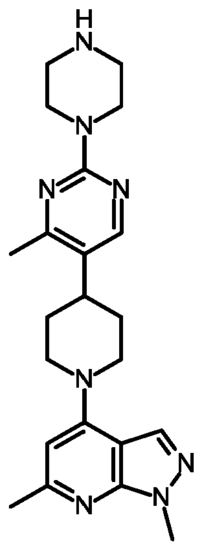

The title compound was prepared in analogy to the preparation of **Example 3** by using 5-bromo-2-chloro-4-methylpyrimidine (CAS: 633328-95-7, Vendor: Accela) instead of 2,5-dichloropyrazine. **Example 11** (34 mg) was obtained as a white solid. MS: calc'd 407 ($MH^+$), measured 407 ($MH^+$). $^1H$ NMR (400 MHz, CDCl3) $\delta$ = 8.10 (s, 1H), 7.93 (s, 1H), 6.24 (s, 1H), 4.27 (br d, *J* = 12.8 Hz, 2H), 4.07 (s, 3H), 3.81 - 3.74 (m, 4H), 3.18 (dt, *J* = 2.6, 12.5 Hz, 2H), 2.95 - 2.81 (m, 5H), 2.57 (s, 3H), 2.43 (s, 3H), 1.97 - 1.82 (m, 4H).

**Example 12**

**1,6-dimethyl-4-[4-(3-methyl-5-piperazin-1-yl-pyrazin-2-yl)-1-piperidyl]pyrazolo[3,4-b]pyridine**

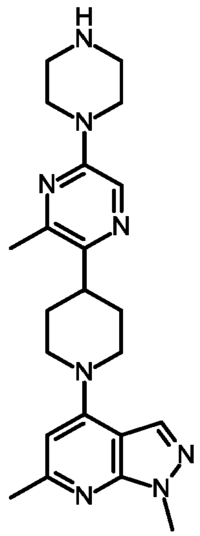

The title compound was prepared in analogy to the preparation of **Example 3** by using 2,5-dichloro-3-methylpyrazine (CAS: 107378-41-6, Vendor: Bide) instead of 2,5-dichloropyrazine. **Example 12** (16 mg) was obtained as a white solid. MS: calc'd 407 ($MH^+$), measured 407 ($MH^+$). $^1H$ NMR (400MHz, METHANOL-d4) $\delta$ = 8.09 (s, 1H), 7.92 (s, 1H), 6.41 (s, 1H), 4.37 (br d, *J* = 13.3 Hz, 2H), 4.00 (s, 3H), 3.59 - 3.50 (m, 4H), 3.39 - 3.34 (m, 1H), 3.32 - 3.28 (m, 1H), 3.28 - 3.17 (m, 1H), 3.00 - 2.88 (m, 4H), 2.54 (s, 3H), 2.51 (s, 3H), 2.07 - 1.94 (m, 2H), 1.93 - 1.82 (m, 2H).

**Example 13**

**4-[4-(3-ethyl-5-piperazin-1-yl-2-pyridyl)-1-piperidyl]-1,6-dimethyl-pyrazolo[3,4-b]pyridine**

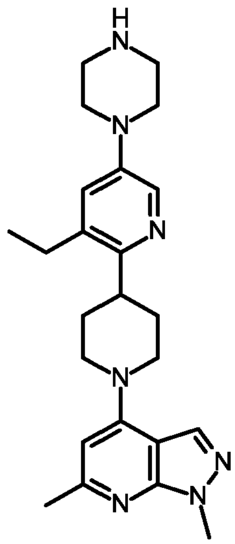

The title compound was prepared in analogy to the preparation of **Example 1** by using *tert-butyl* 4-[6-(1-benzyloxycarbonyl-3,6-dihydro-2*H*-pyridin-4-yl)-5-vinyl-3-pyridyl]piperazine-1-carboxylate (compound **13c**) instead of compound **1c. Example 13** (5 mg) was obtained as a white solid. MS: calc'd 420 ($MH^+$), measured 420 ($MH^+$). $^1H$ NMR (400MHz, METHANOL-d4) $\delta$ = 8.10 (s, 1H), 7.99 (d, *J* = 2.8 Hz, 1H), 7.22 (d, *J* = 2.8 Hz, 1H), 6.42 (s, 1H), 4.39 (br d, *J* = 13.4 Hz, 2H), 4.01(s, 3H), 3.39 - 3.34 (m, 1H), 3.32 - 3.23 (m, 2H),3.20 - 3.12 (m, 4H), 3.04 - 2.95 (m, 4H), 2.77 (q, *J* = 7.5 Hz, 2H),

2.54 (s, 3H), 2.18 - 2.02 (m, 2H), 1.85 (br d, *J* = 11.1 Hz, 2H), 1.28 (t, *J* = 7.5 Hz, 3H).

The compound **13c** was prepared according to the following scheme:

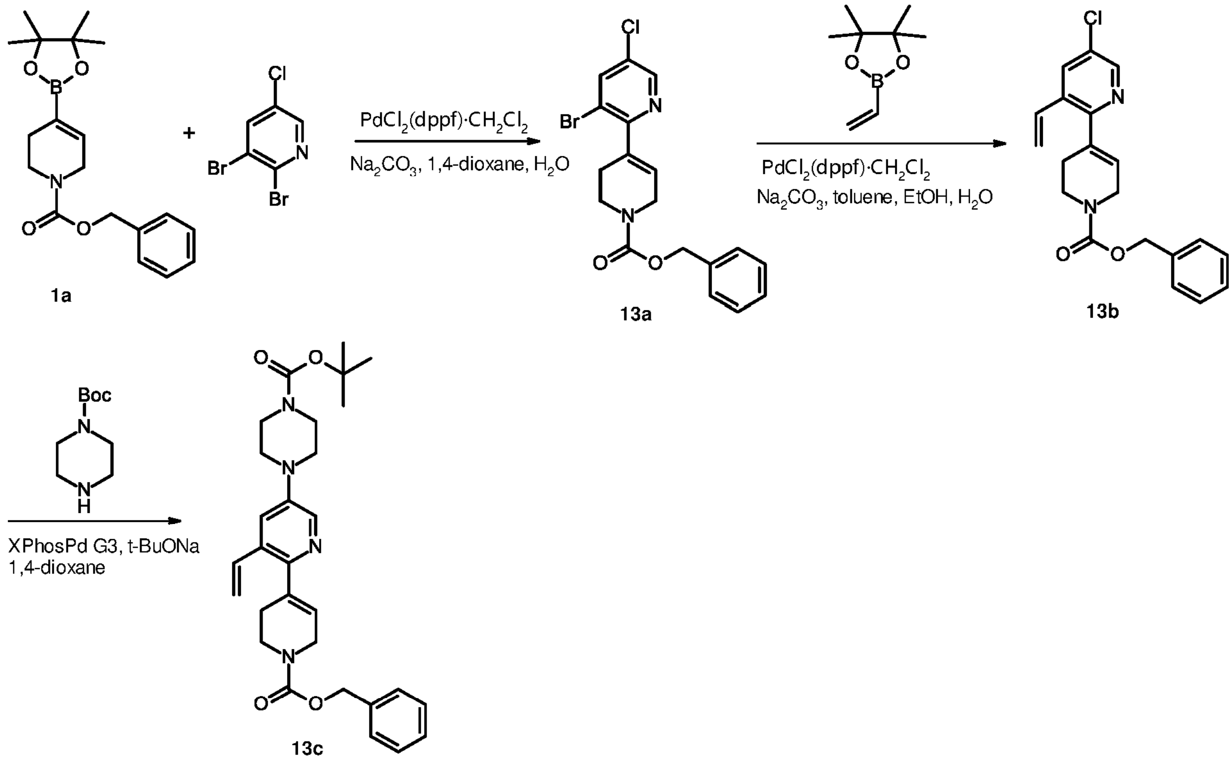


1a 13a 13b 13c

### Step 1: preparation of benzyl 4-(3-bromo-5-chloro-2-pyridyl)-3,6-dihydro-2*H*-pyridine-1-carboxylate (compound 13a)

To the mixture of benzyl 4-(4,4,5,5-tetramethyl-1,3,2-dioxaborolan-2-yl)-3,6-dihydro-2*H*-pyridine-1-carboxylate (compound **1a,** 2.53 g, 7.37 mmol) and 2,3-dibromo-5-chloropyridine (CAS: 137628-17-2, Vendor: Accela, 2.00 g, 7.37 mmol) in 1,4-dioxane (10 mL) and water (1 mL) was added $PdCl_2$(dppf).$CH_2Cl_2$ (301 mg, 0.37 mmol) and $Na_2CO_3$ (1.56 g, 14.74 mmol). The mixture was stirred at 80°C for 0.5 h under $N_2$ atmosphere. After being cooled to rt, the mixture was filtered and the solid was washed with 1,4-dioxane (15mL) three times and concentrated. The residue was purified by flash column eluting with a gradient of PE/EA (3/1) to afford compound **13a** (680 mg, 1.67 mmol) as a light yellow solid. MS: calc'd 407 ($MH^+$), measured 407 ($MH^+$).

### Step 2: preparation of benzyl 4-(5-chloro-3-vinyl-2-pyridyl)-3,6-dihydro-2*H*-pyridine-1-carboxylate (compound 13b)

To the mixture of benzyl 4-(3-bromo-5-chloro-2-pyridyl)-3,6-dihydro-2*H*-pyridine-1-carboxylate (compound **13a,** 100 mg, 0.25 mmol), vinylboronic acid pinacol ester (94 mg, 0.61 mmol) and $Na_2CO_3$ (20 mg, 0.490 mmol) in toluene (1 mL), ethanol (0.5 mL) and water (0.5 mL) was added $PdCl_2$(dppf).$CH_2Cl_2$ (20 mg, 0.02 mmol), the mixture was stirred at 100°C for 1 h under the $N_2$ atmosphere. This reaction was repeated four times (each for 100 mg of compound **13c**). The combined reaction mixture was filtered and washed with EtOH. The filtrate was concentrated. The residue was purified by flash column eluting with a gradient of PE/EA (5/1 to 0/1) to give compound **13b** (260 mg) as a light yellow oil. MS: calc'd 355 ($MH^+$), measured 355 ($MH^+$).

### Step 3: preparation of *tert*-butyl 4-[6-(1-benzyloxycarbonyl-3,6-dihydro-2*H*-pyridin-4-yl)-5-vinyl-3-pyridyl]piperazine-1-carboxylate (compound 13c)

To the mixture of *tert*-butyl piperazine-1-carboxylate (105 mg, 0.56 mmol) and benzyl 4-(5-chloro-3-vinyl-2-pyridyl)-3,6-dihydro-2*H*-pyridine-1-carboxylate (compound **13b,** 100 mg, 0.28 mmol) in 1,4-dioxane (2.5 mL) was added Xphos-Pd-G3 (24 mg, 0.03 mmol) and *t*-BuONa (67 mg, 0.70 mmol). After being stirred at 100°C for 1.5 h under $N_2$, the reaction mixture was combined with another same batch and worked up, and then the mixture was filtered, washed with

EtOH and concentrated. The residue was purified by flash column eluting with a gradient of PE/EA (1/0 to 1/8) to afford compound **13c** (90 mg) as a light yellow solid. MS: calc'd 505 ($MH^+$), measured 505 ($MH^+$).

**Example 14**

**4-[4-(3-methoxy-5-piperazin-1-yl-2-pyridyl)-1-piperidyl]-1,6-dimethyl-pyrazolo[3,4-b]pyridine**

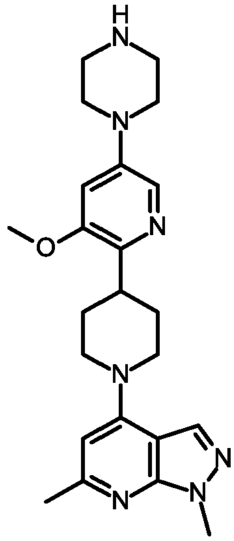

The title compound was prepared in analogy to the preparation of **Example 9** by using 5-bromo-2-chloro-3-methoxy-pyridine (CAS: 286947-03-3, Vendor: Accela) instead of 5-bromo-2-chloro-4-methylpyridine. **Example 14** (37 mg) was obtained as a white solid. MS: calc'd 422 ($MH^+$), measured 422 ($MH^+$). $^1H$ NMR (400MHz, METHANOL-d4) $\delta$ = 8.09 (s, 1H), 7.70 (d, *J* = 2.3 Hz, 1H), 6.99 (d, *J* = 2.3 Hz, 1H), 6.41 (s, 1H), 4.37 (br d, *J* = 13.2 Hz, 2H), 4.00 (s, 3H), 3.89 (s, 3H), 3.44 (tt, *J* = 3.9, 11.7 Hz, 1H), 3.32 - 3.25 (m, 2H), 3.20 (dd, *J* = 4.0, 6.2 Hz, 4H), 3.03 - 2.94 (m, 4H), 2.54 (s, 3H), 2.07 - 1.95 (m, 2H), 1.94 - 1.85 (m, 2H).

**Example 15**

**(3*S*,4*R*)-1-[6-[1-(1,6-dimethylpyrazolo[3,4-b]pyridin-4-yl)-4-piperidyl]-5-methyl-3-pyridyl]-3-fluoro-piperidin-4-amine**

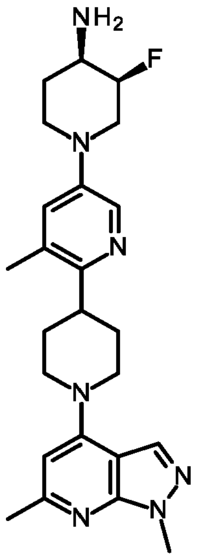

The title compound was prepared according to the following scheme:

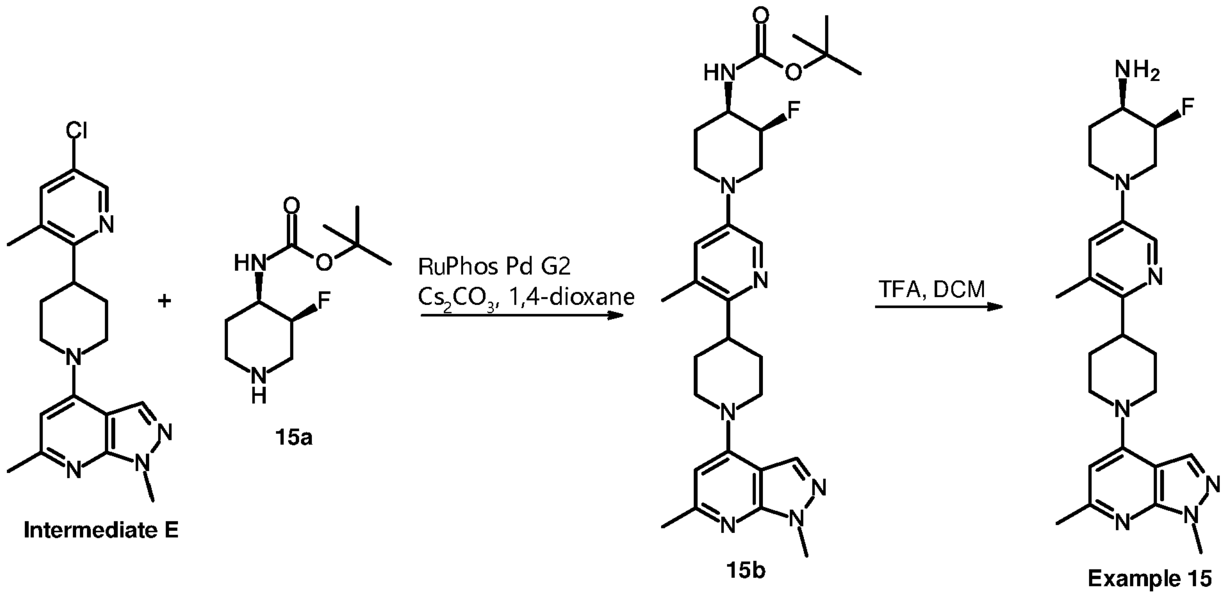


Intermediate E

15a

15b

Example 15

**Step 1: preparation of *tert*-butyl *N*-[(3*S*,4*R*)-1-[6-[1-(1,6-dimethylpyrazolo[3,4-b]pyridin-4-yl)-4-piperidyl]-5-methyl-3-pyridyl]-3-fluoro-4-piperidyl]carbamate (compound 15b)**

To the mixture of 4-[4-(5-chloro-3-methyl-2-pyridyl)-1-piperidyl]-1,6-dimethyl-pyrazolo[3,4-b]pyridine (**Intermediate E**, 50 mg, 0.14 mmol) and *tert*-butyl *N*-[(3*S*,4*R*)-3-fluoro-4-piperidyl]carbamate (compound **15a,** CAS: 1434126-99-4, Vendor: PharmaBlock, 40 mg, 0.18 mmol) in 1,4-dioxane was added RuPhos Pd G2 (11 mg, 0.014 mmol) and cesium carbonate (92 mg, 0.28 mmol). The mixture was heated to 110 °C overnight. After being cooled to rt, the solid was filtered off and washed with EA (10 mL) twice. The combined organic layer was concentrated and purified by flash column eluting with a gradient of EA(with 10% MeOH)/PE (0 to 80%) to give compound **15b** which was directly used in the next step. MS: calc'd 538 ($MH^+$), measured 538 ($MH^+$).

**Step 2: preparation of (3*S*,4*R*)-1-[6-[1-(1,6-dimethylpyrazolo[3,4-b]pyridin-4-yl)-4-piperidyl]-5-methyl-3-pyridyl]-3-fluoro-piperidin-4-amine (Example 15)**

The mixture of compound **15b** in DCM (5 mL) and TFA (2 mL) was stirred at rt for 2h. The mixture was concentrated and purified by reversed flash column eluting with a gradient of ACN/ Water (with 0.05% TFA) (0 to 30%) to give **Example 15** (56 mg) as a white solid. MS: calc'd 438 ($MH^+$), measured 438 ($MH^+$). $^1H$ NMR (400 MHz, METHANOL-

$d_4$) δ = 8.37 (s, 1H), 8.15 (d, *J* = 2.9 Hz, 1H), 8.02 (d, *J* = 2.7 Hz, 1H), 6.74 (s, 1H), 5.08 (d, *J* = 49.2 Hz, 1H), 4.76 - 4.61 (m, 2H), 4.48 - 4.34 (m, 1H), 4.16 - 4.03 (m, 4H), 3.76 - 3.59 (m, 4H), 3.43 - 3.25 (m, 1H), 3.24 - 3.09 (m, 1H), 2.64 (s, 3H), 2.59 (s, 3H), 2.22 - 2.09 (m, 4H), 2.09 - 2.00 (m, 2H).

**Example 16**

**4-[4-[5-[(3*R*)-3-(methoxymethyl)piperazin-1-yl]-3-methyl-2-pyridyl]-1-piperidyl]-1,6-dimethyl-pyrazolo[3,4-b]pyridine**

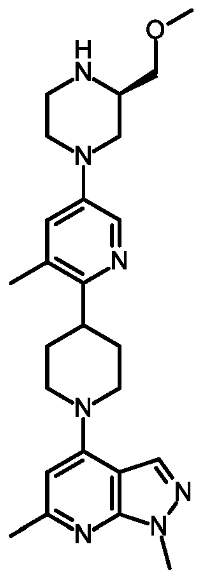

The title compound was prepared in analogy to the preparation of **Example 15** by using tert-butyl (2R)-2-(methoxymethyl)piperazine-1-carboxylate (CAS: 1023301-73-6, Vendor: PharmaBlock) instead of compound **15a. Example 16** (58 mg) was obtained as a white solid. MS: calc'd 450 ($MH^+$), measured 450 ($MH^+$). $^1H$ NMR (400 MHz, METHANOL-$d_4$) δ = 8.37 (s, 1H), 8.13 (d, *J* = 2.8 Hz, 1H), 7.73 (d, *J* = 2.6 Hz, 1H), 6.73 (s, 1H), 4.70 - 4.62 (m, 2H), 4.08 (s, 3H), 3.98 - 3.90 (m, 2H), 3.72 - 3.57 (m, 6H), 3.52 - 3.46 (m, 1H), 3.45 (s, 3H), 3.36 - 3.27 (m, 1H), 3.20 - 3.06 (m, 2H), 2.63 (s, 3H), 2.53 (s, 3H), 2.14 - 2.03 (m, 4H).

**Example 17**

**(3*S*,4*S*)-1-[6-[1-(1,6-dimethylpyrazolo[3,4-b]pyridin-4-yl)-4-piperidyl]-5-methyl-3-pyridyl]-3-methoxy-piperidin-4-amine**

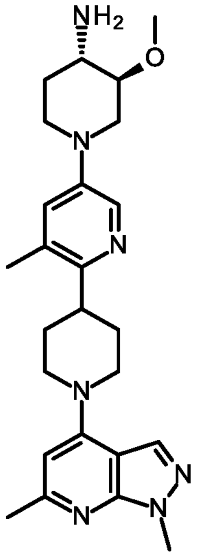

The title compound was prepared in analogy to the preparation of **Example 15** by using *tert-butyl* N-[(3S,4S)-3-methoxy-4-piperidyl]carbamate (CAS: 907544-19-8, Vendor: PharmaBlock) instead of compound 15a. **Example 17** (50 mg) was obtained as a white solid. MS: calc'd 450 ($MH^+$), measured 450 ($MH^+$). $^1H$ NMR (400 MHz, METHANOL-$d_4$) $\delta$ = 8.37 (s, 1H), 8.17 (d, *J* = 2.9 Hz, 1H), 8.03 (d, *J* = 2.8 Hz, 1H), 6.75 (s, 1H), 4.74 - 4.65 (m, 2H), 4.39 - 4.29 (m, 1H), 4.08 (s, 3H), 4.03 - 3.91 (m, 1H), 3.75 - 3.60 (m, 3H), 3.55 (s, 3H), 3.41 - 3.32 (m, 1H), 3.26 - 3.14 (m, 1H), 3.00 (dt, *J* = 2.7, 12.8 Hz, 1H), 2.72 (dd, *J* = 10.1, 12.5 Hz, 1H), 2.64 (s, 3H), 2.60 (s, 3H), 2.21 - 2.09 (m, 5H), 1.79 (dq, *J* = 4.6, 12.5 Hz, 1H).

**Example 18 and Example 19**

**1-methyl-4-[*cis*-3-methyl-4-(6-piperazin-l-yl-3-pyridyl)-1-piperidyl]pyrazolo[3,4-b]pyridine (Example 18) and 1-methyl-4-[*trans*-3-methyl-4-(6-piperazin-1-yl-3-pyridyl)-1-piperidyl]pyrazolo[3,4-b]pyridine (Example 19)**

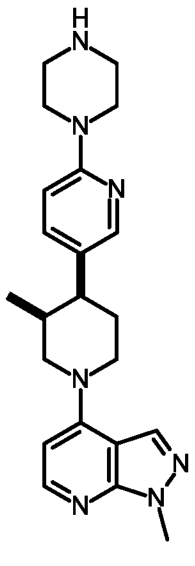

(Example 18)

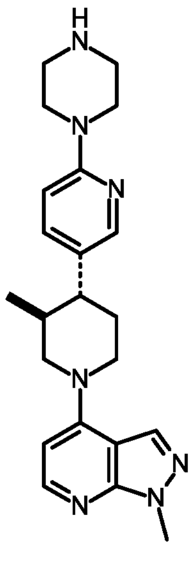

(Example 19)

The title compounds were prepared in analogy to the preparation of **Example 15** by using *tert-butyl* piperazine-1-carboxylate and **Intermediate** C instead of compound **15a** and **Intermediate** E. The **Example 18** and **Example 19** were separated by prep-HPLC.

**Example 18** (6 mg) was obtained as a light yellow solid. MS: calc'd 392 ($MH^+$), measured 392 ($MH^+$). $^1H$ NMR (400 MHz, METHANOL-$d_4$) $\delta$ = 8.50 (s, 1H), 8.07 - 7.99 (m, 2H), 7.69 (dd, *J* = 2.3, 8.9 Hz, 1H), 7.05 (d, *J* = 8.9 Hz, 1H), 6.86 (d, *J* = 7.6 Hz, 1H), 4.70 (br d, *J* = 13.7 Hz, 1H), 4.54 (br d, *J* = 13.4 Hz, 1H), 4.07 (s, 3H), 3.85 - 3.77 (m, 5H), 3.68 - 3.54 (m,

1H), 3.39 - 3.33 (m, 5H), 2.46 - 2.28 (m, 2H), 1.98 (br dd, *J* = 2.8, 13.8 Hz, 1H), 0.72 (d, *J* = 7.0 Hz, 3H).

**Example 19** (8 mg) was obtained as a light yellow solid. MS: calc'd 392 ($MH^+$), measured 392 ($MH^+$). $^1H$ NMR (400 MHz, METHANOL-$d_4$) $\delta$ = 8.41 (s, 1H), 8.08 - 8.02 (m, 2H), 7.70 (dd, *J* = 2.3, 9.0 Hz, 1H), 7.07 (d, *J* = 9.0 Hz, 1H), 6.87 (d, *J* = 7.5 Hz, 1H), 4.65 (br d, *J* = 13.8 Hz, 1H), 4.55 (br d, *J* = 12.8 Hz, 1H), 4.08 (s, 3H), 3.85 - 3.78 (m, 4H), 3.66 - 3.53 (m, 1H), 3.38 - 3.33 (m, 4H), 3.28 - 3.23 (m, 1H), 2.65 (dt, *J* = 4.0, 11.4 Hz, 1H), 2.09 - 1.86 (m, 3H), 0.89 (d, *J* = 6.5 Hz, 3H). The relative configuration of the two molecules were confirmed by 2D-NMR.

**Example 20 and Example 21**

***1,6-dimethyl-4-[cis-3-methyl-4-(4-methyl-6-piperazin-1-yl-3-pyridyl)-1-*piperidyl]pyrazolo[3,4-b]pyridine (Example 20) and 1,6-dimethyl-4-[trans-3-methyl-4-(4-methyl-6-piperazin-1-yl-3-pyridyl)-1-piperidyl]pyrazolo[3,4-b] pyridine (Example 21)**

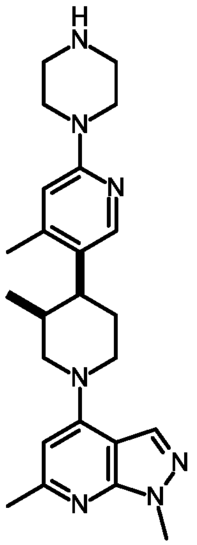

(Example 20)

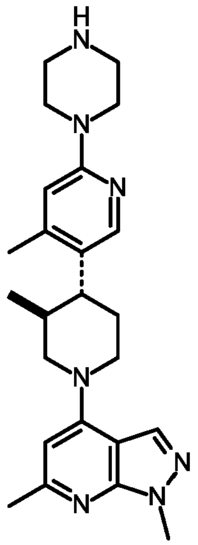

(Example 21)

The title compounds were prepared according to the following scheme:

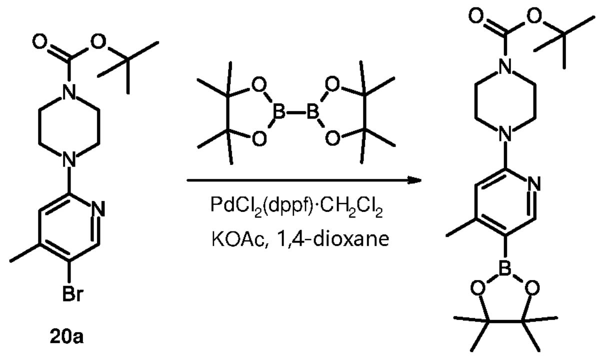


20a

20b

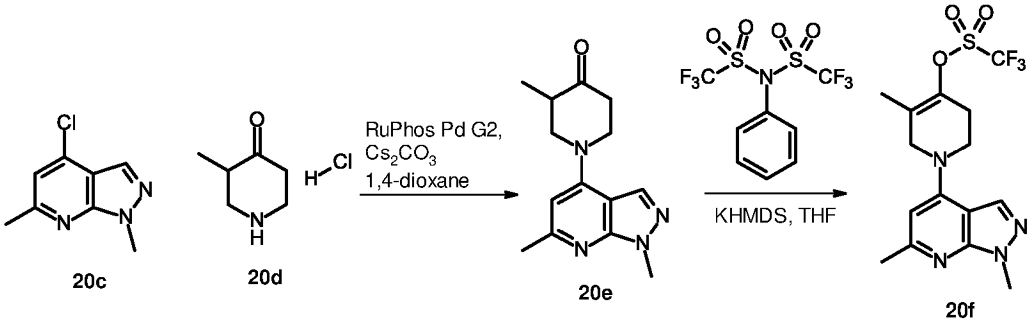


20c 20d 20e 20f

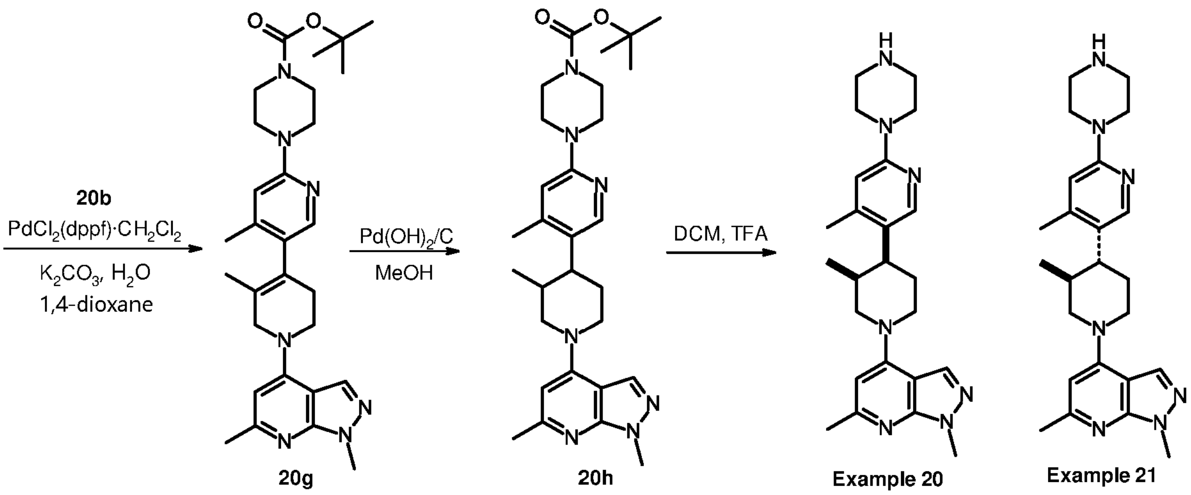


20g 20h Example 20 Example 21

**Step 1: preparation of *tert*-butyl 4-[4-methyl-5-(4,4,5,5-tetramethyl-1,3,2-dioxaborolan-2-yl)-2-pyridyl]piperazine-1-carboxylate (compound 20b)**

To the mixture of *tert*-butyl 4-(5-bromo-4-methylpyridin-2-yl)piperazine-1-carboxylate (compound 20a, CAS: 944582-92-7, Vendor: Bide Pharmatech, 100 mg, 281 μmol) and 4,4,4',4',5,5,5',5'-octamethyl-2,2'-bi(1,3,2-dioxaborolane) (107 mg, 421 μmol) in 1,4-dioxane (10 mL) was added potassium acetate (83 mg, 842 μmol) and $PdCl_2$ (dppf)·$CH_2Cl_2$ (23 mg, 28 μmol). The mixture was charged with $N_2$, and stirred at 110 °C overnight. After being cooled to rt, the mixture was filtered off and the solid was washed with EA (10 mL) twice. The combined organic layer was concentrated to give the crude product compound 20b (113 mg), which was used in the next step without further purification. MS: calc'd 404 ($MH^+$), measured 404 ($MH^+$).

**Step 2: preparation of 1-(1,6-dimethylpyrazolo[3,4-b]pyridin-4-yl)-3-methyl-piperidin-4-one (compound 20e)**

To the mixture of 4-chloro-1,6-dimethyl-pyrazolo[3,4-b]pyridine (compound 20c, CAS: 19867-78-8, Vendor: PharmaBlock, 511 mg, 2.82 mmol) and 3-methylpiperidin-4-one hydrochloride (compound **20d,** CAS: 4629-78-1, Vendor: PharmaBlock, 351 mg, 2.35 mmol) in 1,4-dioxane (20 mL) was added RuPhos Pd G2 (182 mg, 235 μmol) and $Cs_2CO_3$ (2.29 g, 7.04 mmol). The mixture was charged with $N_2$ and stirred at 110 °C overnight. After being cooled to rt, the solid was

filtered off and washed with EA (10 mL) twice. The combined mixture was concentrated and purified by flash column eluting with a gradient of EA (with 10% MeOH)/PE (0% to 80%) to give the desired product 20e (272 mg) as a yellow oil. MS: calc'd 259 ($MH^+$), measured 259 ($MH^+$).

**Step 3: preparation of [1-(1,6-dimethylpyrazolo[3,4-b]pyridin-4-yl)-5-methyl-3,6-dihydro-2H-pyridin-4-yl] trifluoromethanesulfonate (compound 20f)**

To a solution of 1-(1,6-dimethylpyrazolo[3,4-b]pyridin-4-yl)-3-methyl-piperidin-4-one (compound 20e, 200 mg, 774 μmol) in THF (10 mL) was added KHMDS (0.5M in THF, 2.32 mL, 1.16 mmol) in -78°C and stirred for 0.5h. Then 1,1,1-trifluoro-N-phenyl-N-(trifluoromethylsulfonyl)methanesulfonamide (415 mg, 1.16 mmol) was added and the reaction mixture was stirred at -78°C for another 2h. After being warmed to rt, the mixture was directly purified by flash column eluting with a gradient of EA (with 10% MeOH)/PE (0% to 60%) to give the desired product compound **20f** (230 mg). MS: calc'd 391 ($MH^+$), measured 391 ($MH^+$).

**Step 4: preparation of** tert-butyl **4-[5-[1-(1,6-dimethylpyrazolo[3,4-b]pyridin-4-yl)-5-methyl-3,6-dihydro-2H-pyridin-4-yl]-4-methyl-2-pyridyl]piperazine-1-carboxylate (compound 20g)**

To a mixture of [1-(1,6-dimethylpyrazolo[3,4-b]pyridin-4-yl)-5-methyl-3,6-dihydro-2H-pyridin-4-yl] trifluoromethanesulfonate (compound **20f,** 50 mg, 128 μmol) and tert-butyl 4-[4-methyl-5-(4,4,5,5-tetramethyl-1,3,2-dioxaborolan-2-yl)-2-pyridyl]piperazine-1-carboxylate (compound **20b,** 113 mg, 280 μmol) in 1,4-dioxane (5 mL) and water (1 mL) was added $K_2CO_3$ (53 mg, 384 μmol) and $PdCl_2(dppf)\cdot CH_2Cl_2$ (21 mg, 26 μmol). The mixture was charged with $N_2$ and stirred at 100 °C for 2h. After being cooled to rt, the mixture was dried over $Na_2SO_4$, filtered off and the solid was washed with EA (10 mL) twice. The combined organic layer was concentrated to give the crude product compound 20g (66 mg), which was used in next step without further purification. MS: calc'd 518 ($MH^+$), measured 518 ($MH^+$).

**Step 5: preparation of** tert-butyl 4-[5-[1-(1,6-dimethylpyrazolo[3,4-b]pyridin-4-yl)-3-**methyl-4-piperidyl]-4-methyl-2-pyridyl]piperazine-1-carboxylate (compound 20h)**

To a flask containing tert-butyl 4-[5-[1-(1,6-dimethylpyrazolo[3,4-b]pyridin-4-yl)-5-methyl-3,6-dihydro-2H-pyridin-4-yl]-4-methyl-2-pyridyl]piperazine-1-carboxylate (compound 20g, 66 mg, 128 μmol) was added $Pd(OH)_2$ (10 wt.% in carbon, 8.99 mg, 64 μmol) and MeOH (10 mL). The suspension was purged with $H_2$ for 3 times, then stirred under hydrogen balloon at rt overnight. The mixture was filtered and concentrated. The residue was purified by flash column eluting with a gradient of EA (with 10% MeOH)/PE (0% to 80%) to give the desired product 20h (25 mg). MS: calc'd 520 ($MH^+$), measured 520 ($MH^+$).

**Step 6: preparation of 1,6-dimethyl-4-[cis-3-methyl-4-(4-methyl-6-piperazin-1-yl-3-pyridyl)-1-piperidyl]pyrazolo[3,4-b]pyridine (Example 20) and *1,6-dimethyl-4-[trans-3*-methyl-4-(4-methyl-6-piperazin-1-yl-3-pyridyl)-1-piperidyl]pyrazolo[3,4-b]pyridine (Example 21)**

To the mixture of tert-butyl 4-[5-[1-(1,6-dimethylpyrazolo[3,4-b]pyridin-4-yl)-3-methyl-4-piperidyl]-4-methyl-2-pyridyl]piperazine-1-carboxylate (compound 20h, 25 mg, 48 μmol) in DCM (10 mL) was added TFA (2 mL). After being stirred at rt for 2h, the mixture was concentrated and purified by reversed flash column eluting with a gradient of ACN/Water (with 0.5% TFA) (0% to 30%) to give the desired products. The structures of **Example 20** and **Example 21** were confirmed by 2D-NMR.

**Example 20** (9 mg) was obtained as a light yellow solid. MS: calc'd 420 ($MH^+$), measured 420 ($MH^+$). $^1H$ NMR (400 MHz, METHANOL-$d_4$) δ = 8.45 (s, 1H), 7.81 (s, 1H), 7.20 (s, 1H), 6.76 (s, 1H), 4.71 (br d, *J* = 13.2 Hz, 1H), 4.55 (br d, *J* = 13.7 Hz, 1H), 4.10 (s, 3H), 3.97 - 3.87 (m, 4H), 3.83 (br d, *J* = 11.9 Hz, 1H), 3.62 (br t, *J* = 12.4 Hz, 1H), 3.53 (td, *J* = 3.6, 12.9 Hz, 1H), 3.46 - 3.39 (m, 4H), 2.65 (s, 3H), 2.56 (s, 3H), 2.54 - 2.41 (m, 2H), 1.93 - 1.84 (m, 1H), 0.76 (d, *J* = 7.0 Hz, 3H).

**Example 21** (2 mg) was obtained as a light yellow solid. MS: calc'd 420 ($MH^+$), measured 420 ($MH^+$). $^1H$ NMR (400 MHz, METHANOL-$d_4$) δ = 8.24 (s, 1H), 7.84 (s, 1H), 6.90 (s, 1H), 6.65 (s, 1H), 4.56 - 4.43 (m, 2H), 3.98 (s, 3H), 3.73 - 3.68 (m, 4H), 3.54 - 3.43 (m, 1H), 3.28 - 3.23 (m, 4H), 3.20 - 3.13 (m, 1H), 2.83 (dt, *J* = 3.9, 11.5 Hz, 1H), 2.54 (s, 3H), 2.36 (s, 3H), 2.10 - 1.99 (m, 1H), 1.99 - 1.92 (m, 1H), 1.76 - 1.65 (m, 1H), 0.81 (d, *J* = 6.6 Hz, 3H).

**Example 22 and Example 23**

**1,6-dimethyl-4-[cis-3-methyl-4-(3-methyl-5-piperazin-1-yl-pyrazin-2-yl)-1-piperidyl]pyrazolo[3,4-b]pyridine (Example 22) and 1,6-dimethyl-4-[trans-3-methyl-4-(3-methyl-5-piperazin-1-yl-pyrazin-2-yl)-1-piperidyl]pyrazolo [3,4-b]pyridine (Example 23)**

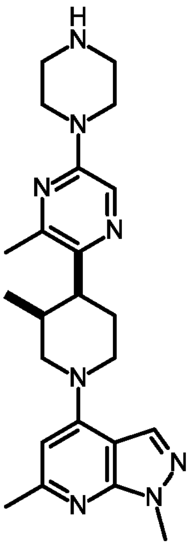

(Example 22)

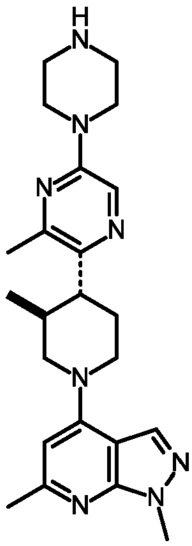

(Example 23)

The title compounds were prepared in analogy to the preparation of **Example 24** and **Example 25** by using tert-butyl 4-[5-[1-(1,6-dimethylpyrazolo[3,4-b]pyridin-4-yl)-3-methyl-3,6-dihydro-2H-pyridin-4-yl]-6-methyl-pyrazin-2-yl]piperazine-1-carboxylate (compound 22b) instead of compound 24c. The structures of **Example 22** and **Example 23** were confirmed by 2D-NMR.

**Example 22** (1 mg) was obtained as a yellow solid. MS: calc'd 421 ($MH^+$), measured 421 ($MH^+$). $^1H$ NMR (400 MHz, METHANOL-d4) δ = 8.30 (s, 1H), 8.07 (s, 1H), 6.71 (s, 1H), 4.60 (br d, *J* = 13.8 Hz, 1H), 4.50 (br d, *J* = 12.3 Hz, 1H), 4.07 (s, 3H), 3.87 - 3.80 (m, 5H), 3.59 (br s, 1H), 3.35 - 3.33 (m, 4H), 3.07 - 3.00 (m, 1H), 2.64 - 2.60 (m, 4H), 2.52 (s, 4H), 1.96 (br s, 1H), 0.85 (d, *J* = 6.5 Hz, 3H).

**Example 23** (1 mg) was obtained as a yellow solid. MS: calc'd 421 ($MH^+$), measured 421 ($MH^+$). $^1H$ NMR (400 MHz, METHANOL-d4) δ = 8.37 (s, 1H), 8.09 (s, 1H), 6.70 (s, 1H), 4.51 (br d, *J* = 12.8 Hz, 1H), 4.43 (br d, *J* = 11.4 Hz, 1H), 4.09 - 4.06 (m, 3H), 3.90 - 3.80 (m, 5H), 3.79 - 3.68 (m, 1H), 3.60 - 3.53 (m, 1H), 3.36 - 3.35 (m, 1H), 3.34 - 3.32 (m, 3H), 2.62 (s, 3H), 2.56 - 2.43 (m, 5H), 1.93 (br d, *J* = 9.9 Hz, 1H), 0.72 (d, *J* = 7.0 Hz, 3H).

Compound **22b** was prepared according to the following scheme:

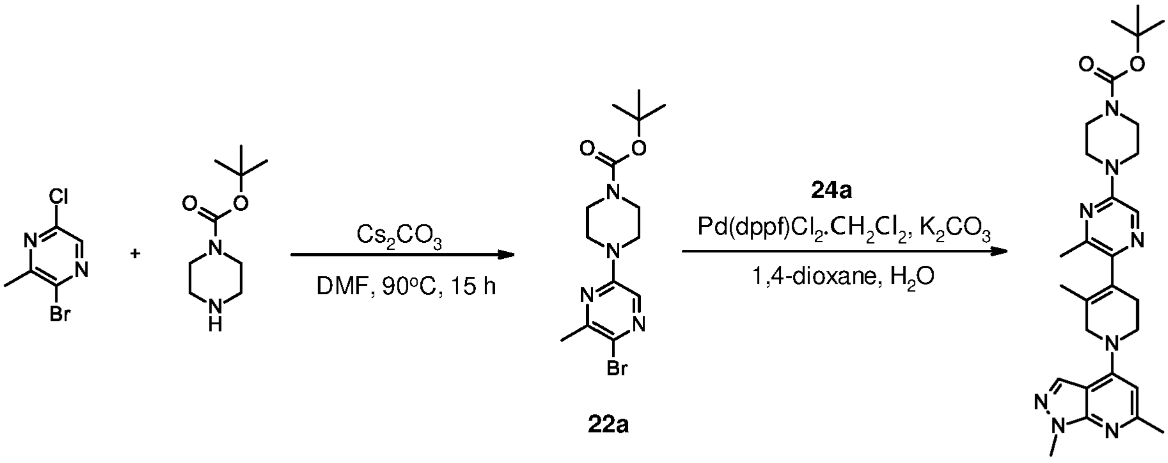


**22a**

**22b**

**Step 1: preparation of *tert-butyl* 4-(5-bromo-6-methyl-pyrazin-2-yl)piperazine-1-carboxylate (compound 22a)**

To a solution of 2-bromo-5-chloro-3-methyl-pyrazine (200 mg, 0.96 mmol) in DMF (3 mL) was added *tert-butyl* piperazine-1-carboxylate (180 mg, 0.96 mmol) and cesium carbonate (314 mg, 0.96 mmol). The reaction mixture was stirred at 90°C for 15 h. After being cooled to rt, the reaction mixture was filtered and the filtrate was concentrated under reduced pressure. The residue was purified by flash column eluting with a gradient of MeOH/DCM (0% to 10%) to give compound **22a** (77 mg) as a yellow oil. MS: calc'd 357 ($MH^+$), measured 357 ($MH^+$).

**Step 2: preparation of *tert*-butyl 4-[5-[1-(1,6-dimethylpyrazolo[3,4-b]pyridin-4-yl)-5-methyl-3,6-dihydro-2H-pyridin-4-yl]-6-methyl-pyrazin-2-yl]piperazine-1-carboxylate (compound 22b)**

To a solution of 1,6-dimethyl-4-[5-methyl-4-(4,4,5,5-tetramethyl-1,3,2-dioxaborolan-2-yl)-3,6-dihydro-2H-pyridin-1-yl]pyrazolo[3,4-b]pyridine (compound **24a,** 95 mg, 0.26 mmol) in 1,4-dioxane (5 mL) and water (1 mL) was added tert-butyl 4-(5-bromo-6-methyl-pyrazin-2-yl)piperazine-1-carboxylate (compound **22a,** 77 mg, 0.22 mmol), Pd(dppf) $Cl_2.CH_2Cl_2$ (18 mg, 0.22 mmol) and $K_2CO_3$ (45 mg, 0.32 mmol). The reaction mixture was stirred at 90°C for 2 h under $N_2$ atmosphere. After being cooled to rt, the reaction mixture was concentrated under reduced pressure, the residue was purified by flash column eluting with a gradient of MeOH/DCM (0% to 10%) to give compound **22b** (130 mg) as a brown oil. MS: calc'd 519 ($MH^+$), measured 519 ($MH^+$). The structures of **Example 22** and **Example 23** were confirmed by 2D-NMR.

**Example 24 and Example 25**

**1,6-dimethyl-4-[cis-3-methyl-4-(3-methyl-5-piperazin-1-yl-2-pyridyl)-1-piperidyl]pyrazolo[3,4-b]pyridine (Example 24) and 1,6-dimethyl-4-[trans-3-methyl-4-(3-methyl-5-piperazin-1-yl-2-pyridyl)-1-piperidyl]pyrazolo[3,4-b]pyridine (Example 25)**

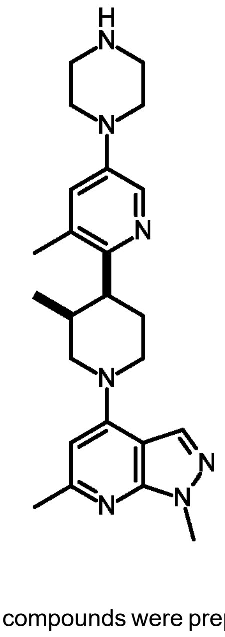
(Example 24)

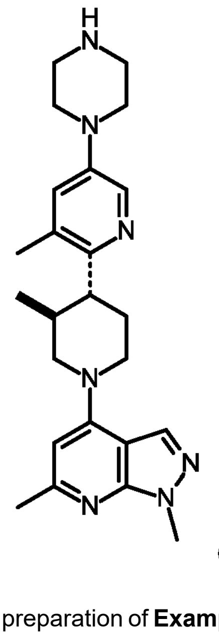
(Example 25)

The title compounds were prepared in analogy to the preparation of **Example 20** and **Example 21** by using tert-butyl 4-[6-[1-(1,6-dimethylpyrazolo[3,4-b]pyridin-4-yl)-5-methyl-3,6-dihydro-2H-pyridin-4-yl]-5-methyl-3-pyridyl]piperazine-1-carboxylate (compound **24c)** instead of compound 20g. The structures of **Example 24** and **Example 25** were confirmed by 2D-NMR.

**Example 24** (16 mg) was obtained as a light yellow solid. MS: calc'd 420 ($MH^+$), measured 420 ($MH^+$). $^1H$ NMR (500 MHz, METHANOL-$d_4$) $\delta$ = 8.42 (s, 1H), 8.20 - 8.14 (m, 1H), 7.99 - 7.91 (m, 1H), 6.76 (s, 1H), 4.70 (br d, *J* = 13.4 Hz, 1H), 4.53 (br d, *J* = 13.3 Hz, 1H), 4.09 (s, 3H), 3.86 (br d, *J* = 13.6 Hz, 2H), 3.73 - 3.60 (m, 5H), 3.49 - 3.33 (m, 4H), 2.76 - 2.61 (m, 4H), 2.60 - 2.52 (m, 4H), 2.03 - 1.93 (m, 1H), 0.76 (d, *J* = 7.0 Hz, 3H).

**Example 25** (8 mg) was obtained as a light yellow solid. MS: calc'd 420 ($MH^+$), measured 420 ($MH^+$). $^1H$ NMR (500 MHz, METHANOL-$d_4$) $\delta$ = 8.33 (s, 1H), 8.17 (d, *J* = 2.4 Hz, 1H), 7.78 (br s, 1H), 6.75 (s, 1H), 4.67 (br d, *J* = 13.4 Hz, 1H), 4.57 (br d, *J* = 14.6 Hz, 1H), 4.08 (s, 3H), 3.69 - 3.53 (m, 6H), 3.44 - 3.36 (m, 4H), 3.36 - 3.24 (m, 1H), 2.63 (s, 3H), 2.53 (s, 3H), 2.45 - 2.35 (m, 1H), 2.12 - 2.00 (m, 2H), 0.88 (d, *J* = 6.6 Hz, 3H).

Compound **24c** were prepared according to the following scheme:

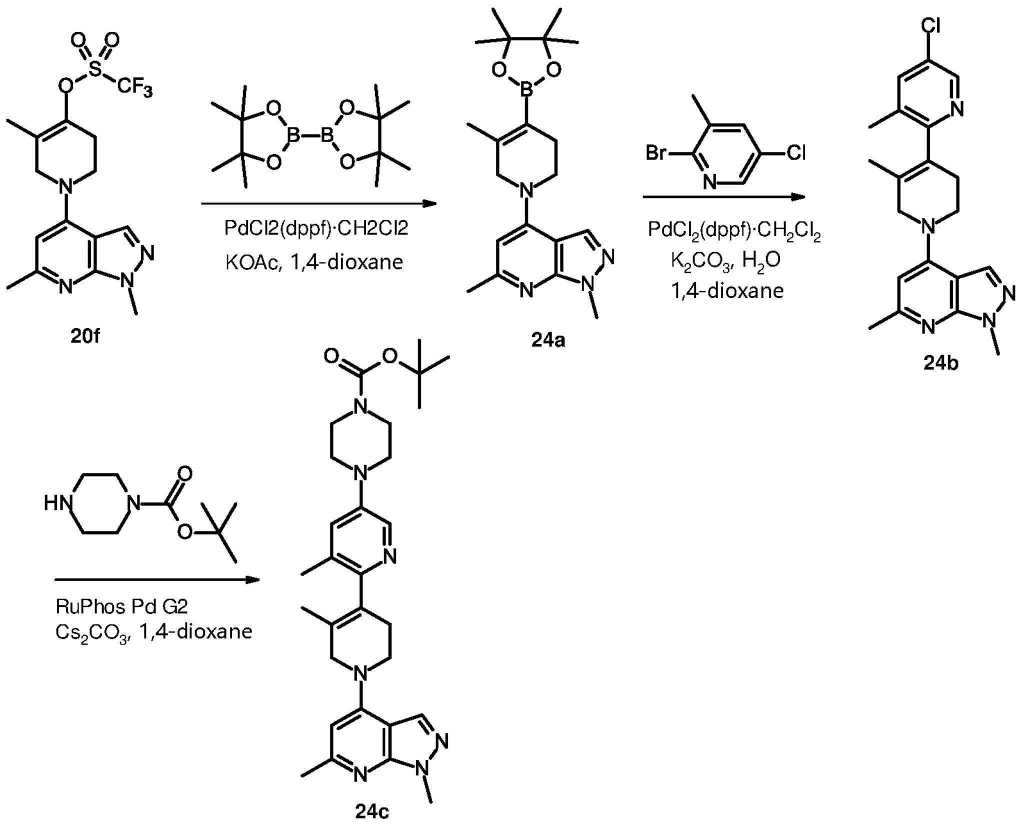


**20f** **24a** **24b** **24c**

**Step 1: preparation of 1,6-dimethyl-4-[5-methyl-4-(4,4,5,5-tetramethyl-1,3,2-dioxaborolan-2-yl)-3,6-dihydro-2H-pyridin-1-yl]pyrazolo[3,4-b]pyridine (compound 24a)**

To a mixture of [1-(1,6-dimethylpyrazolo[3,4-b]pyridin-4-yl)-5-methyl-3,6-dihydro-2H-pyridin-4-yl] trifluoromethanesulfonate (compound 20f, 300 mg, 0.768 mmol) and 4,4,4',4',5,5,5',5'-octamethyl-2,2'-bi(1,3,2-dioxaborolane) (292.72 mg, 1.15 mmol) in 1,4-dioxane (10 mL) was added PdCl$_2$(dppf)·CH$_2$Cl$_2$ (63 mg, 0.077 mmol) and potassium acetate (226 mg, 2.31 mmol). The mixture was charged with N$_2$ and stirred at 90 °C for 2h. After being cooled to rt, the mixture was filtered and the solid was washed with EA (10 mL) twice. The combined organic layer was concentrated to give the crude product compound 24a (283 mg) which was used in the next step without further purification. MS: calc'd 369 (MH$^+$), measured 369 (MH$^+$).

**Step 2: preparation of 4-[4-(5-chloro-3-methyl-2-pyridyl)-5-methyl-3,6-dihydro-2H-pyridin-1-yl]-1,6-dimethyl-pyrazolo[3,4-b]pyridine (compound 24b)**

To the mixture of 1,6-dimethyl-4-[5-methyl-4-(4,4,5,5-tetramethyl-1,3,2-dioxaborolan-2-yl)-3,6-dihydro-2*H*-pyridin-1-yl]pyrazolo[3,4-b]pyridine (compound **24a,** 283 mg, 0.768 mmol) and 2-bromo-5-chloro-3-methyl-pyridine (CAS: 65550-77-8, Vendor: Accela, 175 mg, 0.845 mmol) in 1,4-dioxane (10 mL) and water (2 mL) was added PdCl$_2$(dppf)·CH$_2$Cl$_2$ (31 mg, 0.038 mmol) and K$_2$CO$_3$ (212 mg, 1.54 mmol). The mixture was charged with N$_2$ and stirred at 90 °C overnight. After being cooled to rt, the mixture was extracted by DCM (20mL) three times, dried over Na$_2$SO$_4$, filtered and concentrated and purified by flash column eluting with a gradient of EA/PE (0% to 30%) to afford the desired product compound **24b** (260 mg). MS: calc'd 368 (MH$^+$), measured 368 (MH$^+$).

**Step 3: preparation of *tert*-butyl 4-[6-[1-(1,6-dimethylpyrazolo[3,4-b]pyridin-4-yl)-5-methyl-3,6-dihydro-2H-pyridin-4-yl]-5-methyl-3-pyridyl]piperazine-1-carboxylate (compound 24c)**

To the mixture of 4-[4-(5-chloro-3-methyl-2-pyridyl)-5-methyl-3,6-dihydro-2H-pyridin-1-yl]-1,6-dimethyl-pyrazolo[3,4-b]pyridine (compound **24b,** 130 mg, 0.353 mmol ), and 1-Boc-piperazine (99 mg, 0.530 mmol) in 1,4-dioxane (5 mL) was added cesium carbonate (230 mg, 0.707 mmol) and RuPhos Pd G2 (27 mg, 0.035 mmol). The mixture was charged

with $N_2$ and stirred at 110 °C overnight. After being cooled to rt, the solid was filtered off and washed with EA (10 mL) twice. The combined organic layer was concentrated and purified by flash column eluting with a gradient of EA(with 10% MeOH)/PE (0% to 80%) to give the desired product 24c (136 mg). MS: calc'd 518 ($MH^+$), measured 518 ($MH^+$).

**Example 26**

**(3R,4R)-1-[6-[(3S,4R)-1-(1,6-dimethylpyrazolo[3,4-b]pyridin-4-yl)-3-methyl-4-piperidyl]-5-methyl-3-pyridyl]-4-methoxy-pyrrolidin-3-amine**

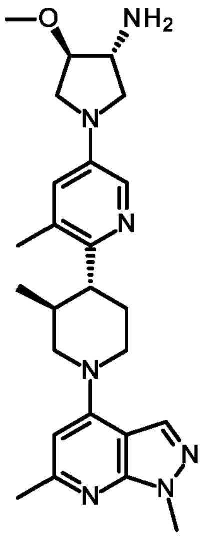

The title compound was prepared in analogy to the preparation of **Example 15** by using *tert-butyl* N-[(3R,4R)-4-methoxypyrrolidin-3-yl]carbamate (CAS: 1932066-52-8, Vendor: PharmaBlock) and **Intermediate B** instead of compound **15a** and **Intermediate E. Example 26** (75 mg) was obtained as a white solid. MS: calc'd 450 ($MH^+$), measured 450 ($MH^+$). $^1$H NMR (400 MHz, METHANOL-d4) $\delta$= 8.34 (s, 1 H), 7.85 (d, *J* = 2.50 Hz, 1 H), 7.58 (d, *J* = 2.50 Hz, 1 H), 6.77 (s, 1 H), 4.69 (br d, *J* = 14.01 Hz, 1 H), 4.59 (br d, *J* = 13.01 Hz, 1 H), 4.19 (dt, *J* = 5.63, 2.94 Hz, 1 H), 4.09 (s, 3 H), 3.99 (dt, *J* = 6.25, 3.38 Hz, 1 H), 3.87 (dd, *J* = 11.51, 5.50 Hz, 1 H), 3.80 (dd, *J* = 11.26, 6.25 Hz, 1 H), 3.68 - 3.58 (m, 1 H), 3.55 (dd, *J* = 11.26, 3.25 Hz, 1 H), 3.51 - 3.42 (m, 4 H), 3.40 - 3.32 (m, 2 H), 2.64 (s, 3 H), 2.57 (s, 3 H), 2.44 - 2.33 (m, 1 H), 2.14 - 2.04 (m, 2 H) 0.90 (d, *J* = 6.50 Hz, 3 H).

**Example 27**

**2-[6-[(3S,4R)-1-(1,6-dimethylpyrazolo[3,4-b]pyridin-4-yl)-3-methyl-4-piperidyl]-5-methyl-3-pyridyl]-5-oxa-2,8-diazaspiro[3.5]nonane**

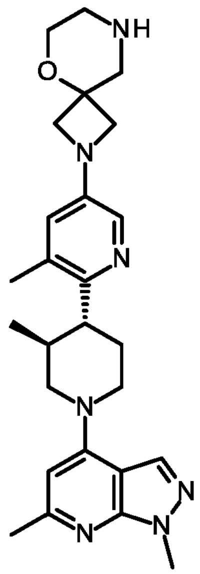

The title compound was prepared in analogy to the preparation of **Example 15** by using tert-butyl 5-oxa-2,8-diazaspiro[3.5]nonane-8-carboxylate (CAS: 1251005-61-4, Vendor: PharmaBlock) and **Intermediate B** instead of compound **15a** and **Intermediate E. Example 27** (65 mg) was obtained as a white solid. MS: calc'd 462 ($MH^+$), measured 462 ($MH^+$). $^1H$ NMR (400 MHz, METHANOL-d4) $\delta$ = 8.06 (s, 1H), 7.60 (d, J=2.5 Hz, 1H), 6.78 (d, J=2.4 Hz, 1H), 6.41 (s, 1H), 4.38 (br d, *J* = 13.8 Hz, 1H), 4.29 (br d, *J* = 11.9 Hz, 1H), 4.00 (s, 3H), 3.91 (d, *J*= 8.0 Hz, 2H), 3.74 - 3.63 (m, 4H), 3.02 (s, 3H), 2.99 - 2.96 (m, 1H), 2.92 - 2.87 (m, 1H), 2.84 - 2.80 (m, 2H), 2.53 (s, 3H), 2.37 (s, 3H), 2.31 (br dd, *J* = 6.5, 11.0 Hz, 1H), 1.98 - 1.88 (m, 1H), 1.84 - 1.75 (m, 1H),, 0.77 (d, *J* = 6.5 Hz, 3H).

**Example 28**

**(6*S*)-4-[6-[(3*S*,4*R*)-1-(1,6-dimethylpyrazolo[3,4-b]pyridin-4-yl)-3-methyl-4-piperidyl]-5-methyl-3-pyridyl]-1,4-oxazepan-6-amine**

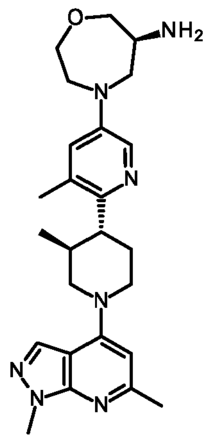

The title compound was prepared in analogy to the preparation of **Example 15** by using *tert-butyl N*-[(6*S*)-1,4-oxazepan-6-yl]carbamate (CAS: 2306247-11-8, Vendor: PharmaBlock) and **Intermediate B** instead of compound **15a** and **Intermediate E. Example 28** (75 mg) was obtained as a light yellow solid. MS: calc'd 450 ($MH^+$), measured 450 ($MH^+$). $^1H$ NMR (500 MHz, METHANOL-$d_4$) $\delta$ = 8.39 (s, 1H), 8.11 (d, *J* = 3.1 Hz, 1H), 8.02 (d, *J* = 2.6 Hz, 1H), 6.83 (s, 1H), 4.73 (br d, *J* = 10.4 Hz, 1H), 4.63 (br d, *J* = 12.4 Hz, 1H), 4.20 - 4.09 (m, 5H), 4.05 - 3.98 (m, 1H), 3.95 - 3.80 (m, 5H), 3.75 - 3.62 (m, 2H), 3.47 - 3.34 (m, 2H), 2.68 (s, 3H), 2.63 (s, 3H), 2.54 - 2.45 (m, 1H), 2.23 - 2.12 (m, 2H), 0.95 (d, *J* = 6.6 Hz, 3H).

**Example 29**

**4-[(3*S*,4*R*)-4-[5-[(3*R*)-3-(methoxymethyl)piperazin-1-yl]-3-methyl-2-pyridyl]-3-methyl-1-piperidyl]-1,6-dimethyl-pyrazolo[3,4-b]pyridine**

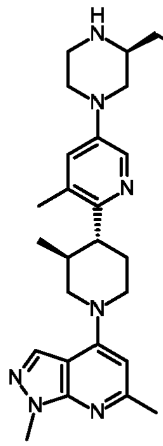

The title compound was prepared in analogy to the preparation of **Example 15** by using *tert-butyl* (2*R*)-2-(methoxymethyl)piperazine-1-carboxylate (CAS: 1023301-73-6, Vendor: PharmaBlock) and **Intermediate B** instead of compound **15a** and **Intermediate E. Example 29** (147 mg) was obtained as yellow solid. MS: calc'd 464 ($MH^+$), measured 464 ($MH^+$). $^1H$ NMR (500 MHz, METHANOL-$d_4$) $\delta$ = 8.37 (s, 1H), 8.21 (d, *J* = 2.9 Hz, 1H), 8.11 (d, *J* = 2.6 Hz, 1H), 6.81 (s, 1H), 4.71 (br d, *J* = 12.2 Hz, 1H), 4.65 - 4.58 (m, 1H), 4.15 - 4.04 (m, 5H), 3.77 - 3.70 (m, 1H), 3.70 - 3.62 (m, 3H), 3.54 - 3.49 (m, 1H), 3.48 - 3.41 (m, 4H), 3.38 - 3.31 (m, 3H), 3.25 (br dd, *J* = 10.3, 13.7 Hz, 1H), 2.66 (s, 3H), 2.64 - 2.59 (m, 3H), 2.53 - 2.44 (m, 1H), 2.20 - 2.11 (m, 2H), 0.92 (d, *J* = 6.6 Hz, 3H).

**Example 30**

**4-[(3*S*,4*R*)-4-[5-(4,7-diazaspiro[2.5]octan-7-yl)-3-methyl-2-pyridyl]-3-methyl-1-piperidyl]-1,6-dimethyl-pyrazolo[3,4-b]pyridine**

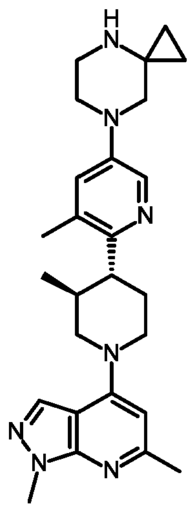

The title compound was prepared in analogy to the preparation of **Example 15** by using tert-butyl 4,7-diazaspiro[2.5]octane-4-carboxylate (CAS: 674792-08-6, Vendor: Accela) and **Intermediate** B instead of compound **15a** and **Intermediate E. Example 30** (42 mg) was obtained as a white solid. MS: calc'd 446 ($MH^+$), measured 446 ($MH^+$). $^1H$ NMR (400 MHz, METHANOL-d4) $\delta$ = 8.34 (s, 1H), 8.20 (d, *J* = 2.3 Hz, 1H), 7.97 (br s, 1H), 6.76 (s, 1H), 4.73 - 4.65 (m, 1H), 4.59 (br d, *J* = 13.3 Hz, 1H), 4.09 (s, 3H), 3.74 - 3.69 (m, 2H), 3.68 - 3.59 (m, 1H), 3.58 - 3.49 (m, 4H), 3.45 - 3.33 (m, 2H), 2.64 (s, 3H), 2.58 (s, 3H), 2.48 - 2.35 (m, 1H), 2.20 - 2.03 (m, 2H), 1.22 - 1.03 (m, 4H), 0.90 (d, *J* = 6.6 Hz, 3H).

**Example 31**

**(3*R*,4*R*)-1-[6-[(3*S*,4*R*)-1-(1,6-dimethylpyrazolo[3,4-b]pyridin-4-yl)-3-methyl-4-piperidyl]-5-methyl-3-pyridyl]-3-fluoro-piperidin-4-amine**

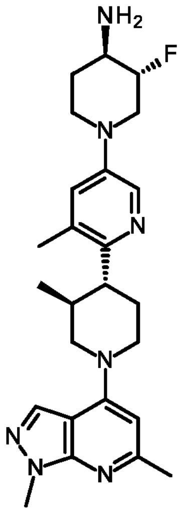

The title compound was prepared in analogy to the preparation of **Example 15** by using *tert-butyl* N-[(3R,4R)-3-fluoropiperidin-4-yl]carbamate (CAS: 1523530-29-1, Vendor: PharmaBlock) and **Intermediate B** instead of compound **15a** and **Intermediate E. Example 31** (44 mg) was obtained as light yellow solid. MS: calc'd 452 ($MH^+$), measured 452 ($MH^+$). $^1H$ NMR (500 MHz, METHANOL-$d_4$) $\delta$ = 8.33 (s, 1H), 8.19 (d, *J* = 2.7 Hz, 1H), 7.89 (d, *J* = 2.6 Hz, 1H), 6.75 (s, 1H), 4.78 - 4.63 (m, 2H), 4.57 (br d, *J* = 13.0 Hz, 1H), 4.35 - 4.27 (m, 1H), 4.08 (s, 3H), 3.98 - 3.88 (m, 1H), 3.66 - 3.57 (m, 1H), 3.57 - 3.46 (m, 1H), 3.37 - 3.31 (m, 2H), 3.08 - 2.97 (m, 2H), 2.63 (s, 3H), 2.55 (s, 3H), 2.44 - 2.34 (m, 1H), 2.28 - 2.19 (m, 1H), 2.13 - 2.02 (m, 2H), 1.82 (dq, *J* = 4.3, 12.6 Hz, 1H), 0.88 (d, *J* = 6.6 Hz, 3H).

**Example 32**

**1-[6-[(3*S*,4*R*)-1-(1,6-dimethylpyrazolo[3,4-b]pyridin-4-yl)-3-methyl-4-piperidyl]-5-methyl-3-pyridyl]-3-methyl-azetidin-3-amine**

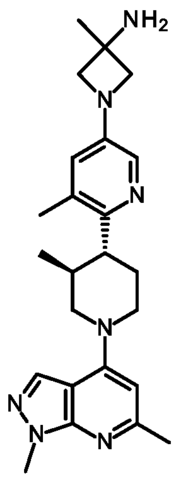

The title compound was prepared in analogy to the preparation of **Example 15** by using *tert*-butyl *N*-(3-methylazetidin-3-yl)carbamate hydrochloridecarbamate (CAS: 1408076-37-8, Vendor: PharmaBlock) and **Intermediate**

**B** instead of compound **15a** and **Intermediate E. Example 32** (66 mg) was obtained as light yellow solid. MS: calc'd 420 ($MH^+$), measured 420 ($MH^+$). $^1$H NMR (500 MHz, METHANOL-$d_4$) δ = 8.33 (s, 1H), 7.77 (d, *J* = 2.7 Hz, 1H), 7.42 (d, *J* = 2.6 Hz, 1H), 6.75 (s, 1H), 4.67 (br d, *J* = 13.6 Hz, 1H), 4.58 (br d, *J* = 13.1 Hz, 1H), 4.17 (d, *J* = 8.9 Hz, 2H), 4.12 - 4.06 (m, 5H), 3.67 - 3.58 (m, 1H), 3.37 - 3.31 (m, 2H), 2.63 (s, 3H), 2.54 (s, 3H), 2.43 - 2.34 (m, 1H), 2.12 - 2.05 (m, 2H), 1.69 (s, 3H), 0.88 (d, *J* = 6.6 Hz, 3H).

**Example 33**

**(4*aR*,7*aR*)-6-[6-[(3*S*,4*R*)-1-(1,6-dimethylpyrazolo[3,4-b]pyridin-4-yl)-3-methyl-4-piperidyl]-5-methyl-3-pyridyl]-3,4,4a,5,7,7a-hexahydro-2H-pyrrolo[3,4-b] [1,4]oxazine**

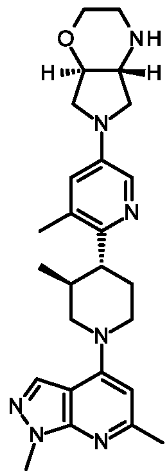

The title compound was prepared in analogy to the preparation of **Example 15** by using *tert*-butyl (4*aR*,7*aR*)-octahydropyrrolo[3,4-b]morpholine-4-carboxylate (CAS: 1932337-68-2, Vendor: PharmaBlock) and **Intermediate B** instead of compound **15a** and **Intermediate E. Example 33** (66 mg) was obtained as light yellow solid. MS: calc'd 462 ($MH^+$), measured 462 ($MH^+$). $^1$H NMR (500 MHz, METHANOL-$d_4$) δ = 8.33 (s, 1H), 7.83 (d, *J* = 2.9 Hz, 1H), 7.56 (d, *J* = 2.6 Hz, 1H), 6.75 (s, 1H), 4.68 (br d, *J* = 13.3 Hz, 1H), 4.64 - 4.52 (m, 1H), 4.25 (dd, J = 3.7, 13.1 Hz, 1H), 4.21 - 4.12 (m, 1H), 4.08 (s, 3H), 4.04 - 3.97 (m, 1H), 3.89 - 3.80 (m, 2H), 3.63 (dt, *J*= 7.6, 10.1 Hz, 2H), 3.52 (dd, *J* = 2.1, 13.3 Hz, 1H), 3.49 - 3.44 (m, 1H), 3.40 (dd, *J* = 4.2, 12.9 Hz, 1H), 3.37 - 3.32 (m, 3H), 2.63 (s, 3H), 2.56 (s, 3H), 2.43 - 2.34 (m, 1H), 2.14 - 2.07 (m, 2H), 0.89 (d, *J* = 6.6 Hz, 3H).

**Example 35**

**1,6-dimethyl-4-[(3*R*,4*S*)-3-methyl-4-(5-piperazin-1-yl-2-pyridyl)-1-piperidyl]pyrazolo[3,4-b]pyridine**

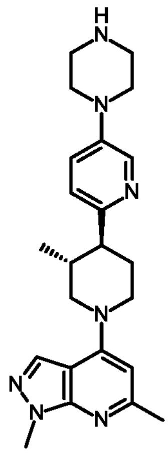

The title compound was prepared in analogy to the preparation of **Example 15** by using *tert-butyl* piperazine-1-carboxylate and **Intermediate D** instead of compound **15a** and **Intermediate E. Example 35** (5 mg) was obtained as a white solid. MS: calc'd 406 ($MH^+$), measured 406 ($MH^+$). $^1H$ NMR (400 MHz, METHANOL-d4) δ = 8.35 (s, 2H), 8.05 - 7.85 (m, 1H), 7.77 - 7.62 (m, 1H), 6.77 (s, 1H), 4.67 (br d, *J* = 13.4 Hz, 1H), 4.60 - 4.51 (m, 1H), 4.09 (s, 3H), 3.66 - 3.56 (m, 5H), 3.44 - 3.36 (m, 4H), 3.30 - 3.19 (m, 1H), 3.09 - 2.95 (m, 1H), 2.64 (s, 3H), 2.33 - 2.19 (m, 1H), 2.17 - 1.99 (m, 2H), 0.90 (d, *J* = 6.6 Hz, 3H).

**Example 34**

**1,6-dimethyl-4-[(3*S*,4*R*)-3-methyl-4-(5-piperazin-1-yl-2-pyridyl)-1-piperidyl]pyrazolo[3,4-b]pyridine**

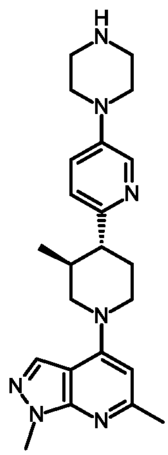

The title compound was prepared in analogy to the preparation of Example 15 by using *tert-butyl* piperazine-1-carboxylate and **Intermediate A** instead of compound **15a** and **Intermediate E. Example 34** (20 mg) was obtained as a white solid. MS: calc'd 406 ($MH^+$), measured 406 ($MH^+$). $^1H$ NMR (400 MHz, METHANOL-d4) δ = 8.35 (s, 1H), 8.32 (d, *J* = 2.9 Hz, 1H), 7.82 (dd, *J* = 2.9, 9.0 Hz, 1H), 7.58 (d, *J* = 8.8 Hz, 1H), 6.76 (s, 1H), 4.69 - 4.62 (m, 1H), 4.55 (br dd, *J* = 2.2, 13.9 Hz, 1H), 4.09 (s, 3H), 3.63 - 3.53 (m, 5H), 3.47 - 3.36 (m, 4H), 3.29 - 3.22 (m, 1H), 2.95 (dt, *J* = 4.4, 11.2 Hz, 1H), 2.64 (s, 3H), 2.30 - 2.17 (m, 1H), 2.15 - 1.98 (m, 2H), 0.89 (d, *J* = 6.6 Hz, 3H).

**Example 36**

**(3*R*,4*R*)-1-[6-[(3*R*,4*S*)-1-(1,6-dimethylpyrazolo[3,4-b]pyridin-4-yl)-3-methyl-4-piperidyl]-3-pyridyl]-4-methoxy-pyrrolidin-3-amine**

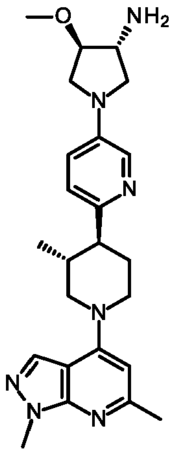

The title compound was prepared in analogy to the preparation of **Example 15** by using *tert-butyl* N-[(3R,4R)-4-methoxypyrrolidin-3-yl]carbamate (CAS: 1932066-52-8, Vendor: PharmaBlock) and **Intermediate** D instead of compound **15a** and **Intermediate E. Example 36** (28 mg) was obtained as a white solid. MS: calc'd 436 ($MH^+$), measured 436 ($MH^+$). $^1H$ NMR (400 MHz, METHANOL-d4) $\delta$ = 8.35 (s, 1H), 7.99 (d, *J* = 2.75 Hz, 1 H), 7.73 - 7.67 (m, 1H), 7.67 - 7.60 (m, 1H), 6.77 (s, 1H), 4.71 - 4.63 (m, 1H), 4.61 - 4.53 (m, 1H), 4.24 - 4.15 (m, 1H), 4.09 (s, 3H), 4.00 (td, *J* = 3.2, 6.3 Hz, 1H), 3.89 (dd, *J* = 5.6, 11.1 Hz, 1H), 3.81 (dd, *J* = 6.3, 11.3 Hz, 1H), 3.62 - 3.54 (m, 2H), 3.50 - 3.43 (m, 4H), 3.29 - 3.22 (m, 1H), 3.01 (dt, *J* = 4.3, 11.4 Hz, 1H), 2.64 (s, 3H), 2.28 - 2.02 (m, 3H), 0.90 (d, *J* = 6.5 Hz, 3H).

**Example 37**

**4-[(3*R*,4*S*)-4-[5-[(3*R*)-3-(methoxymethyl)piperazin-1-yl]-2-pyridyl]-3-methyl-1-piperidyl]-1,6-dimethyl-pyrazolo [3,4-b]pyridine**

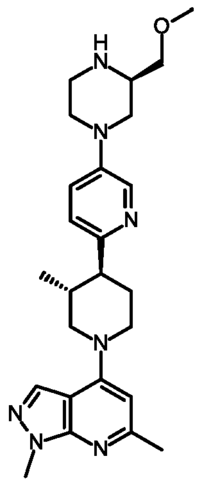

The title compound was prepared in analogy to the preparation of **Example 15** by using *tert-butyl* (2*R*)-2-(methoxymethyl)piperazine-1-carboxylate (CAS: 1023301-73-6, Vendor: PharmaBlock) and **Intermediate D** instead of compound **15a** and **Intermediate E. Example 37** (5 mg) was obtained as a white solid. MS: calc'd 450 ($MH^+$), measured 450 ($MH^+$). $^1H$ NMR (400 MHz, METHANOL-d4) $\delta$ = 8.05 (d, *J* = 2.8 Hz, 1H), 7.96 (s, 1H), 7.33 - 7.27 (m, 1H), 7.11 (d, *J* = 8.7 Hz, 1H), 6.31 (s, 1H), 4.27 (br d, *J* = 13.0 Hz, 1H), 4.18 (br d, *J* = 13.6 Hz, 1H), 3.89 (s, 3H), 3.55 (br t, *J* = 12.7 Hz, 2H), 3.46 - 3.34 (m, 2H), 3.31 (s, 3H), 3.17 - 3.09 (m, 3H), 3.01 - 2.92 (m, 1H), 2.88 - 2.72 (m, 2H), 2.62 - 2.47 (m, 2H), 2.43 (s, 3H), 2.09 - 1.98 (m, 1H), 1.86 - 1.75 (m, 2H), 0.69 (d, *J* = 6.5 Hz, 3H).

**Example 38**

**2-[6-[(3*R*,4*S*)-1-(1,6-dimethylpyrazolo[3,4-b]pyridin-4-yl)-3-methyl-4-piperidyl]-3-pyridyl]-5-oxa-2,8-diazaspiro [3.5]nonane**

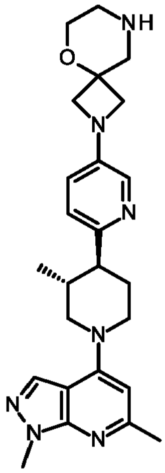

The title compound was prepared in analogy to the preparation of Example 15 by using tert-butyl 5-oxa-2,8-diazaspiro[3.5]nonane-8-carboxylate (CAS: 1251005-61-4, Vendor: PharmaBlock) and **Intermediate** D instead of compound 15a and Intermediate E. Example 38 (11 mg) was obtained as a white solid. MS: calc'd 448 ($MH^+$), measured 448 ($MH^+$). $^1H$ NMR (400 MHz, METHANOL-d4) δ = 8.07 (s, 1H), 7.74 (d, *J* = 2.8 Hz, 1H), 7.16 (d, *J* = 8.4 Hz, 1H), 6.95 (dd, *J* = 2.8, 8.4 Hz, 1H), 6.42 (s, 1H), 4.38 (br d, *J* = 12.8 Hz, 1H), 4.29 (br d, *J* = 11.0 Hz, 1H), 4.01 (s, 3H), 3.94 (d, *J* = 8.0 Hz, 2H), 3.70 (br d, *J* = 7.8 Hz, 4H), 3.31 - 3.22 (m, 1H), 3.02 (s, 2H), 2.99 - 2.89 (m, 1H), 2.85 - 2.77 (m, 2H), 2.63 - 2.56 (m, 1H), 2.54 (s, 3H), 2.20 - 2.04 (m, 1H), 1.98 - 1.87 (m, 2H), 0.81 (d, *J* = 6.5 Hz, 3H).

## Example 39

**(3*S*,4*S*)-1-[6-[(3*R*,4*S*)-1-(1,6-dimethylpyrazolo[3,4-b]pyridin-4-yl)-3-methyl-4-piperidyl]-3-pyridyl]-3-methoxy-piperidin-4-amine**

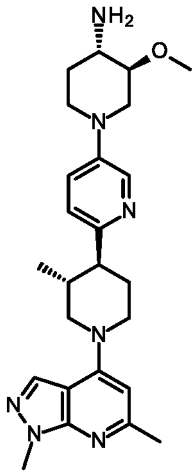

The title compound was prepared in analogy to the preparation of **Example 15** by using *tert-butyl* N-[(3S,4S)-3-methoxy-4-piperidyl]carbamate (CAS: 907544-19-8, Vendor: PharmaBlock) and **Intermediate D** instead of compound **15a** and **Intermediate E. Example 39** (19 mg) was obtained as a white solid. MS: calc'd 450 ($MH^+$), measured 450 ($MH^+$). $^1H$ NMR (400 MHz, METHANOL-d4) δ = 8.37 - 8.36 (m, 1H), 8.35 (s, 1H), 8.07 (dd, *J* = 8.8, 2.9 Hz, 1H), 7.74 (d, *J* = 9.3 Hz, 1H), 6.76 (s, 1H), 4.67 (br d, *J* = 14.3 Hz, 1H), 4.61 - 4.52 (m, 1H), 4.35 (br dd, J= 12.2, 3.4 Hz, 1H), 4.09 (s, 3H), 3.98 (br d, *J*

= 13.2 Hz, 1H), 3.64 - 3.57 (m, 1H), 3.55 (s, 3H), 3.40 (td, *J* = 10.0, 4.4 Hz, 1H), 3.29 - 3.14 (m, 2H), 2.95 - 3.10 (m, 2H), 2.74 (dd, J = 12.2, 10.3 Hz, 1H), 2.64 (s, 3H), 2.29 - 2.04 (m, 4H), 1.81 (qd, *J* = 12.6, 4.7 Hz, 1H), 0.91 (d, *J* = 6.4 Hz, 3H).

**Example 40**

**(6*S*)-4-[6-[(3*R*,4*S*)-1-(1,6-dimethylpyrazolo[3,4-b]pyridin-4-yl)-3-methyl-4-piperidyl]-3-pyridyl]-1,4-oxazepan-6-amine**

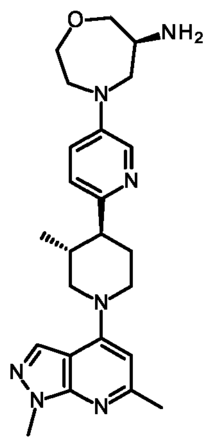

The title compound was prepared in analogy to the preparation of **Example 15** by using *tert-butyl N*-[(6*S*)-1,4-oxazepan-6-yl]carbamate (CAS: 2306247-11-8, Vendor: PharmaBlock) and **Intermediate D** instead of compound **15a** and **Intermediate E. Example 40** (70 mg) was obtained as a white solid. MS: calc'd 436 ($MH^+$), measured 436 ($MH^+$). $^1H$ NMR (500 MHz, METHANOL-$d_4$) $\delta$ = 8.28 (s, 1H), 8.18 (d, *J* = 2.7 Hz, 1H), 8.00 (dd, *J* = 2.8, 9.2 Hz, 1H), 7.75 (d, *J*= 9.3 Hz, 1H), 6.71 (s, 1H), 4.60 (br d, *J* = 11.7 Hz, 1H), 4.49 (br d, *J* = 12.8 Hz, 1H), 4.10 - 3.97 (m, 5H), 3.95 - 3.88 (m, 1H), 3.84 - 3.78 (m, 2H), 3.77 - 3.68 (m, 3H), 3.64 - 3.47 (m, 2H), 3.27 - 3.21 (m, 1H), 3.01 (dt, *J* = 3.8, 11.5 Hz, 1H), 2.56 (s, 3H), 2.25 - 2.14 (m, 1H), 2.14 - 2.07 (m, 1H), 2.06 - 1.94 (m, 1H), 0.85 (d, *J* = 6.6 Hz, 3H).

**Example 41**

**(3*R*,4*R*)-1-[6-[(3*R*,4*S*)-1-(1,6-dimethylpyrazolo[3,4-b]pyridin-4-yl)-3-methyl-4-piperidyl]-3-pyridyl]-3-fluoro-piperidin-4-amine**

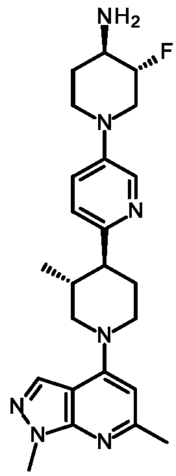

The title compound was prepared in analogy to the preparation of **Example 15** by using *tert*-butyl *N*-[(3*R*,4*R*)-3-fluoropiperidin-4-yl]carbamate (CAS: 1523530-29-1, Vendor: PharmaBlock) and **Intermediate D** instead of compound

**15a** and **Intermediate E. Example 41** (10 mg) was obtained as white solid. MS: calc'd 438 ($MH^+$), measured 438 ($MH^+$). $^1$H NMR (500 MHz, METHANOL-$d_4$) δ = 8.09 - 8.03 (m, 1H), 7.95 (s, 1H), 7.30 (dd, *J* = 3.0, 8.6 Hz, 1H), 7.08 (d, *J* = 8.5 Hz, 1H), 6.29 (s, 1H), 4.38 - 4.21 (m, 2H), 4.18 - 4.13 (m, 1H), 3.94 - 3.82 (m, 4H), 3.55 (br dd, *J* = 1.3, 12.7 Hz, 1H), 3.24 - 3.19 (m, 2H), 3.18 - 3.09 (m, 1H), 2.92 - 2.78 (m, 2H), 2.77 - 2.66 (m, 2H), 2.49 (dt, *J* = 4.7, 11.1 Hz, 1H), 2.09 - 1.89 (m, 2H), 1.86 - 1.76 (m, 2H), 1.52 (br dd, *J*= 4.1, 12.4 Hz, 1H), 1.24 - 1.14 (m, 1H), 0.68 (d, *J* = 6.6 Hz, 3H).

**Example 42**

**4-[(3*R*,4*S*)-4-[5-(4,7-diazaspiro[2.5]octan-7-yl)-2-pyridyl]-3-methyl-1-piperidyl]-1,6-dimethyl-pyrazolo[3,4-b]pyridine**

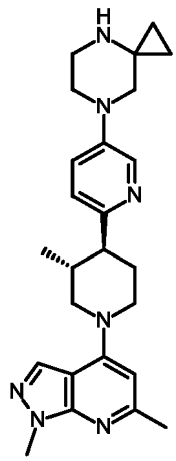

The title compound was prepared in analogy to the preparation of **Example 15** by using tert-butyl 4,7-diazaspiro [2.5]octane-4-carboxylate (CAS: 674792-08-6, Vendor: PharmaBlock) and **Intermediate D** instead of compound **15a** and **Intermediate E. Example 42** (19 mg) was obtained as white solid. MS: calc'd 432 ($MH^+$), measured 432 ($MH^+$). $^1$H NMR (500 MHz, METHANOL-$d_4$) δ = 7.99 (d, *J* = 2.7 Hz, 1H), 7.95 (s, 1H), 7.24 (dd, *J* = 2.9, 8.7 Hz, 1H), 7.07 (d, *J*= 8.7 Hz, 1H), 6.30 (s, 1H), 4.26 (br d, *J* = 13.1 Hz, 1H), 4.20 - 4.11 (m, 1H), 3.89 (s, 3H), 3.19 - 3.08 (m, 3H), 3.01 - 2.96 (m, 2H), 2.95 (s, 2H), 2.86 - 2.78 (m, 1H), 2.53 - 2.45 (m, 1H), 2.42 (s, 3H), 2.07 - 1.97 (m, 1H), 1.89 - 1.75 (m, 2H), 0.68 (d, *J* = 6.6 Hz, 3H), 0.59 (br d, *J* = 8.4 Hz, 4H).

**Example 43**

**1-[6-[(3*R*,4*S*)-1-(1,6-dimethylpyrazolo[3,4-b]pyridin-4-yl)-3-methyl-4-piperidyl]-3-pyridyl]-3-methyl-azetidin-3-amine**

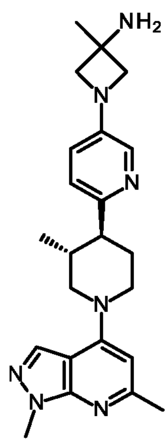

The title compound was prepared in analogy to the preparation of **Example 15** by using *tert-butyl N*-(3-methylazetidin-3-yl)carbamate hydrochloridecarbamate (CAS: 1408076-37-8, Vendor: PharmaBlock) and **Intermediate D** instead of compound **15a** and **Intermediate E. Example 43** (33 mg) was obtained as white solid. MS: calc'd 406 ($MH^+$), measured 406 ($MH^+$). $^1H$ NMR (500 MHz, METHANOL-$d_4$) δ = 8.24 (s, 1H), 7.83 (d, *J* = 2.7 Hz, 1H), 7.58 (d, *J* = 8.9 Hz, 1H), 7.41 (dd, J = 2.9, 8.9 Hz, 1H), 6.66 (s, 1H), 4.56 (br d, *J* = 13.6 Hz, 1H), 4.46 (br d, *J* = 12.7 Hz, 1H), 4.09 (d, *J* = 9.0 Hz, 2H), 4.03 - 3.95 (m, 5H), 3.48 (br t, *J* = 12.0 Hz, 1H), 3.19 - 3.11 (m, 1H), 2.91 (dt, *J= 4.2,* 11.5 Hz, 1H), 2.54 (s, 3H), 2.19 - 1.90 (m, 3H), 1.60 (s, 3H), 0.79 (d, *J = 6.6* Hz, 3H).

**Example 44**

The following tests were carried out in order to determine the activity of the compounds of formula (I) and (Ia) in HEK293-Blue-hTLR-7/8/9 cells assay.

**HEK293-Blue-hTLR-7 cells assay:**

A stable HEK293-Blue-hTLR-7 cell line was purchased from InvivoGen (Cat.#: hkb-htlr7, San Diego, California, USA). These cells were originally designed for studying the stimulation of human TLR7 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR7 cells with TLR7 ligands. Therefore the reporter expression was declined by TLR7 antagonist under the stimulation of a ligand, such as R848 (Resiquimod), for incubation of 20 hrs. The cell culture supernatant SEAP reporter activity was determined using QUANTI-Blue™ kit (Cat.#: rep-qb1, Invivogen, San Diego, Ca, USA) at a wavelength of 640 nm, a detection medium that turns purple or blue in the presence of alkaline phosphatase.

HEK293-Blue-hTLR7 cells were incubated at a density of 250,000~450,000 cells/mL in a volume of 170 μL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 μL test compound in a serial dilution in the presence of final DMSO at 1% and 10 μL of 20uM R848 in above DMEM, perform incubation under 37 °C in a $CO_2$ incubator for 20 hrs. Then 20 μL of the supernatant from each well was incubated with 180 μL Quanti-blue substrate solution at 37 °C for 2 hrs and the absorbance was read at 620~655 nm using a spectrophotometer. The signaling pathway that TLR7 activation leads to downstream NF-κB activation has been widely accepted, and therefore similar reporter assay was modified for evaluating TLR7 antagonist.

**HEK293-Blue-hTLR-8 cells assay:**

A stable HEK293-Blue-hTLR-8 cell line was purchased from InvivoGen (Cat.#: hkb-htlr8, San Diego, California, USA). These cells were originally designed for studying the stimulation of human TLR8 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR8 cells with TLR8 ligands. Therefore the reporter expression was declined by TLR8 antagonist under the stimulation of a ligand, such as R848, for incubation of 20 hrs. The cell culture supernatant SEAP reporter activity was determined using QUANTI-Blue™ kit (Cat.#: rep-qb1, Invivogen, San Diego, Ca, USA) at a wavelength of 640 nm, a detection medium that turns purple or blue in the presence of alkaline phosphatase.

HEK293-Blue-hTLR8 cells were incubated at a density of 250,000~450,000 cells/mL in a volume of 170 μL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 μL test compound in a serial dilution in the presence of final DMSO at 1% and 10 μL of 60uM R848 in above DMEM, perform incubation under 37 °C in a $CO_2$ incubator for 20 hrs. Then 20 μL of the supernatant from each well was incubated with 180 μL Quanti-blue substrate solution at 37°C for 2 hrs and the absorbance was read at 620~655 nm using a spectrophotometer. The signaling pathway that TLR8 activation leads to downstream NF-κB activation has been widely accepted, and therefore similar reporter assay was modified for evaluating TLR8 antagonist.

**HEK293-Blue-hTLR-9 cells assay:**

A stable HEK293-Blue-hTLR-9 cell line was purchased from InvivoGen (Cat.#: hkb-htlr9, San Diego, California, USA). These cells were originally designed for studying the stimulation of human TLR9 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-*1 via* stimulating HEK-Blue hTLR9 cells with TLR9 ligands. Therefore the reporter expression was declined by TLR9 antagonist under the

stimulation of a ligand, such as ODN2006 (Cat.#: t1r1-2006-1, Invivogen, San Diego, California, USA), for incubation of 20 hrs. The cell culture supernatant SEAP reporter activity was determined using QUANTI-Blue™ kit (Cat.#: rep-qbl, Invivogen, San Diego, California, USA) at a wavelength of 640 nm, a detection medium that turns purple or blue in the presence of alkaline phosphatase.

HEK293-Blue-hTLR9 cells were incubated at a density of 250,000~450,000 cells/mL in a volume of 170 μL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 μL test compound in a serial dilution in the presence of final DMSO at 1% and 10 μL of 20uM ODN2006 in above DMEM, perform incubation under 37 °C in a $CO_2$ incubator for 20 hrs. Then 20 μL of the supernatant from each well was incubated with 180 μL Quanti-blue substrate solution at 37 °C for 2 h and the absorbance was read at 620~655 nm using a spectrophotometer. The signaling pathway that TLR9 activation leads to downstream NF-κB activation has been widely accepted, and therefore similar reporter assay was modified for evaluating TLR9 antagonist.

The compounds of formula (I) or (Ia) have human TLR7 and TLR8 inhibitory activities ($IC_{50}$ value) <0.5 μM. Moreover, some compounds also have human TLR9 inhibitory activity <0.5μM. Activity data of the compounds of the present invention were shown in Table 2.

**Table 2. The activity of the compounds of present invention in HEK293-Blue-hTLR-7/8/9 cells assays**

| Example No | HEK/hTLR7 $IC_{50}$ (μM) | HEK/hTLR8 $IC_{50}$ (μM) | HEK/hTLR9 $IC_{50}$ (μM) |
|---|---|---|---|
| **1** | **0.053** | **0.012** | **0.275** |
| **2** | **0.027** | **0.021** | **0.142** |
| **3** | **0.020** | **0.050** | **0.124** |
| **4** | **0.031** | **0.059** | **0.379** |
| **5** | **0.008** | **0.064** | **0.364** |
| **6** | **0.007** | **0.037** | **0.208** |
| **7** | **0.011** | **0.029** | **0.052** |
| **8** | **0.052** | **0.008** | **0.195** |
| **9** | **0.039** | **0.031** | **0.120** |
| **10** | **0.039** | **0.013** | **0.212** |
| **11** | **0.037** | **0.042** | **0.104** |
| **13** | **0.026** | **0.044** | **0.168** |
| **14** | **0.012** | **0.013** | **0.225** |
| **15** | **0.024** | **0.047** | **0.130** |
| **16** | **0.023** | **0.032** | **0.143** |
| **17** | **0.022** | **0.039** | **0.073** |
| **20** | **0.050** | **0.006** | **0.220** |
| **21** | **0.016** | **0.003** | **0.372** |
| **22** | **0.049** | **0.006** | **0.315** |
| **23** | **0.011** | **0.007** | **0.368** |
| **24** | **0.082** | **0.005** | **0.076** |
| **25** | **0.009** | **0.007** | **0.098** |
| **26** | **0.012** | **0.014** | **0.109** |
| **27** | **0.009** | **0.007** | **0.184** |
| **28** | **0.007** | **0.002** | **0.126** |
| **29** | **0.005** | **0.008** | **0.238** |
| **31** | **0.005** | **0.009** | **0.219** |
| **32** | **0.008** | **0.015** | **0.186** |

(continued)

| Example No | HEK/hTLR7 $IC_{50}$ (μM) | HEK/hTLR8 $IC_{50}$ (μM) | HEK/hTLR9 $IC_{50}$ (μM) |
|---|---|---|---|
| **33** | **0.011** | **0.003** | **0.079** |
| **34** | **0.082** | **0.011** | **0.165** |
| **35** | **0.006** | **0.009** | **0.158** |
| **36** | **0.018** | **0.021** | **0.170** |
| **37** | **0.009** | **0.013** | **0.332** |
| **38** | **0.013** | **0.011** | **0.192** |
| **39** | **0.012** | **0.010** | **0.211** |
| **40** | **0.009** | **0.007** | **0.173** |
| **41** | **0.006** | **0.010** | **0.231** |
| **43** | **0.016** | **0.006** | **0.203** |

**Example 45**

**hERG channel inhibition assay:**

The hERG channel inhibition assay is a highly sensitive measurement that identifies compounds exhibiting hERG inhibition related to cardiotoxicity in vivo. The hERG $K^+$ channels were cloned in humans and stably expressed in a CHO (Chinese hamster ovary) cell line. $CHO_{hERG}$ cells were used for patch-clamp (voltage-clamp, whole-cell) experiments. Cells were stimulated by a voltage pattern to activate hERG channels and conduct $I_{KhERG}$ currents (rapid delayed outward rectifier potassium current of the hERG channel). After the cells were stabilized for a few minutes, the amplitude and kinetics of $I_{KhERG}$ were recorded at a stimulation frequency of 0.1 Hz (6 bpm). Thereafter, the test compound was added to the preparation at increasing concentrations. For each concentration, an attempt was made to reach a steady-state effect, usually, this was achieved within 3-10 min at which time the next highest concentration was applied. The amplitude and kinetics of $I_{KhERG}$ are recorded in each concentration of the drug which were compared to the control values (taken as 100%). (references: Redfern WS, Carlsson L, Davis AS, Lynch WG, MacKenzie I, Palethorpe S, Siegl PK, Strang I, Sullivan AT, Wallis R, Camm AJ, Hammond TG. 2003; Relationships between preclinical cardiac electrophysiology, clinical QT interval prolongation and torsade de pointes for a broad range of drugs: evidence for a provisional safety margin in drug development. Cardiovasc. Res. 58:32-45, Sanguinetti MC, Tristani-Firouzi M. 2006; hERG potassium channels and cardiac arrhythmia. Nature 440:463-469, Webster R, Leishman D, Walker D. 2002; Towards a drug concentration effect relationship for QT prolongation and torsades de pointes. Curr. Opin. Drug Discov. Devel. 5:116-26).

Results of hERG are given in Table 3. A safety ratio (hERG $IC_{20}$ /$EC_{50}$) > 30 suggests a sufficient window to differentiate the pharmacology by inhibiting TLR7/8/9 pathways from the potential hERG related cardiotoxicity. According to the calculation of hERG $IC_{20}$ / TLR7/8/9 $IC_{50}$ below which serves as early selectivity index to assess hERG liability, obviously reference compounds ER-887258, ER-888285, ER-888286, R1 and R2 have much narrower safety window compared to the compounds of this invention.

**Table 3. hERG and safety ratio results**

| Example No | hERG $IC_{20}$ (μM) | hERG $IC_{50}$ (μM) | hERG $IC_{20}$ / TLR7 $IC_{50}$ | hERG $IC_{20}$ / TLR8 $IC_{50}$ | hERG $IC_{20}$ / TLR9 $IC_{50}$ |
|---|---|---|---|---|---|
| **1** | **4.0** | **>10.0** | **75.5** | **333.3** | **14.5** |
| **2** | **> 10.0** | **>20.0** | **>370.4** | **>476.2** | **>70.4** |
| **6** | **4.4** | **>10.0** | **628.6** | **118.9** | **21.2** |
| **7** | **5.1** | **>20.0** | **463.6** | **175.9** | **98.1** |
| **11** | **7.5** | **>20.0** | **202.7** | **178.6** | **72.1** |
| **12** | **7.0** | **>20.0** | **205.9** | **233.3** | **15.5** |
| **25** | **> 10.0** | **>20.0** | **>1111.1** | **>1428.6** | **>102.0** |
| **26** | **> 10.0** | **>20.0** | **>833.3** | **>714.3** | **>91.7** |

(continued)

| Example No | hERG $IC_{20}$ (μM) | hERG $IC_{50}$ (μM) | hERG $IC_{20}$ / TLR7 $IC_{50}$ | hERG $IC_{20}$ / TLR8 $IC_{50}$ | hERG $IC_{20}$ / TLR9 $IC_{50}$ |
|---|---|---|---|---|---|
| **28** | **6.4** | **>20** | **914.3** | **3200.0** | **50.8** |
| **29** | **4.4** | **>10** | **880.0** | **550.0** | **18.5** |
| **30** | **4.9** | **>10.0** | **544.4** | **350.0** | **11.6** |
| **32** | **4.4** | **>10.0** | **550.0** | **293.3** | **23.7** |
| **33** | **4.1** | **>10.0** | **372.7** | **1366.7** | **51.9** |
| **35** | **4.4** | **>10.0** | **733.3** | **488.9** | **27.8** |
| **36** | **> 10.0** | **>20.0** | ***>555.6*** | **>476.2** | **>58.8** |
| **37** | **> 10.0** | **>20.0** | **>1111.1** | **>769.2** | **>30.1** |
| **38** | **5.2** | **>20.0** | **400.0** | **472.7** | **27.1** |
| **40** | **5.9** | **>20.0** | **655.6** | **842.9** | **34.1** |
| **43** | **> 10.0** | **>20.0** | **>625.0** | **>1666.7** | **>49.3** |

**Example 46**

**Human PBMC Cell-Based Assay**

Unlike the HEK reporter cell lines, human peripheral blood mononuclear cell (PBMC) represents primary human immune cells in blood mainly consisting of lymphocytes, monocytes, and dendritic cells. These cells express TLR7, TLR8, or TLR9, and therefore are natural responders to respective ligand stimulation. Upon activation of these TLRs, PBMCs secrete similar cytokines and chemokines in vitro and in vivo, and therefore the in vitro potency of a TLR7/8/9 antagonist in human PBMC is readily translatable to its pharmacodynamics response in vivo.

Human peripheral blood mononuclear cells (PBMC) were isolated from freshly-drawn lithium-heparinized (Lithium Heparin Plus blood Collection tube, BD Vacutainer®) healthy donor whole blood by density gradient (Ficoll-PaqueTM PLUS, GE Healthcare life Sciences). Briefly, 50mL of blood was diluted with 25mL PBS (without $Ca^{2+}$, $Mg^{2+}$) in a 50mL conical tube with porous barrier (Leucosep tube, Greiner bio-one), where 15.5mL Ficoll-Paque was under laid after spinning. Tubes were centrifuged for 20 minutes at 800×g (1946 rpm) with the brake in the off position, and PBMC were collected from the buffy coat. Cells were then washed twice in PBS, and red blood cells were lysed by suspension in 2mL (Red Blood Cell Lysis Buffer, Alfa Aesar) for 5-10 minutes at room temperature. After a final wash in PBS, PBMC were resuspended at a final concentration of $2\times10^6$ cells/mL in RPMI-1640 media with GlutaMAXTM (Gibco) supplemented with 10% Fetal Bovine Serum (Sigma) and plated at 150μL/well ($3\times10^5$ cells/well) in tissue culture treated round bottom 96-well plates (Corning Incorporated). Antagonist compounds (compounds of this invention) solubilized and serial diluted in 100% DMSO were added in duplicate to cells to yield a final concentration of 1% DMSO (v/v). PBMC were incubated with antagonist compounds for 30 minutes at 37°C, 5% $CO_2$ before adding various TLR agonist reagents in 48 μL complete media per well as follows (final concentrations indicated): CpG ODN 2216 (InvivoGen) at 1μM for TLR9, ORN 06/LyoVec (InvivoGen) at 1μg/mL for TLR8 and R848 (InvivoGen) at 1μg/mL for TLR7 and TLR8. PBMC were incubated overnight at 37°C with 5% $CO_2$. Cell culture supernatants were collected, and levels of various human cytokines were assessed by Luminex assay (ProcartaPlexTM Multiplex Immunoassay, Invitrogen) or ELISA procedure according to the manufacturer's recommended protocol (eBioscience, ThermoFisher Scientific). Viability of the cells was also checked with Cell Viability Assay (CellTiter Glo®Luminescent Cell Viability Assay, Promega).

**Table 4. hPBMC results**

| Example No | hPBMC/TLR9 $IC_{50}$ (nM) |
|---|---|
| **2** | **470** |
| **6** | **617** |
| **7** | **420** |
| **14** | **340** |
| **21** | **848** |

(continued)

| Example No | hPBMC/TLR9 $IC_{50}$ (nM) |
|---|---|
| **25** | **320** |
| **26** | **330** |
| **27** | **380** |
| **28** | **520** |
| **29** | **710** |
| **30** | **780** |
| **31** | **410** |
| **32** | **320** |
| **33** | **146** |
| ***35*** | **870** |
| **40** | **540** |
| **43** | **470** |

**Example 47**

**Human microsome stability assay**

The human microsomal stability assay is used for early assessment of metabolic stability of a test compound in human liver microsomes.

Human liver microsomes (Cat.NO.: 452117, Corning, USA;Cat.NO.:H2610, Xenotech, USA) were preincubated with test compound for 10 minutes at 37°C in 100 mM potassium phosphate buffer, pH 7.4. The reactions were initiated by adding NADPH regenerating system. The final incubation mixtures contained 1 μM test compound, 0.5 mg/mL liver microsomal protein, 1 mM $MgCl_2$, 1 mM NADP, 1 unit/mL isocitric dehydrogenase and 6 mM isocitric acid in 100 mM potassium phosphate buffer, pH 7.4. After incubation times of 0, 3, 6, 9, 15 and 30 minutes at 37°C, 300 μL of cold acetonitrile (including internal standard) was added to 100 μL incubation mixture to terminate the reaction. Following precipitation and centrifugation, the amount of compound remaining in the samples were determined by LC-MS/MS. Controls of no NADPH regenerating system at zero and 30 minutes were also prepared and analyzed. The compounds of present invention showed good human liver microsome stability determined in the above assay, results are shown in Table 5 below.

**Table 5. Human liver microsome stability of the compounds of present invention**

| Example No | Clearance of Human microsome (mL/min/kg) |
|---|---|
| **N8** | **16.3** |
| **N79** | **18.2** |
| **2** | **8.2** |
| **3** | **9.3** |
| **5** | **9.1** |
| **6** | **9.3** |
| **7** | **6.2** |
| **12** | **9.7** |
| **13** | **7.2** |
| **14** | **6.7** |
| **19** | **6.2** |
| **20** | **9.0** |

(continued)

| Example No | Clearance of Human microsome (mL/min/kg) |
|---|---|
| **21** | **7.8** |
| **27** | **9.1** |
| **28** | **6.2** |
| **29** | **6.8** |
| **32** | **8.8** |
| **33** | **6.2** |
| **34** | **9.8** |
| **35** | **6.2** |
| **37** | **6.2** |
| **38** | **7.8** |
| **39** | **6.7** |
| **40** | **8.1** |
| **41** | **7.2** |
| **43** | **6.5** |

**Example 48**

**3T3 in vitro phototoxicity assay**

Phototoxicity is defined as a toxic response that is elicited after the first exposure of the skin to certain chemicals and subsequent exposure to light, or that is induced similarly by skin irradiation after systemic administration of a chemical substance. The assay used in this study is designed to detect the phototoxic potential of a chemical by using a simple in vitro cytotoxicity assay with Balb/c 3T3 mouse fibroblasts. The principle of this test is a comparison of the cytotoxicity of a chemical when tested with and without exposure to a non-toxic dose of UVA-light. Cytotoxicity is expressed as a dose dependent reduction of the growth rate of cells as determined by uptake of the vital dye Neutral Red one day after treatment.

**1. Method**

**Preparation of stock solution and dosage of test item**

A small amount of substance was weighed and formulated freshly in DMSO just before the start of the exposure of the cells. This stock solution or appropriate dilutions with DMSO were added to the cell suspensions to obtain the required final concentrations. All solutions were generally prepared in Eppendorf caps and discarded after use.

Reference substance

Chlorpromazine (HCL) (Sigma, Batch/Lot No.: 120M1328V) , test concentration: 300 μg/mL, Solvent: PBS / 3% DMSO

**Measurement of UV absorption spectrum**

The absorption spectra as such or with UV-A or with UV-B pre-irradiation were recorded between 240 nm and 400 nm with a Lambda-2 spectral photometer (Perkin Elmer).

| | | | |
|---|---|---|---|
| UV radiation sources: | for UV-A: Sol 500 with filter H1 | Main spectrum: | 315-690 nm |
| | | Irradiance: | approx. 1.67 $mW/cm^2$ |
| | | Radiation dose : | approx. 5 $J/cm^2$ |

(continued)

| | | |
|---|---|---|
| for UV-B: | Philips TL 20W/12 | |
| | Main spectrum: | 290-320 nm |
| | Irradiance: | approx. 0.083 $mW/cm^2$ |
| | Radiation dose: | approx. 0.05 $J/cm^2$ |

**Determination of phototoxicity**

For this study the Neutral Red uptake (NRU) assay of Borenfreund and Puerner (Borenfreund, E, Puerner JA. Toxicity determined in vitro by morphological alterations and Neutral Red absorption. Toxicology Lett. 1985; 24:119-124.) modified according to INVITTOX protocol No 78 (ERGATT/FRAME data bank of in vitro techniques in toxicology. INVITTOX PROTOCOL No 78. 3T3 NRU Phototoxicity Assay. March 1994) has been adapted to examine a possible phototoxic potential of the test item. This assay is based on the active uptake of the Neutral Red dye into the lysosomes of cultured murine fibroblasts. Because lysosomal membranes are known to be a site of action of many phototoxic compounds, this assay can provide a measure of potential for phototoxic injury.

**Preparation of cell culture**

A murine fibroblasts clone A 31 (ATCC no. CCL 163 - passage No. 108) were cultured in 175 $cm^2$ tissue culture grade flasks, containing sDMEM (Dulbecco's Minimal Essential Medium, supplemented with 10% fetal calf serum, 2 mM L-glutamine, 100 units/ml Penicillin and 100 μg/ml streptomycin) at 37°C in a humidified atmosphere of 6% $CO_2$. Before cells approach confluence they were removed from flasks by trypsinisation. Prior to use in an assay, the cells were transferred to 96-well microtiter plates at a concentration of 1x $10^4$ cells/well in 100 μl volumes of sDMEM and allowed to attach for 24 h.

**Exposure to test item**

For incubation with murine fibroblasts, the test item was diluted in PBS / 3% DMSO (detailed concentrations see in results).

Culture medium (Dulbecco's Modified Eagle Medium(DMEM), GlutaMAX (Gibco Ref 21885-025), 10% Fetal Bovine Serum (FBS) (Gibco Ref 10270-106), 100IU/ml Penicillin and 100 μg/ml Streptomycin (Gibco Ref 15140-122)) was removed from the wells and murine fibroblasts were washed with PBS. Afterwards 100 μL of PBS / 3% DMSO containing the test item was added and target cells were incubated for 1 h at 37°C with 6% $CO_2$.

**UV exposure**

For each test item the microtiter plates were prepared according to Table 6. "UVA plates" were exposed to approx. 5 $J/cm^2$ UVA light, the "Dark plates" were kept in the dark and served as cytotoxicity control. Plates with chlorpromazine hydrochloride served as positive control. UV flux was measured with a UV-meter (Dr. Gröbel RM21).

Following UV irradiation, the test item was removed from the wells (one washing step with PBS) and replaced with sDMEM. Target cells were then incubated overnight at 37°C in 6% $CO_2$.

**Table 6. 96-well microtiter plate setup**

| | 1 | 2 | 3 | 4 | 5 | 6 | 7 | 8 | 9 | 10 | 11 | 12 |
|---|---|---|---|---|---|---|---|---|---|---|---|---|
| A | S1 | S2 | | | | | | | | | S2 | S1 |
| B | S1 | S2 | | | | | | | | | S2 | S1 |
| C | S1 | S2 | | | | | | | | | S2 | S1 |
| D | S1 | S2 | U01 | U02 | U03 | U04 | U05 | U06 | U07 | U08 | S2 | S1 |
| E | S1 | S2 | | | | | | | | | S2 | S1 |
| F | S1 | S2 | | | | | | | | | S2 | S1 |
| G | S1 | S2 | | | | | | | | | S2 | S1 |

(continued)

| | 1 | 2 | 3 | 4 | 5 | 6 | 7 | 8 | 9 | 10 | 11 | 12 |
|---|---|---|---|---|---|---|---|---|---|---|---|---|
| **H** | S1 | S2 | | | | | | | | | S2 | S1 |
| 96-well microtiter plates were prepared as follows: | | | | | | | | | | | | |

Each plate contained wells with cells and solvent but without test item which were either not incubated with Neutral Red solution (0% standard - S1) or were stained with Neutral Red (100% standard -S2) for calculation of the standard cell viability curve. Wells labeled with U01-U08 contained the different test item concentrations.

**Neutral Red uptake**

The ready to use Neutral Red (NR) staining solution was freshly prepared as follows:

- 0.4% aqueous stock solution was shielded from light and filtered before use to remove NR crystals.
- 1:40 dilution of the stock solution was then prepared in sDMEM and added to the cells.

After the incubation the wells to be assayed were filled with 100 μL of the sDMEM containing Neutral Red. The target cells were incubated with the NR for 3 h at 37°C in 6% $CO_2$.

**Measurement of Neutral Red uptake**

Unincorporated Neutral Red was removed from the target cells and the wells washed with at least 100 μL of PBS. 150 μL of Neutral Red desorb solution (1% glacial acetic acid, 50% ethanol in aqua bidest) was then added to quantitatively extract the incorporated dye. After at least 10 mins of vigorous shaking of the plates on a microtiter plate shaker until Neutral Red has been extracted from the cells and formed a homogeneous solution, the absorption of the resulting colored solution was measured with a SPECTRAmax PLUS microtiter plate reader (Molecular Devices) at 540 nm.

**Calculation of cell viability**

Cell viability was calculated with the SOFTmax Pro software package (Molecular Devices). First a two-point standard curve (0% and 100% viability) was calculated with the linear curve fit option of the program based on the following formula:

$$Y = A + (B \times X)$$

(A = y-intercept of the line; B = slope of the line;
0% cell viability = cells with solvent, but without test item and Neutral Red;
100% cell viability = cells with solvent and Neutral Red, but without test item)

By this means the viability of the cells incubated with increasing concentrations of the test chemical was calculated. Chlorpromazine (HCl) served as positive control in the experiment.

**Calculation of $IC_{50}$ values**

All calculations were performed with the SOFTmax Pro analysis software package (Molecular Devices - for details see:
http://www.mbl.edu/jbpc/files/2014/05/SoftMax_Pro_User_Guide.pdf)

**Calculation of discrimination factor for phototoxicity**

For evaluation of phototoxic potential, the $IC_{50}$ values determined with and without UV exposure were compared.

$$\text{Factor} = IC_{50}\ (\text{-UV}) / IC_{50}\ (\text{+UV})$$

For discrimination between phototoxic and non-phototoxic test chemicals a cut-off factor of >5 was applied

(Liebsch M, Spielmann H, Balls M, Brand M, Döring B, Dupuis J, Holzhüter HG, Klecak G, L.Eplattenier H, Lovell W, Maurer T, Moldenhauer F, Moore L, Pape W, Pfannenbecker U, Potthast JM, De Silva O, Steiling W, Willshaw A. First results of the EC/COLIPA Validation Project. In Vitro Phototoxicity Testing. In: In Vitro Skin Toxicology: Irritation, Phototoxicity, Sensitization; Vol. 10. Alternative Methods in Toxicology,-Eds. Rougier A, Maibach HI, Goldberg AM; Mary Ann Liebert Publ.: New York, USA 1994, pp. 243-251).

Test items which are not cytotoxic to murine fibroblasts even at the highest concentrations tested, but show a strong dose dependent decrease in cell viability after UV exposure are considered also phototoxic (Spielmann H, Balls M, Dupuis J, Pape WJW, Pechovitch G, Silva DeO, Holzhütter, HG, Clothier R, Desolle P, Gerberick F, Liebsch M, Lowell WW, Maurer T, Pfannenbecker U, Potthast JM, Csato M, Sladowski D, Steiling W, Brantom P. The international EU/COLIPA in vitro phototoxicity validation study: Results of phase II (blind trial). Part 1: The 3T3 NRU phototoxicity test. Toxicology in Vitro 1998, 12: 305-327).

The test results were shown below, the compounds of this invention showed very good phototoxicity profile.

**Table 7. The 3T3 test results for the compound of this invention**

| Example No | Phototoxicity factor | $IC_{50}$ (UV-A) ($\mu$g/mL) |
|---|---|---|
| **28** | 1 | >120 |
| **40** | 1 | >120 |

**Example 49**

**Embryonic Stem Cell Test**

The in vitro mouse Embryonic Stem Cell Test (mEST) assay is an implemented routine assay at Roche. The original EST was developed by Horst Spielmann and his group in 1997 as an in vitro model for the screening of embryotoxicity, based on a blastocyst-derived permanent embryonic mouse ESC (mESC) D3 cell line derived from mouse 129 strains and was validated by European Centre for the Validation of Alternative Methods (ECVAM).

We further optimized and modified the approach allowing for the application of the assay to pharmaceutical compounds.

**Biological Endpoint and Endpoint Measurement:**

The cytotoxicity (inhibition of growth) of 3T3 fibroblasts, which represents differentiated cells and the cytotoxicity of undifferentiated embryonic stem cells (D3) after 10 days of substance treatment serve as two assay endpoints. This is determined by the use of dehydrogenase enzymes, which are present in the intact mitochondria of living cells to convert yellow soluble substrate 3-(4,5-dimethythiazol-2-yl)-2,5-diphenyl tetrazolium bromide (MTT) into a dark blue insoluble formazan product, which gets sequestered within the cells and is detected quantitatively using an absorbance reader (570nm) after solubilizing the cell membrane.

The third endpoint is the inhibition of differentiation of ES cells into myocards which are cardiac muscle cells after 10 days of treatment. The beating of this cells is evaluated by microscopy.

**Materials and Reagents**

mESC cell: ES-D3 [D3] (ATCC® CRL-1934™)
mouse Fibroblasts: BALB/3T3 clone A31 (ATCC® CCL-163™)
Balb/c 3T3 cell clone A31: American Type Culture Collection (ATCC) Cat No CCL-163
ES-D3 (D3): American Type Culture Collection (ATCC) Cat No CRL-1934
m-LIF: Sigma, Cat No L5158-5UG
NEAA (100x): Gibco, Cat No 11140-035
Trypan blue 0.04%: Gibco, Cat No T10282
MTT: Tocris Bioscience, Cat No 5224/500
5-Fluorouracil: Sigma, Cat No F-6627-5G
Penicillin/Streptomycin: Gibco, Cat No 15140-122
PBS (-$CaCl_2$/-$MgCl_2$): Gibco, Cat No 14190-094
FCS: Hyclone, Cat No SH30070.03

DMEM with Glucose, Glutamine, $NaHCO_3$: Gibco, Cat No 41966-029

**Method**

**PREPARATIONS**

**Media and Endpoint Assay Solutions**

Culture Media:

| 3T3 | D3 |
|---|---|
| 10% FCS | 20% FCS |
| 4 mM Glutamine | 2 mM Glutamine |
| 50 U/ml Penicillin | 50 U/ml Penicillin |
| 50 μg/ml Streptomycin | 50 μg/ml Streptomycin |
| | 1% NAA |
| | 0.1 mM β-ME |
| | 1000 U/ml m-LIF (only added separately to subculture) |

Assay Media:

| 3T3 | D3 |
|---|---|
| 10% FCS | 20% FCS |
| 4 mM Glutamine | 2 mM Glutamine |
| 50 U/ml Penicillin | 50 U/ml Penicillin |
| 50 μg/ml Streptomycin | 50 μg/ml Streptomycin |
| | 1% NAA |
| | 0.1 mM β-ME |

Media for Freezing Cells:

| 3T3 | D3 |
|---|---|
| 20% FCS | 40% FCS |
| 4 mM Glutamine | 2 mM Glutamine |
| 50 U/ml Penicillin | 50 U/ml Penicillin |
| 50 μg/ml Streptomycin | 50 μg/ml Streptomycin |
| | 1% NAA |
| | 0.1 mM β-ME |
| 7 % DMSO | 7 % DMSO |

β-Mercapthoethanol (β-ME) (10 mM)

17.5μL β-ME added to 25 mL of PBS
Store at 4°C for max. 1 week

FCS

Thaw FCS once up by water bath (37°C) and make aliquots of 100mL, 50 mL and 25mL.

Avoid multiple thawing
Store at -20°C

MTT-Solution

5mg MTT/ml PBS
Use a sterile Filter from Millipore and make aliquots of 8mL and 4 mL
Store at -20°C

MTT-Desorb-Solution

20% SDS solved in water/DMF, 1:1, adjust the pH to 4.5 with acetic acid

Test Compounds

Stock solution: 200 mM
Solvent: 100% DMSO

**DIFFERENTIATION ASSAY**

**DAY 0**

1) Cell passage with trypsin 0.05% EDTA for D3 cells.
2) Assembly of the cell suspension: dilute cells to $2.5 \times 10^4$ / mL with 18 ml media (for each test) in 50 mL Falcon tubes.
3) Prepare the petri dishes (PD): add 5-10 mL sterile Dulbecco's PBS (Gibco) into each dish bottom, distribute over the whole dish.
4) Dilution series of test compound in Eppendorf tubes: add 5 μL of compound (1:400 dilution) and 5μL control solution (DMSO) to 2 mL cell suspension, vortex.
5) Preparation of hanging drops in petridishes: vortex tube, aspirate suspension with automatic pipette and multi-dispense 20 μl drops onto the lid of the petri dish, add 2 ml in total in a concentric circle of drops (~ 100 drops); quickly but smoothly turn the cover and put on PD; incubate for 3 days at 37°C / 5% $CO_2$.

**DAY 3**

1) Dilution series of compound in 14 mL PP tubes

6 tubes for the concentrations; fill with 5 mL assay media

1 tube for DMSO (solvent control) fill with 5 mL assay media

2) Dilution series of compound in Eppendorf tubes
Add 12.5 μL of compound (1:400 dilution) and 12.5 μL control solution, vortex
3) Transfer of embryoid bodies in a bacterial Petri dish

Carefully turn PD lid, check the drops for fungus contamination
Rinse several times the drops down with 5 mL of the prepared solution

Transfer in a bacterial petri dish
Incubate for 3 days at 37 °C / 5% $CO_2$

**DAY 5**

1) Dilution series of compound in 50 mL tubes

6 tubes for the concentrations; fill with 25 mL assay media

1 tube for DMSO (solvent control) fill with 25 mL media

2) Dilution series of compound in 1.5 mL tubes
Add 62.5 μL of compound (1:400 dilution) and control solution (DMSO), vortex
3) Preparation of 96 well plates

2 plates for each compound, see Compound-plate-Layout
Add 220 μL media/compound/solvent mix in all 96 wells
Start with low concentration

4) Pipetting of embryoid bodies

Visually control the embryoid bodies in the petri dish
With a 25 μL tip, pipette one embryoid body in each well
Check visually the plate to ensure that at least one embryoid body is present in each well Incubate for 3 days at 37°C / 5% $CO_2$

**DAY 10**

Visualize each well with microscope for beating myocard cells
Assay media and DMSO controls should show at least 80% of beating cardiomyocyte cells (see acceptance criteria)

**CYTOTOXICITY ASSAY**

Stock solution with a concentration of 0.2 mol/L is created for all substances. Test substances are diluted in DMSO solution.

**DAY 0**

1) Create cell suspension for D3 and 3T3 cell lines
2) $2.5\times10^4$ cells/mL for 3T3, $1.5\times10^4$ cells/mL for D3 cells
3) Pipetting of 200 μL medium in the outer wells of a 96-well multi well plate (blanks)
4) Add 50 μL cell suspension into to the remaining inner wells of the 96-well multi well plate (samples)
5) Incubate for 2 h at 37°C/ 5% $CO_2$ to let the cell adhere
6) Pipetting of the test substances or DMSO controls Create concentrations of 2 mL medium and 6.67 μL test substance in a 5 mL tube
7) Add 150 μL/well of the solution into the sample wells (200 μL/well in total)
8) Incubate for 3 days at 37°C/ 5% $CO_2$

**DAY 3, 5 AND 7**

1) Dilute 2 mL of the medium (3T3 or D3 cell medium) with adding 5 μL of test substance (or DMSO control) (1:400) in a

mL tube
2) Remove the medium with a vacuum pump without damaging of the cell layer on the bottom
3) Add 200 μL of the diluted test substances (and DMSO controls) into to the appropriate sample wells

Incubation:

Day 3: 2 days at 37°C/ 5% $CO_2$
Day 5: 2 days at 37°C/ 5% $CO_2$
Day 7: 3 days at 37°C/ 5% $CO_2$

**DAY 10**

Preliminary observe cellular changes, substance precipitation or any other effects visually under the light microscope

MTT-Measurements:

1) Create final MTT solution by adding 4 mL MTT into 40 mL DMEM and warm up to 37°C
2) Remove medium out of the 96-well plates by discarding the medium carefully
3) Add 200 μL of MTT solution to each well with a multiwall pipet
4) Incubate the plates for 3h at 37°C/ 5% $CO_2$
5) Warm up the MTT-Desorb solution to 37°C
6) Remove the MTT-solution carefully
7) Add 130 μL of MTT-Desorb solution into each well and incubate the plates for 30 min at 37°C in the incubator, then put the plates for at least 2-3 hours on a plate shaker
8) Measure the absorption on a plate reader at 570 nm

**ACCEPTANCE CRITERIA**

Differentiation Endpoint: at least 80% of beating cardiomyocytes in a total assay needed for acceptance of a valid assay

Cytotoxicity Endpoints:

Acceptable ranges of DMSO control and POS control and the determination of OD values of D3 (about 1.8 - 2.2) and 3T3 (0.8 - 1.0) should be in their appropriate ranges

**Data Analysis**

Differentiation Endpoint:

Determination of the total number of beating cardiomyocytes (at least one beating cardiomyocyte per well = one positive count, no beating cardiomyocytes per well = negative count), normalization to the positive DMSO controls

Cytotoxicity Endpoints:

Determination of the mean values of the OD 570 of the blanks (value indicates the adhesion of the dye to plastic material and residual amount of medium). Subtract this value from sample values and continue to calculate with the corrected values.

Determination of the mean values of the OD 570 of the treated sample wells. Determination of the mean values of the OD 570 of the solvent control wells are set as 100%. The Viability is calculated in % normalized to the DMSO solvent control.

**Prediction Model**

Data files of optical densities (OD570) generated by a microplate reader were copied into an EXCEL spreadsheet. Mean OD values, standard deviations and viabilities were calculated automatically. The following endpoints from the assays could be calculated graphically from the concentration-response curve in the spreadsheet:

IC50 D3 - the concentration of test substance at which 50% of D3 cells have died
IC50 3T3 - the concentration of test substance at which 50% of the 3T3 cells have died.
ID50 D3 - the concentration of test substance at which there is a 50% reduction in the differentiation of D3 cells into contracting cardiomyocytes.

The IC50 values of the D3 and 3T3 cells from the cytotoxicity assay and the ID50 of the D3 differentiation assay were entered into the statistical evaluation developed from the modified prediction model used by Scholz et al. 1999a:

$$\mathrm{D12_3} = \frac{\lg\ IC50\ D3 + \lg IC50\ 3T3}{2} - \lg ID50$$

D12_3 < 0.5 means "negative"
D12_3 > 0.6 means "positive"

Predictive scores between 0.5 and 0.6 are labelled borderline results.

Inconclusive results are also possible, for example, if solubility limits the dose ranges tested to an extent that no IC50 or ID50 values can be determined for one or more dose response curves (Withlow et al. 2007)

**Table 8. mEST results for the compound of this invention**

| Example No | Predictive scores D12_3 |
|---|---|
| **28** | 0.44 |
| **40** | 0.36 |

**Example 50**

**Single dose pharmacokinetics (PK) study in Male Wister-Han Rats**

Pharmacokinetic properties of selected compounds were assessed by single dose PK studies in Male Wister-Han Rats (vendor: Beijing Vital River Laboratory Animal Technology Co., Ltd). Briefly, two groups of animals were administered a single dose of respective compound intravenously (IV, bolus) at 2 mg/kg or orally (PO, by gavage) at 10 mg/kg. Blood samples (approximately 150 μL) were collected via Jugular vein at 5 min (only for IV), 15 min, 30 min, 1 h, 2 h, 4 h, 7 h and 24 h post-dose. Blood samples were placed into tubes containing EDTA-K2 anticoagulant and centrifuged at 3000 rpm for 15 min at 4°C to separate plasma from the samples. After centrifugation, the resulting plasma was transferred to clean tubes for bioanalysis with LC/MS/MS. The pharmacokinetic parameters were calculated using non-compartmental analysis. The volume of distribution (Vss), half-life ($T_{1/2}$) and clearance (CL) were obtained based on the plasma concentration-time curve after IV dose. The peak concentration ($C_{max}$) was recorded directly from experimental observations after PO dose. The area under the plasma concentration-time curve ($AUC_{0\text{-}last}$) was calculated using the linear trapezoidal rule up to the last detectable concentration. The bioavailability (F) was calculated based on the dose normalized $AUC_{0\text{-}last}$ after IV and PO dose.

The Vss of a drug represents the degree to which a drug is distributed in body tissue rather than the plasma. Vss is directly proportional with the amount of drug distributed into tissue. A higher Vss indicates a greater amount of tissue distribution.

Results of PK parameters following IV and PO administration are given in Table 9.

**Table 9. PK parameters for the compounds of this invention**

| Example No | PO $C_{max}$ (ng/mL) | PO $AUC_{0\text{-}last}$ (h*ng/mL) | IV $AUC_{0\text{-}last}$ (h*ng/mL) | CL (mL/min/kg) | Vss (L/kg) | $T_{1/2}$ (h) | F (%) |
|---|---|---|---|---|---|---|---|
| **7** | 465 | 7514 | 1516 | 16 | 16.8 | 14.7 | 100 |

(continued)

| Example No | PO $C_{max}$ (ng/mL) | PO $AUC_{0\text{-last}}$ (h*ng/mL) | IV $AUC_{0\text{-last}}$ (h*ng/mL) | CL (mL/min/kg) | Vss (L/kg) | $T_{1/2}$ (h) | F (%) |
|---|---|---|---|---|---|---|---|
| **26** | 620 | 9260 | 2197 | 10.3 | 12.5 | 15.7 | 84.3 |
| **28** | 122 | 1800 | 974 | 30.2 | 16.7 | 8.84 | 37 |
| **40** | 216 | 2837 | 1062 | 32.8 | 13.2 | 7.26 | 53.4 |

## Claims

1. **1.** A compound of formula (I),

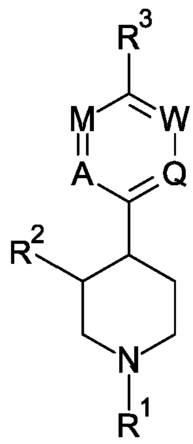

(I),

wherein

$R^1$ is

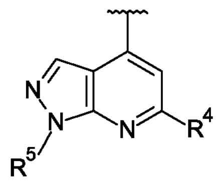

;

wherein $R^4$ is H or $C_{1\text{-}6}$alkyl; $R^5$ is $C_{1\text{-}6}$alkyl;
$R^2$ is H or $C_{1\text{-}6}$alkyl;
$R^3$ is piperazinyl, ($C_{1\text{-}6}$alkoxy$C_{1\text{-}6}$alkyl)piperazinyl, 3,4,4a,5,7,7a-hexahydro-2H-pyrrolo[3,4-b][1,4]oxazinyl, 4,7-diazaspiro[2.5]octanyl, 5-oxa-2,8-diazaspiro[3.5]nonanyl, amino-1,4-oxazepanyl, amino($C_{1\text{-}6}$alkoxy)piperidinyl, amino($C_{1\text{-}6}$alkoxy)pyrrolidinyl, amino($C_{1\text{-}6}$alkyl)azetidinyl or aminohalopiperidinyl;
A is N or $CR^6$; wherein $R^6$ is H, $C_{1\text{-}6}$alkyl or $C_{1\text{-}6}$alkoxy;
M is N or $CR^7$; wherein $R^7$ is H or $C_{1\text{-}6}$alkyl;
W is N or CH;
Q is N or CH;
with the condition that no more than two of A, M, W and Q are N simultaneously; or a pharmaceutically acceptable salt thereof.

2. **2.** A compound of formula (Ia), according to claim 1

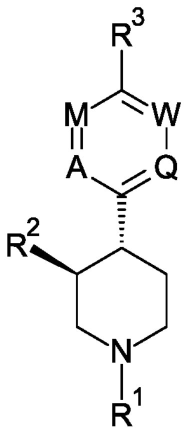

(Ia),

wherein

$R^1$ is

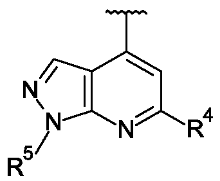

;

wherein $R^4$ is H or $C_{1-6}$alkyl; $R^5$ is $C_{1-6}$alkyl;
$R^2$ is H or $C_{1-6}$alkyl;
$R^3$ is piperazinyl, ($C_{1-6}$alkoxy$C_{1-6}$alkyl)piperazinyl, 3,4,4a,5,7,7a-hexahydro-2H-pyrrolo[3,4-b][1,4]oxazinyl, 4,7-diazaspiro[2.5]octanyl, 5-oxa-2,8-diazaspiro[3.5]nonanyl, amino-1,4-oxazepanyl, amino($C_{1-6}$alkoxy)piperidinyl, amino($C_{1-6}$alkoxy)pyrrolidinyl, amino($C_{1-6}$alkyl)azetidinyl or aminohalopiperidinyl;
A is N or $CR^6$; wherein $R^6$ is H, $C_{1-6}$alkyl or $C_{1-6}$alkoxy;
M is N or $CR^7$; wherein $R^7$ is H or $C_{1-6}$alkyl;
W is N or CH;
Q is N or CH;
with the condition that no more than two of A, M, W and Q are N simultaneously;
or a pharmaceutically acceptable salt thereof.

**3.** A compound according to claims 1 or 2, wherein

$R^1$ is

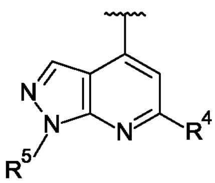

;

wherein $R^4$ is methyl; $R^5$ is methyl;
$R^2$ is H or methyl;
$R^3$ is 6-amino-1,4-oxazepan-4-yl, 3-amino-4-methoxy-pyrrolidin-1-yl or piperazin-1-yl;
A is $CR^6$; wherein $R^6$ is H or methyl;
M is $CR^7$; wherein $R^7$ is H;
W is CH;
Q is N;
or a pharmaceutically acceptable salt thereof.

**4.** A compound of formula (Ib), according to claim 1

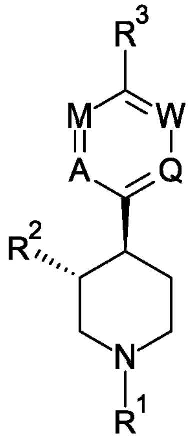

(Ib),

wherein

$R^1$ is

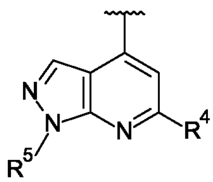

;

wherein $R^4$ is $C_{1-6}$alkyl; $R^5$ is $C_{1-6}$alkyl;
$R^2$ is $C_{1-6}$alkyl;
$R^3$ is piperazinyl, ($C_{1-6}$alkoxy$C_{1-6}$alkyl)piperazinyl, 4,7-diazaspiro[2.5]octanyl, 5-oxa-2,8-diazaspiro[3.5]nonanyl, amino-1,4-oxazepanyl, amino($C_{1-6}$alkoxy)piperidinyl, amino($C_{1-6}$alkoxy)pyrrolidinyl, amino($C_{1-6}$alkyl)azetidinyl or aminohalopiperidinyl;
A is CH;
M is CH;
W is CH;
Q is N;
or a pharmaceutically acceptable salt thereof.

**5.** A compound according to claim 4, wherein $R^4$ is methyl; $R^5$ is methyl; $R^2$ is methyl.

**6.** A compound according to any one of claims 4 to 5, wherein $R^3$ is 6-amino-1,4-oxazepan-4-yl.

**7.** A compound according to claim 4, wherein

$R^1$ is

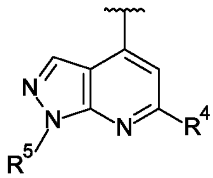

;

wherein $R^4$ is methyl; $R^5$ is methyl;
$R^2$ is methyl;
$R^3$ is 6-amino-1,4-oxazepan-4-yl;

A is CH;
M is CH;
W is CH;
Q is N;
or a pharmaceutically acceptable salt thereof.

**8.** A compound according to claim 1 selected from:

1,6-dimethyl-4-[4-(4-piperazin-1-ylphenyl)-1-piperidyl]pyrazolo[3,4-b]pyridine;
1,6-dimethyl-4-[4-(5-piperazin-1-yl-2-pyridyl)-1-piperidyl]pyrazolo[3,4-b]pyridine;
1,6-dimethyl-4-[4-(5-piperazin-1-ylpyrazin-2-yl)-1-piperidyl]pyrazolo[3,4-b]pyridine;
1,6-dimethyl-4-[4-(5-piperazin-1-ylpyrimidin-2-yl)-1-piperidyl]pyrazolo[3,4-b]pyridine;
1-methyl-4-[4-(3-methyl-5-piperazin-1-yl-2-pyridyl)-1-piperidyl]pyrazolo[3,4-b]pyridine;
1-methyl-4-[4-(4-methyl-6-piperazin-1-yl-3-pyridyl)-1-piperidyl]pyrazolo[3,4-b]pyridine;
1,6-dimethyl-4-[4-(3-methyl-5-piperazin-1-yl-2-pyridyl)-1-piperidyl]pyrazolo[3,4-b]pyridine;
1,6-dimethyl-4-[4-(4-methyl-6-piperazin-1-yl-3-pyridyl)-1-piperidyl]pyrazolo[3,4-b]pyridine;
1,6-dimethyl-4-[4-(4-methyl-5-piperazin-1-yl-2-pyridyl)-1-piperidyl]pyrazolo[3,4-b]pyridine;
1,6-dimethyl-4-[4-(2-methyl-6-piperazin-1-yl-3-pyridyl)-1-piperidyl]pyrazolo[3,4-b]pyridine;
1,6-dimethyl-4-[4-(4-methyl-2-piperazin-1-yl-pyrimidin-5-yl)-1-piperidyl]pyrazolo[3,4-b]pyridine;
1,6-dimethyl-4-[4-(3-methyl-5-piperazin-1-yl-pyrazin-2-yl)-1-piperidyl]pyrazolo[3,4-b]pyridine;
4-[4-(3-ethyl-5-piperazin-1-yl-2-pyridyl)-1-piperidyl]-1,6-dimethyl-pyrazolo[3,4-b]pyridine;
4-[4-(3-methoxy-5-piperazin-1-yl-2-pyridyl)-1-piperidyl]-1,6-dimethyl-pyrazolo[3,4-b]pyridine;
(3*S*,4*R*)-1-[6-[1-(1,6-dimethylpyrazolo[3,4-b]pyridin-4-yl)-4-piperidyl]-5-methyl-3-pyridyl]-3-fluoro-piperidin-4-amine;
4-[4-[5-[(3*R*)-3-(methoxymethyl)piperazin-1-yl]-3-methyl-2-pyridyl]-1-piperidyl]-1,6-dimethyl-pyrazolo[3,4-b]pyridine;
(3*S*,4*S*)-1-[6-[1-(1,6-dimethylpyrazolo[3,4-b]pyridin-4-yl)-4-piperidyl]-5-methyl-3-pyridyl]-3-methoxy-piperidin-4-amine;
1-methyl-4-[*cis*-3-methyl-4-(6-piperazin-1-yl-3-pyridyl)-1-piperidyl]pyrazolo[3,4-b]pyridine;
1-methyl-4-[*trans*-3-methyl-4-(6-piperazin-1-yl-3-pyridyl)-1-piperidyl]pyrazolo[3,4-b]pyridine;
1,6-dimethyl-4-[*cis*-3-methyl-4-(4-methyl-6-piperazin-1-yl-3-pyridyl)-1-piperidyl]pyrazolo[3,4-b]pyridine;
1,6-dimethyl-4-[*trans*-3-methyl-4-(4-methyl-6-piperazin-1-yl-3-pyridyl)-1-piperidyl]pyrazolo[3,4-b]pyridine;
1,6-dimethyl-4-[*cis*-3-methyl-4-(3-methyl-5-piperazin-1-yl-pyrazin-2-yl)-1-piperidyl]pyrazolo[3,4-b]pyridine;
1,6-dimethyl-4-[*trans*-3-methyl-4-(3-methyl-5-piperazin-1-yl-pyrazin-2-yl)-1-piperidyl]pyrazolo[3,4-b]pyridine;
1,6-dimethyl-4-[*cis*-3-methyl-4-(3-methyl-5-piperazin-1-yl-2-pyridyl)-1-piperidyl]pyrazolo[3,4-b]pyridine;
1,6-dimethyl-4-[*trans*-3-methyl-4-(3-methyl-5-piperazin-1-yl-2-pyridyl)-1-piperidyl]pyrazolo[3,4-b]pyridine;
(3*R*,4*R*)-1-[6-[(3*S*,4*R*)-1-(1,6-dimethylpyrazolo[3,4-b]pyridin-4-yl)-3-methyl-4-piperidyl]-5-methyl-3-pyridyl]-4-methoxy-pyrrolidin-3-amine;
2-[6-[(3*S*,4*R*)-1-(1,6-dimethylpyrazolo[3,4-b]pyridin-4-yl)-3-methyl-4-piperidyl]-5-methyl-3-pyridyl]-5-oxa-2,8-diazaspiro[3.5]nonane;
(6*S*)-4-[6-[(3*S*,4*R*)-1-(1,6-dimethylpyrazolo[3,4-b]pyridin-4-yl)-3-methyl-4-piperidyl]-5-methyl-3-pyridyl]-1,4-oxazepan-6-amine;
4-[(3*S*,4*R*)-4-[5-[(3*R*)-3-(methoxymethyl)piperazin-1-yl]-3-methyl-2-pyridyl]-3-methyl-1-piperidyl]-1,6-dimethyl-pyrazolo[3,4-b]pyridine;
4-[(3S,4R)-4-[5-(4,7-diazaspiro[2.5]octan-7-yl)-3-methyl-2-pyridyl]-3-methyl-1-piperidyl]-1,6-dimethyl-pyrazolo[3,4-b]pyridine;
(3*R*,4*R*)-1-[6-[(3*S*,4*R*)-1-(1,6-dimethylpyrazolo[3,4-b]pyridin-4-yl)-3-methyl-4-piperidyl]-5-methyl-3-pyridyl]-3-fluoro-piperidin-4-amine;
1-[6-[(3*S*,4*R*)-1-(1,6-dimethylpyrazolo[3,4-b]pyridin-4-yl)-3-methyl-4-piperidyl]-5-methyl-3-pyridyl]-3-methyl-azetidin-3-amine;
(4*aR*,7*aR*)-6-[6-[(3*S*,4*R*)-1-(1,6-dimethylpyrazolo[3,4-b]pyridin-4-yl)-3-methyl-4-piperidyl]-5-methyl-3-pyridyl]-3,4,4a,5,7,7a-hexahydro-2H-pyrrolo[3,4-b][1,4]oxazine;
1,6-dimethyl-4-[(3*R*,4*S*)-3-methyl-4-(5-piperazin-1-yl-2-pyridyl)-1-piperidyl]pyrazolo[3,4-b]pyridine;
1,6-dimethyl-4-[(3*S*,4*R*)-3-methyl-4-(5-piperazin-1-yl-2-pyridyl)-1-piperidyl]pyrazolo[3,4-b]pyridine;
(3*R*,4*R*)-1-[6-[(3*R*,4*S*)-1-(1,6-dimethylpyrazolo[3,4-b]pyridin-4-yl)-3-methyl-4-piperidyl]-3-pyridyl]-4-methoxy-pyrrolidin-3-amine;
4-[(3*R*,4*S*)-4-[5-[(3*R*)-3-(methoxymethyl)piperazin-1-yl]-2-pyridyl]-3-methyl-1-piperidyl]-1,6-dimethyl-pyrazolo[3,4-b]pyridine;

2-[6-[(3*R*,4*S*)-1-(1,6-dimethylpyrazolo[3,4-b]pyridin-4-yl)-3-methyl-4-piperidyl]-3-pyridyl]-5-oxa-2,8-diazaspiro[3.5]nonane;
(3*S*,4*S*)-1-[6-[(3*R*,4*S*)-1-(1,6-dimethylpyrazolo[3,4-b]pyridin-4-yl)-3-methyl-4-piperidyl]-3-pyridyl]-3-methoxy-piperidin-4-amine;
(6*S*)-4-[6-[(3*R*,4*S*)-1-(1,6-dimethylpyrazolo[3,4-b]pyridin-4-yl)-3-methyl-4-piperidyl]-3-pyridyl]-1,4-oxazepan-6-amine;
(3*R*,4*R*)-1-[6-[(3*R*,4*S*)-1-(1,6-dimethylpyrazolo[3,4-b]pyridin-4-yl)-3-methyl-4-piperidyl]-3-pyridyl]-3-fluoro-piperidin-4-amine;
4-[(3*R*,4*S*)-4-[5-(4,7-diazaspiro[2.5]octan-7-yl)-2-pyridyl]-3-methyl-1-piperidyl]-1,6-dimethyl-pyrazolo[3,4-b]pyridine; and
1-[6-[(3*R*,4*S*)-1-(1,6-dimethylpyrazolo[3,4-b]pyridin-4-yl)-3-methyl-4-piperidyl]-3-pyridyl]-3-methyl-azetidin-3-amine;
or a pharmaceutically acceptable salt thereof.

**9.** A process for the preparation of a compound according to any one of claims 1 to 8 comprising the following step:

a) the Buchwald-Hartwig C-N bond formation reaction between compound of formula (IV),

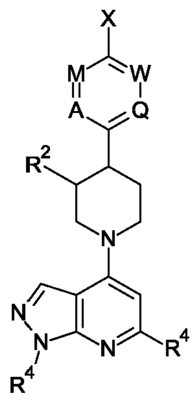

(IV),

and $R^3$-H , in the presence of a catalyst and a base;
b) the Buchwald-Hartwig C-N bond formation reaction between compound of formula (VI),

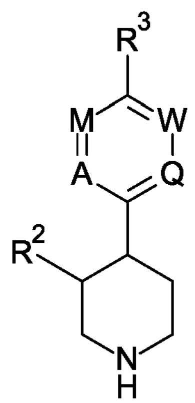

(VI),

and compound of formula (II),

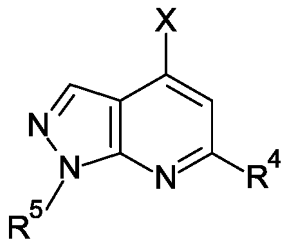

(II),

in the presence of a catalyst and a base;

c) the direct substation reaction between compound of formula (VI) and compound of formula (II) at in the presence of a base;

wherein

X is halogen;

in step a) and b), the catalyst is RuPhos Pd G2 or $Pd_2(dba)_3$/XantPhos; the base is $Cs_2CO_3$ or t-BuONa;

in step c), the base is DIPEA or CsF;

wherein $R^1$ to $R^5$, A, M, W and Q are defined as in any one of claims 1 to 7.

**10.** A compound or pharmaceutically acceptable salt according to any one of claims 1 to 8 for use as therapeutically active substance.

**11.** A pharmaceutical composition comprising a compound in accordance with any one of claims 1 to 8 and a therapeutically inert carrier.

**12.** A compound according to any one of claims 1 to 8 for use in the treatment or prophylaxis of systemic lupus erythematosus or lupus nephritis.

**13.** The use of a compound according to any one of claims 1 to 8 for the preparation of a medicament for the treatment or prophylaxis of systemic lupus erythematosus or lupus nephritis.

**14.** The use of a compound according to any one of claims 1 to 8 for the preparation of a medicament for TLR7 and TLR8 and TLR9 antagonist.

**15.** A compound or pharmaceutically acceptable salt according to any one of claims 1 to 8 for the treatment or prophylaxis of systemic lupus erythematosus or lupus nephritis.

**Patentansprüche**

**1.** Verbindung der Formel (I),

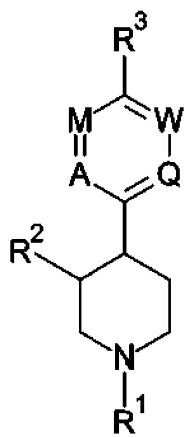

(I),

wobei

$R^1$

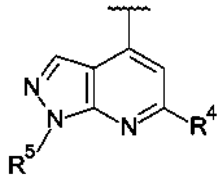

ist; wobei $R^4$ H oder $C_{1-6}$-Alkyl ist; $R^5$ $C_{1-6}$-Alkyl ist;
$R^2$ H oder $C_{1-6}$-Alkyl ist;
$R^3$ Piperazinyl, ($C_{1-6}$-Alkoxy-$C_{1-6}$-alkyl)piperazinyl, 3,4,4a,5,7,7a-Hexahydro-2H-pyrrolo[3,4-b][1,4]oxazinyl, 4,7-Diazaspiro[2.5]octanyl, 5-Oxa-2,8-diazaspiro[3.5]nonanyl, Amino-1,4-oxazepanyl, Amino($C_{1-6}$-alkoxy)piperidinyl, Amino($C_{1-6}$-alkoxy)pyrrolidinyl, Amino($C_{1-6}$-alkyl)azetidinyl oder Aminohalogenpiperidinyl ist;
A N oder $CR^6$ ist; wobei $R^6$ H, $C_{1-6}$-Alkyl oder $C_{1-6}$-Alkoxy ist;
M N oder $CR^7$ ist; wobei $R^7$ H oder $C_{1-6}$-Alkyl ist;
W N oder CH ist;
Q N oder CH ist;
mit der Maßgabe, dass nicht mehr als zwei von A, M, W und Q gleichzeitig N sind;
oder ein pharmazeutisch verträgliches Salz davon.

**2.** Verbindung der Formel (Ia) nach Anspruch 1

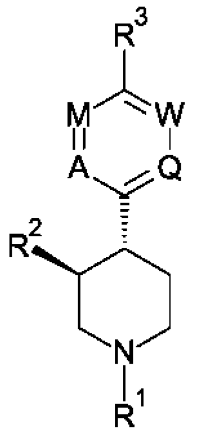

(Ia),

wobei

$R^1$

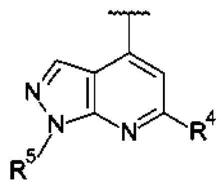

ist; wobei $R^4$ H oder $C_{1-6}$-Alkyl ist; $R^5$ $C_{1-6}$-Alkyl ist;
$R^2$ H oder $C_{1-6}$-Alkyl ist;
$R^3$ Piperazinyl, ($C_{1-6}$-Alkoxy-$C_{1-6}$-alkyl)piperazinyl, 3,4,4a,5,7,7a-Hexahydro-2H-pyrrolo[3,4-b][1,4]oxazinyl, 4,7-Diazaspiro[2.5]octanyl, 5-Oxa-2,8-diazaspiro[3.5]nonanyl, Amino-1,4-oxazepanyl, Amino($C_{1-6}$-alkoxy)piperidinyl, Amino($C_{1-6}$-alkoxy)pyrrolidinyl, Amino($C_{1-6}$-alkyl)azetidinyl oder Aminohalogenpiperidinyl ist;
A N oder $CR^6$ ist; wobei $R^6$ H, $C_{1-6}$-Alkyl oder $C_{1-6}$-Alkoxy ist;

M N oder $CR^7$ ist; wobei $R^7$ H oder $C_{1-6}$-Alkyl ist;
W N oder CH ist;
Q N oder CH ist;
mit der Maßgabe, dass nicht mehr als zwei von A, M, W und Q gleichzeitig N sind;
oder ein pharmazeutisch verträgliches Salz davon.

**3.** Verbindung nach Anspruch 1 oder 2, wobei

$R^1$

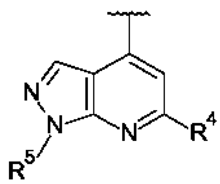

ist; wobei $R^4$ Methyl ist; $R^5$ Methyl ist;
$R^2$ H oder Methyl ist;
$R^3$ 6-Amino-1,4-oxazepan-4-yl, 3-Amino-4-methoxypyrrolidin-1-yl oder Piperazin-1-yl ist;
A $CR^6$ ist; wobei $R^6$ H oder Methyl ist;
M $CR^7$ ist; wobei $R^7$ H ist;
W CH ist;
Q N ist;
oder ein pharmazeutisch verträgliches Salz davon.

**4.** Verbindung der Formel (Ib) nach Anspruch 1

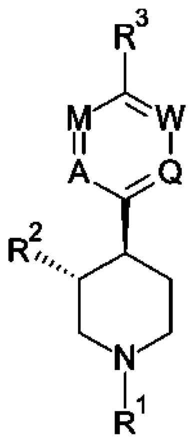

(Ib),

wobei

$R^1$

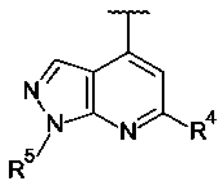

ist; wobei $R^4$ $C_{1-6}$-Alkyl ist; $R^5$ $C_{1-6}$-Alkyl ist;
$R^2$ $C_{1-6}$-Alkyl ist;
$R^3$ Piperazinyl, ($C_{1-6}$-Alkoxy-$C_{1-6}$-alkyl)piperazinyl, 4,7-Diazaspiro[2.5]octanyl, 5-Oxa-2,8-diazaspiro[3.5]nonanyl, Amino-1,4-oxazepanyl, Amino($C_{1-6}$-alkoxy)piperidinyl, Amino($C_{1-6}$-alkoxy)pyrrolidinyl, Amino($C_{1-6}$-alkyl)-azetidinyl oder Aminohalogenpiperidinyl ist;
A CH ist;
M CH ist;
W CH ist;
Q N ist;
oder ein pharmazeutisch verträgliches Salz davon.

**5.** Verbindung nach Anspruch 4, wobei $R^4$ Methyl ist; $R^5$ Methyl ist; $R^2$ Methyl ist.

**6.** Verbindung nach einem der Ansprüche 4 bis 5, wobei $R^3$ 6-Amino-1,4-oxazepan-4-yl ist.

**7.** Verbindung nach Anspruch 4, wobei

$R^1$

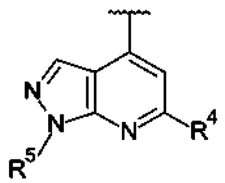

ist; wobei $R^4$ Methyl ist; $R^5$ Methyl ist;
$R^2$ Methyl ist;
$R^3$ 6-Amino-1,4-oxazepan-4-yl ist;
A CH ist;
M CH ist;
W CH ist;
Q N ist;
oder ein pharmazeutisch verträgliches Salz davon.

**8.** Verbindung nach Anspruch 1, ausgewählt aus:

1,6-Dimethyl-4-[4-(4-piperazin-1-ylphenyl)-1-piperidyl]pyrazolo[3,4-b]pyridin;
1,6-Dimethyl-4-[4-(5-piperazin-1-yl-2-pyridyl)-1-piperidyl]pyrazolo[3,4-b]pyridin;
1,6-Dimethyl-4-[4-(5-piperazin-1-ylpyrazin-2-yl)-1-piperidyl]pyrazolo[3,4-b]pyridin;
1,6-Dimethyl-4-[4-(5-piperazin-1-ylpyrimidin-2-yl)-1-piperidyl]pyrazolo[3,4-b]pyridin;
1-Methyl-4-[4-(3-methyl-5-piperazin-1-yl-2-pyridyl)-1-piperidyl]pyrazolo[3,4-b]pyridin;
1-Methyl-4-[4-(4-methyl-6-piperazin-1-yl-3-pyridyl)-1-piperidyl]pyrazolo[3,4-b]pyridin;
1,6-Dimethyl-4-[4-(3-methyl-5-piperazin-1-yl-2-pyridyl)-1-piperidyl]pyrazolo[3,4-b]pyridin;
1,6-Dimethyl-4-[4-(4-methyl-6-piperazin-1-yl-3-pyridyl)-1-piperidyl]pyrazolo[3,4-b]pyridin;
1,6-Dimethyl-4-[4-(4-methyl-5-piperazin-1-yl-2-pyridyl)-1-piperidyl]pyrazolo[3,4-b]pyridin;
1,6-Dimethyl-4-[4-(2-methyl-6-piperazin-1-yl-3-pyridyl)-1-piperidyl]pyrazolo[3,4-b]pyridin;
1,6-Dimethyl-4-[4-(4-methyl-2-piperazin-1-yl-pyrimidin-5-yl)-1-piperidyl]pyrazolo[3,4-b]pyridin;
1,6-Dimethyl-4-[4-(3-methyl-5-piperazin-1-yl-pyrazin-2-yl)-1-piperidyl]pyrazolo [3,4-b]pyridin;
4-[4-(3-Ethyl-5-piperazin-1-yl-2-pyridyl)-1-piperidyl]-1,6-dimethyl-pyrazolo [3,4-b]pyridin;
4-[4-(3-Methoxy-5-piperazin-1-yl-2-pyridyl)-1-piperidyl]-1,6-dimethyl-pyrazolo [3,4-b]pyridin;
(3*S*,4*R*)-1-[6-[1-(1,6-Dimethylpyrazolo[3,4-b]pyridin-4-yl)-4-piperidyl]-5-methyl-3-pyridyl]-3-fluor-piperidin-4-amin;
4-[4-[5-[(3*R*)-3-(Methoxymethyl)piperazin-1-yl]-3-methyl-2-pyridyl]-1-piperidyl]-1,6-dimethyl-pyrazolo[3,4-b]pyridin;
(3*S*,4*S*)-1-[6-[1-(1,6-Dimethylpyrazolo[3,4-b]pyridin-4-yl)-4-piperidyl]-5-methyl-3-pyridyl]-3-methoxy-piperidin-4-amin;
1-Methyl-4-[*cis*-3-methyl-4-(6-piperazin-1-yl-3-pyridyl)-1-piperidyl]pyrazolo[3,4-b]pyridin;
1-Methyl-4-[trans-3-methyl-4-(6-piperazin-1-yl-3-pyridyl)-1-piperidyl]pyrazolo[3,4-b]pyridin;
1,6-Dimethyl-4-[cis-3-methyl-4-(4-methyl-6-piperazin-1-yl-3-pyridyl)-1-piperidyl]pyrazolo[3,4-b]pyridin;
1,6-Dimethyl-4-[trans-3-methyl-4-(4-methyl-6-piperazin-1-yl-3-pyridyl)-1-piperidyl]pyrazolo[3,4-b]pyridin;

1,6-Dimethyl-4-[cis-3-methyl-4-(3-methyl-5-piperazin-1-yl-pyrazin-2-yl)-1-piperidyl]pyrazolo[3,4-b]pyridin;
1,6-Dimethyl-4-[trans-3-methyl-4-(3-methyl-5-piperazin-1-yl-pyrazin-2-yl)-1-piperidyl]pyrazolo[3,4-b]pyridin;
1,6-Dimethyl-4-[cis-3-methyl-4-(3-methyl-5-piperazin-1-yl-2-pyridyl)-1-piperidyl]-pyrazolo[3,4-b]pyridin;
1,6-Dimethyl-4-[trans-3-methyl-4-(3-methyl-5-piperazin-1-yl-2-pyridyl)-1-piperidyl]pyrazolo[3,4-b]pyridin;
(3*R*,4*R*)-1-[6-[(3*S*,4*R*)-1-(1,6-Dimethylpyrazolo[3,4-b]pyridin-4-yl)-3-methyl-4-piperidyl]-5-methyl-3-pyridyl]-4-methoxy-pyrrolidin-3-amin;
2-[6-[(3*S*,4*R*)-1-(1,6-Dimethylpyrazolo[3,4-b]pyridin-4-yl)-3-methyl-4-piperidyl]-5-methyl-3-pyridyl]-5-oxa-2,8-diazaspiro[3.5]nonan;
(6*S*)-4-[6-[(3*S*,4*R*)-1-(1,6-Dimethylpyrazolo[3,4-b]pyridin-4-yl)-3-methyl-4-piperidyl]-5-methyl-3-pyridyl]-1,4-oxazepan-6-amin;
4-[(3*S*,4*S*)-4-[5-[(3R)-3-(Methoxymethyl)piperazin-1-yl]-3-methyl-2-pyridyl]-3-methyl-1-piperidyl]-1,6-dimethyl-pyrazolo[3,4-b]pyridin;
4-[(3*S*,4*R*)-4-[5-(4,7-Diazaspiro[2.5]octan-7-yl)-3-methyl-2-pyridyl]-3-methyl-1-piperidyl]-1,6-dimethyl-pyrazolo[3,4-b]pyridin;
(3*R*,4*R*)-1-[6-[(3*S*,4*R*)-1-(1,6-Dimethylpyrazolo[3,4-b]pyridin-4-yl)-3-methyl-4-piperidyl]-5-methyl-3-pyridyl]-3-fluor-piperidin-4-amin;
1-[6-[(3*S*,4*R*)-1-(1,6-Dimethylpyrazolo[3,4-b]pyridin-4-yl)-3-methyl-4-piperidyl]-5-methyl-3-pyridyl]-3-methyl-azetidin-3-amin;
(4*aR*,7*aR*)-6-[6-[(3*S*,4*R*)-1-(1,6-Dimethylpyrazolo[3,4-b]pyridin-4-yl)-3-methyl-4-piperidyl]-5-methyl-3-pyridyl]-3,4,4a,5,7,7a-hexahydro-2H-pyrrolo[3,4-b][1,4]oxazin;
1,6-Dimethyl-4-[(3*R*,4*S*)-3-methyl-4-(5-piperazin-1-yl-2-pyridyl)-1-piperidyl]pyrazolo[3,4-b]pyridin;
1,6-Dimethyl-4-[(3S,4R)-3-methyl-4-(5-piperazin-1-yl-2-pyridyl)-1-piperidyl]pyrazolo[3,4-b]pyridin;
(3*R*,4*R*)-1-[6-[(3*R*,4*S*)-1-(1,6-Dimethylpyrazolo[3,4-b]pyridin-4-yl)-3-methyl-4-piperidyl]-3-pyridyl]-4-methoxy-pyrrolidin-3-amin;
4-[(3*R*,4*S*)-4-[5-[(3*R*)-3-(Methoxymethyl)piperazin-1-yl]-2-pyridyl]-3-methyl-1-piperidyl]-1,6-dimethyl-pyrazolo [3,4-b]pyridin;
2-[6-[(3*R*,4*S*)-1-(1,6-Dimethylpyrazolo[3,4-b]pyridin-4-yl)-3-methyl-4-piperidyl]-3-pyridyl]-5-oxa-2,8-diazaspiro [3.5]nonan;
(3*S*,4*S*)-1-[6-[(3*R*,4*S*)-1-(1,6-Dimethylpyrazolo[3,4-b]pyridin-4-yl)-3-methyl-4-piperidyl]-3-pyridyl]-3-methoxy-piperidin-4-amin;
(6*S*)-4-[6-[(3*R*,4*S*)-1-(1,6-Dimethylpyrazolo[3,4-b]pyridin-4-yl)-3-methyl-4-piperidyl]-3-pyridyl]-1,4-oxazepan-6-amin;
(3*R*,4*R*)-1-[6-[(3*R*,4*S*)-1-(1,6-Dimethylpyrazolo[3,4-b]pyridin-4-yl)-3-methyl-4-piperidyl]-3-pyridyl]-3-fluor-piperidin-4-amin;
4-[(3*R*,4*S*)-4-[5-(4,7-Diazaspiro[2.5]octan-7-yl)-2-pyridyl]-3-methyl-1-piperidyl]-1,6-dimethyl-pyrazolo[3,4-b] pyridin; und
1-[6-[(3*R*,4*S*)-1-(1,6-Dimethylpyrazolo[3,4-b]pyridin-4-yl)-3-methyl-4-piperidyl]-3-pyridyl]-3-methyl-azetidin-3-amin;
oder ein pharmazeutisch verträgliches Salz davon.

**9.** Verfahren zur Herstellung einer Verbindung nach einem der Ansprüche 1 bis 8, umfassend den folgenden Schritt:

a) die Buchwald-Hartwig-C-N-Bindungsbildungsreaktion zwischen einer Verbindung der Formel (IV),

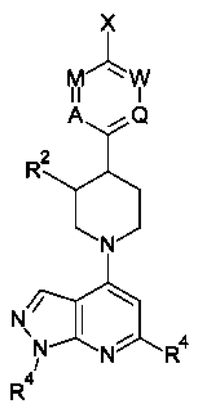

(IV),

und $R^3$-H, in Gegenwart eines Katalysators und einer Base;
b) die Buchwald-Hartwig-C-N-Bindungsbildungsreaktion zwischen einer Verbindung der Formel (VI),

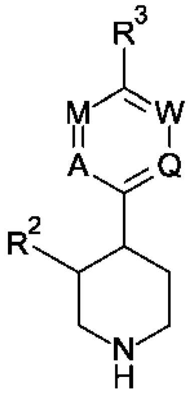

(VI),

und einer Verbindung der Formel (II),

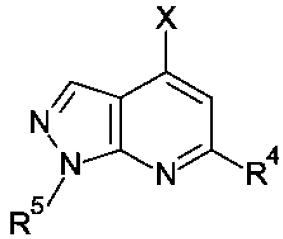

(II),

in Gegenwart eines Katalysators und einer Base;
c) die direkte Substitutionsreaktion zwischen einer Verbindung der Formel (VI) und einer Verbindung der Formel (II) in Gegenwart einer Base;

wobei

X Halogen ist;
in Schritt a) und b) der Katalysator RuPhos Pd G2 oder $Pd_2(dba)_3$/XantPhos ist; die Base $Cs_2CO_3$ oder t-BuONa ist;
in Schritt c) die Base DIPEA oder CsF ist;
wobei $R^1$ bis $R^5$, A, M, W und Q wie in einem der Ansprüche 1 bis 7 definiert sind.

**10.** Verbindung oder pharmazeutisch verträgliches Salz nach einem der Ansprüche 1 bis 8 zur Verwendung als therapeutisch wirksame Substanz.

**11.** Pharmazeutische Zusammensetzung, umfassend eine Verbindung nach einem der Ansprüche 1 bis 8 und einen therapeutisch inerten Träger.

**12.** Verbindung nach einem der Ansprüche 1 bis 8 zur Verwendung bei der Behandlung oder Prophylaxe von systemischem Lupus erythematodes oder Lupus-Nephritis.

**13.** Verwendung einer Verbindung nach einem der Ansprüche 1 bis 8 zur Herstellung eines Medikaments zur Behandlung oder Prophylaxe von systemischem Lupus erythematodes oder Lupus-Nephritis.

**14.** Verwendung einer Verbindung nach einem der Ansprüche 1 bis 8 zur Herstellung eines Medikaments für TLR7- und TLR8- und TLR9-Antagonisten.

**15.** Verbindung oder pharmazeutisch verträgliches Salz nach einem der Ansprüche 1 bis 8 zur Behandlung oder Prophylaxe von systemischem Lupus erythematodes oder Lupus-Nephritis.

## Revendications

1. Composé de formule (I),

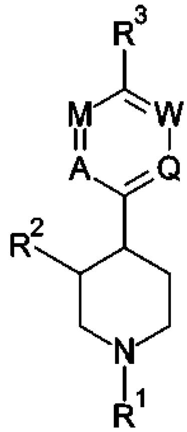

(I),

dans lequel

$R^1$ représente

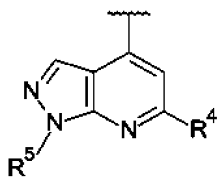

;

dans lequel $R^4$ représente H ou un alkyle en $C_{1-6}$ ; $R^5$ représente un alkyle en $C_{1-6}$ ;
$R^2$ représente H ou un alkyle en $C_{1-6}$ ;
$R^3$ représente un pipérazinyle, un (alcoxy en $C_{1-6}$-alkyle en $C_{1-6}$)pipérazinyle, un 3,4,4a,5,7,7a-hexahydro-2H-pyrrolo[3,4-b][1,4]oxazinyle, un 4,7-diazaspiro[2.5]octanyle, un 5-oxa-2,8-diazaspiro[3.5]nonanyle, un amino-1,4-oxazépanyle, un amino(alcoxy en $C_{1-6}$)pipéridinyle, un amino(alcoxy en $C_{1-6}$)pyrrolidinyle, un amino(alkyle en $C_{1-6}$)azétidinyle ou un aminohalogénopipéridinyle ;
A représente N ou $CR^6$ ; dans lequel $R^6$ représente H, un alkyle en $C_{1-6}$ ou un alcoxy en $C_{1-6}$ ;
M représente N ou $CR^7$ ; dans lequel $R^7$ représente H ou un alkyle en $C_{1-6}$ ;
W représente N ou CH ;
Q représente N ou CH ;
à condition que pas plus de deux parmi A, M, W et Q représentent N simultanément ;
ou un sel pharmaceutiquement acceptable de celui-ci.

2. Composé de formule (Ia), selon la revendication 1

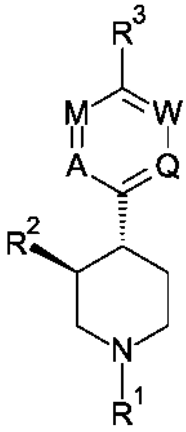

(Ia),

dans lequel

$R^1$ représente

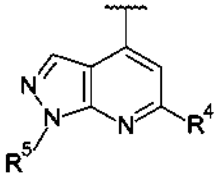

;

dans lequel $R^4$ représente H ou un alkyle en $C_{1-6}$ ; $R^5$ représente un alkyle en $C_{1-6}$ ;
$R^2$ représente H ou un alkyle en $C_{1-6}$ ;
$R^3$ représente un pipérazinyle, un (alcoxy en $C_{1-6}$-alkyle en $C_{1-6}$)pipérazinyle, un 3,4,4a,5,7,7a-hexahydro-2H-pyrrolo[3,4-b][1,4]oxazinyle, un 4,7-diazaspiro[2.5]octanyle, un 5-oxa-2,8-diazaspiro[3.5]nonanyle, un amino-1,4-oxazépanyle, un amino(alcoxy en $C_{1-6}$)pipéridinyle, un amino(alcoxy en $C_{1-6}$)pyrrolidinyle, un amino(alkyle en $C_{1-6}$)azétidinyle ou un aminohalogénopipéridinyle ;
A représente N ou $CR^6$ ; dans lequel $R^6$ représente H, un alkyle en $C_{1-6}$ ou un alcoxy en $C_{1-6}$ ;
M représente N ou $CR^7$ ; dans lequel $R^7$ représente H ou un alkyle en $C_{1-6}$ ;
W représente N ou CH ;
Q représente N ou CH ;
à condition que pas plus de deux parmi A, M, W et Q représentent N simultanément ;
ou un sel pharmaceutiquement acceptable de celui-ci.

**3.** Composé selon les revendications 1 ou 2, dans lequel

$R^1$ représente

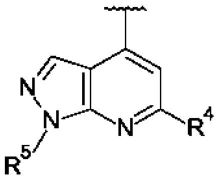

;

dans lequel $R^4$ représente un méthyle ; $R^5$ représente un méthyle ;
$R^2$ représente H ou un méthyle ;
$R^3$ représente un 6-amino-1,4-oxazépan-4-yle, un 3-amino-4-méthoxy-pyrrolidin-1-yle ou un pipérazin-1-yle ;
A représente $CR^6$ ; dans lequel $R^6$ représente H ou un méthyle ;
M représente $CR^7$ ; dans lequel $R^7$ représente H ;
W représente CH ;

Q représente N ;
ou un sel pharmaceutiquement acceptable de celui-ci.

**4.** Composé de formule (Ib), selon la revendication 1

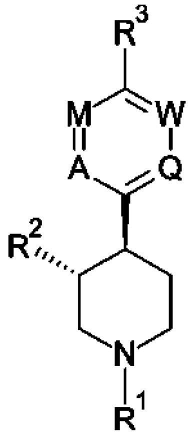

(Ib),

dans lequel

$R^1$ représente

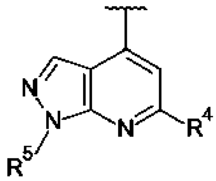

;

dans lequel $R^4$ représente un alkyle en $C_{1-6}$; $R^5$ représente un alkyle en $C_{1-6}$ ;
$R^2$ représente un alkyle en $C_{1-6}$ ;
$R^3$ représente un pipérazinyle, un (alcoxy en $C_{1-6}$-alkyle en $C_{1-6}$)pipérazinyle, un 4,7-diazaspiro[2.5]octanyle, un 5-oxa-2,8-diazaspiro[3.5]nonanyle, un amino-1,4-oxazépanyle, un amino(alcoxy en $C_{1-6}$)pipéridinyle, un amino(alcoxy en $C_{1-6}$)pyrrolidinyle, un amino(alkyle en $C_{1-6}$)azétidinyle ou un aminohalogénopipéridinyle ;
A représente CH ;
M représente CH ;
W représente CH ;
Q représente N ;
ou un sel pharmaceutiquement acceptable de celui-ci.

**5.** Composé selon la revendication 4, dans lequel $R^4$ représente un méthyle ; $R^5$ représente un méthyle ; $R^2$ représente un méthyle.

**6.** Composé selon l'une quelconque des revendications 4 à 5, dans lequel $R^3$ représente un 6-amino-1,4-oxazépan-4-yle.

**7.** Composé selon la revendication 4, dans lequel

$R^1$ représente

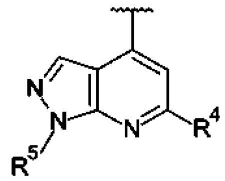

;

dans lequel $R^4$ représente un méthyle ; $R^5$ représente un méthyle ;
$R^2$ représente un méthyle ;
$R^3$ représente un 6-amino-1,4-oxazépan-4-yle ;
A représente CH ;
M représente CH ;
W représente CH ;
Q représente N ;
ou un sel pharmaceutiquement acceptable de celui-ci.

**8.** Composé selon la revendication 1 choisi parmi :

la 1,6-diméthyl-4-[4-(4-pipérazin-1-ylphényl)-1-pipéridyl]pyrazolo[3,4-b]pyridine ;
la 1,6-diméthyl-4-[4-(5-pipérazin-1-yl-2-pyridyl)-1-pipéridyl]pyrazolo[3,4-b]pyridine ;
la 1,6-diméthyl-4-[4-(5-pipérazin-1-ylpyrazin-2-yl)-1-pipéridyl]pyrazolo[3,4-b]pyridine ;
la 1,6-diméthyl-4-[4-(5-pipérazin-1-ylpyrimidin-2-yl)-1-pipéridyl]pyrazolo[3,4-b]pyridine ;
la 1-méthyl-4-[4-(3-méthyl-5-pipérazin-1-yl-2-pyridyl)-1-pipéridyl]pyrazolo[3,4-b]pyridine ;
la 1-méthyl-4-[4-(4-méthyl-6-pipérazin-1-yl-3-pyridyl)-1-pipéridyl]pyrazolo[3,4-b]pyridine ;
la 1,6-diméthyl-4-[4-(3-méthyl-5-pipérazin-1-yl-2-pyridyl)-1-pipéridyl]pyrazolo[3,4-b]pyridine ;
la 1,6-diméthyl-4-[4-(4-méthyl-6-pipérazin-1-yl-3-pyridyl)-1-pipéridyl]pyrazolo[3,4-b]pyridine ;
la 1,6-diméthyl-4-[4-(4-méthyl-5-pipérazin-1-yl-2-pyridyl)-1-pipéridyl]pyrazolo[3,4-b]pyridine ;
la 1,6-diméthyl-4-[4-(2-méthyl-6-pipérazin-1-yl-3-pyridyl)-1-pipéridyl]pyrazolo[3,4-b]pyridine ;
la 1,6-diméthyl-4-[4-(4-méthyl-2-pipérazin-1-yl-pyrimidin-5-yl)-1-pipéridyl]pyrazolo[3,4-b]pyridine ;
la 1,6-diméthyl-4-[4-(3-méthyl-5-pipérazin-1-yl-pyrazin-2-yl)-1-pipéridyl]pyrazolo[3,4-b]pyridine ;
la 4-[4-(3-éthyl-5-pipérazin-1-yl-2-pyridyl)-1-pipéridyl]-1,6-diméthyl-pyrazolo[3,4-b]pyridine ;
la 4-[4-(3-méthoxy-5-pipérazin-1-yl-2-pyridyl)-1-pipéridyl]-1,6-diméthyl-pyrazolo[3,4-b]pyridine ;
la (3*S*,4*R*)-1-[6-[1-(1,6-diméthylpyrazolo[3,4-b]pyridin-4-yl)-4-pipéridyl]-5-méthyl-3-pyridyl]-3-fluoro-pipéridin-4-amine ;
la 4-[4-[5-[(3*R*)-3-(méthoxyméthyl)pipérazin-1-yl]-3-méthyl-2-pyridyl]-1-pipéridyl]-1,6-diméthyl-pyrazolo[3,4-b]pyridine ;
la (3*S*,4*S*)-1-[6-[1-(1,6-diméthylpyrazolo[3,4-b]pyridin-4-yl)-4-pipéridyl]-5-méthyl-3-pyridyl]-3-méthoxy-pipéridin-4-amine ;
la 1-méthyl-4-[*cis*-3-méthyl-4-(6-pipérazin-1-yl-3-pyridyl)-1-pipéridyl]pyrazolo[3,4-b]pyridine ;
la 1-méthyl-4-[*trans*-3-méthyl-4-(6-pipérazin-1-yl-3-pyridyl)-1-pipéridyl]pyrazolo[3,4-b]pyridine ;
la 1,6-diméthyl-4-[*cis*-3-méthyl-4-(4-méthyl-6-pipérazin-1-yl-3-pyridyl)-1-pipéridyl]pyrazolo[3,4-b]pyridine ;
la 1,6-diméthyl-4-[*trans*-3-méthyl-4-(4-méthyl-6-pipérazin-1-yl-3-pyridyl)-1-pipéridyl]pyrazolo[3,4-b]pyridine ;
la 1,6-diméthyl-4-[*cis*-3-méthyl-4-(3-méthyl-5-pipérazin-1-yl-pyrazin-2-yl)-1-pipéridyl]pyrazolo[3,4-b]pyridine ;
la 1,6-diméthyl-4-[*trans*-3-méthyl-4-(3-méthyl-5-pipérazin-1-yl-pyrazin-2-yl)-1-pipéridyl]pyrazolo[3,4-b]pyridine ;
la 1,6-diméthyl-4-[*cis*-3-méthyl-4-(3-méthyl-5-pipérazin-1-yl-2-pyridyl)-1-pipéridyl]pyrazolo[3,4-b]pyridine ;
la 1,6-diméthyl-4-[*trans*-3-méthyl-4-(3-méthyl-5-pipérazin-1-yl-2-pyridyl)-1-pipéridyl]pyrazolo[3,4-b]pyridine ;
la (3*R*,4*R*)-1-[6-[(3*S*,4*R*)-1-(1,6-diméthylpyrazolo[3,4-b]pyridin-4-yl)-3-méthyl-4-pipéridyl]-5-méthyl-3-pyridyl]-4-méthoxy-pyrrolidin-3-amine ;
le 2-[6-[(3*S*,4*R*)-1-(1,6-diméthylpyrazolo[3,4-b]pyridin-4-yl)-3-méthyl-4-pipéridyl]-5-méthyl-3-pyridyl]-5-oxa-2,8-diazaspiro[3.5]nonane ;
la (6*S*)-4-[6-[(3*S*,4*R*)-1-(1,6-diméthylpyrazolo[3,4-b]pyridin-4-yl)-3-méthyl-4-pipéridyl]-5-méthyl-3-pyridyl]-1,4-oxazépan-6-amine ;
la 4-[(3*S*,4*S*)-4-[5-[(3*R*)-3-(méthoxyméthyl)pipérazin-1-yl]-3-méthyl-2-pyridyl]-3-méthyl-1-pipéridyl]-1,6-diméthyl-pyrazolo[3,4-b]pyridine ;
la 4-[(3*S*,4*R*)-4-[5-(4,7-diazaspiro[2.5]octan-7-yl)-3-méthyl-2-pyridyl]-3-méthyl-1-pipéridyl]-1,6-diméthyl-pyrazolo[3,4-b]pyridine ;

la (3*R*,4*R*)-1-[6-[(3*S*,4*R*)-1-(1,6-diméthylpyrazolo[3,4-b]pyridin-4-yl)-3-méthyl-4-pipéridyl]-5-méthyl-3-pyridyl]-3-fluoro-pipéridin-4-amine ;

la 1-[6-[(3*S*,4*R*)-1-(1,6-diméthylpyrazolo[3,4-b]pyridin-4-yl)-3-méthyl-4-pipéridyl]-5-méthyl-3-pyridyl]-3-méthyl-azétidin-3-amine ;

la (4*aR*,7*aR*)-6-[6-[(3*S*,4*R*)-1-(1,6-diméthylpyrazolo[3,4-b]pyridin-4-yl)-3-méthyl-4-pipéridyl]-5-méthyl-3-pyridyl]-3,4,4a,5,7,7a-hexahydro-2H-pyrrolo[3,4-b][1,4]oxazine ;

la 1,6-diméthyl-4-[(3*R*,4*S*)-3-méthyl-4-(5-pipérazin-1-yl-2-pyridyl)-1-pipéridyl]pyrazolo[3,4-b]pyridine ;

la 1,6-diméthyl-4-[(3*S*,4*R*)-3-méthyl-4-(5-pipérazin-1-yl-2-pyridyl)-1-pipéridyl]pyrazolo[3,4-b]pyridine ;

la (3*R*,4*R*)-1-[6-[(3*R*,4*S*)-1-(1,6-diméthylpyrazolo[3,4-b]pyridin-4-yl)-3-méthyl-4-pipéridyl]-3-pyridyl]-4-méthoxy-pyrrolidin-3-amine ;

la 4-[(3*R*,4*S*)-4-[5-[(3*R*)-3-(méthoxyméthyl)pipérazin-1-yl]-2-pyridyl]-3-méthyl-1-pipéridyl]-1,6-diméthyl-pyrazolo[3,4-b]pyridine ;

le 2-[6-[(3*R*,4*S*)-1-(1,6-diméthylpyrazolo[3,4-b]pyridin-4-yl)-3-méthyl-4-pipéridyl]-3-pyridyl]-5-oxa-2,8-diazaspiro[3.5]nonane ;

la (3*S*,4*S*)-1-[6-[(3*R*,4*S*)-1-(1,6-diméthylpyrazolo[3,4-b]pyridin-4-yl)-3-méthyl-4-pipéridyl]-3-pyridyl]-3-méthoxy-pipéridin-4-amine ;

la (6*S*)-4-[6-[(3*R*,4*S*)-1-(1,6-diméthylpyrazolo[3,4-b]pyridin-4-yl)-3-méthyl-4-pipéridyl]-3-pyridyl]-1,4-oxazépan-6-amine ;

la (3*R*,4*R*)-1-[6-[(3*R*,4*S*)-1-(1,6-diméthylpyrazolo[3,4-b]pyridin-4-yl)-3-méthyl-4-pipéridyl]-3-pyridyl]-3-fluoro-pipéridin-4-amine ;

la 4-[(3*R*,4*S*)-4-[5-(4,7-diazaspiro[2.5]octan-7-yl)-2-pyridyl]-3-méthyl-1-pipéridyl]-1,6-diméthyl-pyrazolo[3,4-b]pyridine ; et

la 1-[6-[(3*R*,4*S*)-1-(1,6-diméthylpyrazolo[3,4-b]pyridin-4-yl)-3-méthyl-4-pipéridyl]-3-pyridyl]-3-méthyl-azétidin-3-amine ;

ou un sel pharmaceutiquement acceptable de ceux-ci.

**9.** Procédé de préparation d'un composé selon l'une quelconque des revendications 1 à 8 comprenant l'étape suivante :

a) la réaction de formation de liaison C-N de Buchwald-Hartwig entre un composé de formule (IV),

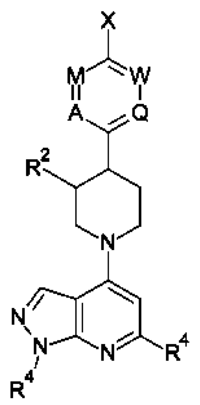

(IV),

et $R^3$-H, en présence d'un catalyseur et d'une base ;

b) la réaction de formation de liaison C-N de Buchwald-Hartwig entre un composé de formule (VI),

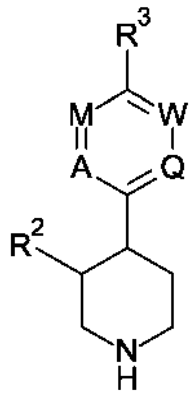

(VI),

et un composé de formule (II),

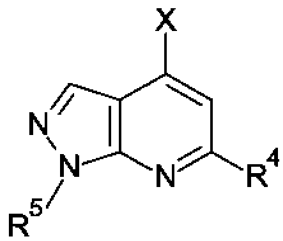

(II),

en présence d'un catalyseur et d'une base ;
c) la réaction de substitution directe entre un composé de formule (VI) et un composé de formule (II) en présence d'une base ;

dans lequel

X représente un halogène ;
dans les étapes a) et b), le catalyseur est RuPhos Pd G2 ou $Pd_2(dba)_3$/XantPhos ; la base est $Cs_2CO_3$ ou t-BuONa ;
dans l'étape c), la base est DIPEA ou CsF ;
dans lequel $R^1$ à $R^5$, A, M, W et Q sont tels que définis dans l'une quelconque des revendications 1 à 7.

**10.** Composé ou sel pharmaceutiquement acceptable selon l'une quelconque des revendications 1 à 8 pour une utilisation comme substance thérapeutiquement active.

**11.** Composition pharmaceutique comprenant un composé selon l'une quelconque des revendications 1 à 8 et un véhicule thérapeutiquement inerte.

**12.** Composé selon l'une quelconque des revendications 1 à 8 pour une utilisation dans le traitement ou la prophylaxie du lupus érythémateux disséminé ou de la néphropathie lupique.

**13.** Utilisation d'un composé selon l'une quelconque des revendications 1 à 8 pour la préparation d'un médicament pour le traitement ou la prophylaxie du lupus érythémateux disséminé ou de la néphropathie lupique.

**14.** Utilisation d'un composé selon l'une quelconque des revendications 1 à 8 pour la préparation d'un médicament antagoniste de TLR7 et TLR8 et TLR9.

**15.** Composé ou sel pharmaceutiquement acceptable selon l'une quelconque des revendications 1 à 8 pour le traitement ou la prophylaxie du lupus érythémateux disséminé ou de la néphropathie lupique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018047081 A
- WO 2019220390 A


### Non-patent literature cited in the description


- **NAVARRA, S. V et al.** *Lancet*, 2011, vol. 377, 721
- **KRIEG, A. M et al.** *Immunol. Rev*, 2007, vol. 220, 251
- **JIMÉNEZ-DALMARONI, M. J. et al.** *Autoimmun Rev*, 2016, vol. 15, 1
- **CHEN, J. Q. et al.** *Clinical Reviews in Allergy & Immunology*, 2016, vol. 50, 1
- **ANSEL, HOWARD C et al.** Ansel's Pharmaceutical Dosage Forms and Drug Delivery Systems. Lippincott, Williams & Wilkins, 2004
- **GENNARO, ALFONSO R et al.** Remington: The Science and Practice of Pharmacy. Lippincott, Williams & Wilkins, 2000
- **ROWE, RAYMOND C**. Handbook of Pharmaceutical Excipients. Pharmaceutical Press, 2005
- *Acc. Chem. Res*, 1998, vol. 31, 805-818
- *Chem. Rev.*, 2016, vol. 116, 12564-12649
- *Topics in Current Chemistry*, 2002, vol. 219, 131-209
- *CHEMICAL ABSTRACTS*, 578-66-5
- *CHEMICAL ABSTRACTS*, 88495-54-9
- *CHEMICAL ABSTRACTS*, 244221-57-6
- *CHEMICAL ABSTRACTS*, 19867-78-8
- *CHEMICAL ABSTRACTS*, 181269-69-2
- *CHEMICAL ABSTRACTS*, 65550-77-8
- *CHEMICAL ABSTRACTS*, 286961-15-7
- *CHEMICAL ABSTRACTS*, 352437-09-3
- *CHEMICAL ABSTRACTS*, 40473-01-6
- *CHEMICAL ABSTRACTS*, 19745-07-4
- *CHEMICAL ABSTRACTS*, 183438-24-6
- *CHEMICAL ABSTRACTS*, 1268520-92-8
- *CHEMICAL ABSTRACTS*, 944582-92-7
- *CHEMICAL ABSTRACTS*, 1783407-55-5
- *CHEMICAL ABSTRACTS*, 778611-64-6
- *CHEMICAL ABSTRACTS*, 375368-83-5
- *CHEMICAL ABSTRACTS*, 633328-95-7
- *CHEMICAL ABSTRACTS*, 107378-41-6
- *CHEMICAL ABSTRACTS*, 137628-17-2
- *CHEMICAL ABSTRACTS*, 286947-03-3
- *CHEMICAL ABSTRACTS*, 1434126-99-4
- *CHEMICAL ABSTRACTS*, 1023301-73-6
- *CHEMICAL ABSTRACTS*, 907544-19-8
- *CHEMICAL ABSTRACTS*, 4629-78-1
- *CHEMICAL ABSTRACTS*, 1932066-52-8
- *CHEMICAL ABSTRACTS*, 1251005-61-4
- *CHEMICAL ABSTRACTS*, 2306247-11-8
- *CHEMICAL ABSTRACTS*, 674792-08-6
- *CHEMICAL ABSTRACTS*, 1523530-29-1
- *CHEMICAL ABSTRACTS*, 1408076-37-8
- *CHEMICAL ABSTRACTS*, 1932337-68-2
- **REDFERN WS** ; **CARLSSON L** ; **DAVIS AS** ; **LYNCH WG** ; **MACKENZIE I** ; **PALETHORPE S** ; **SIEGL PK** ; **STRANG I** ; **SULLIVAN AT** ; **WALLIS R**. Relationships between preclinical cardiac electrophysiology, clinical QT interval prolongation and torsade de pointes for a broad range of drugs: evidence for a provisional safety margin in drug development. *Cardiovasc. Res*, 2003, vol. 58, 32-45
- **SANGUINETTI MC** ; **TRISTANI-FIROUZI M**. hERG potassium channels and cardiac arrhythmia. *Nature*, 2006, vol. 440, 463-469
- **WEBSTER R** ; **LEISHMAN D** ; **WALKER D**. Towards a drug concentration effect relationship for QT prolongation and torsades de pointes. *Curr. Opin. Drug Discov. Devel*, 2002, vol. 5, 116-26
- **BORENFREUND, E** ; **PUERNER JA**. Toxicity determined in vitro by morphological alterations and Neutral Red absorption. *Toxicology Lett*, 1985, vol. 24, 119-124

- First results of the EC/COLIPA Validation Project. In Vitro Phototoxicity Testing. In: In Vitro Skin Toxicology: Irritation, Phototoxicity, Sensitization. **LIEBSCH M** ; **SPIELMANN H** ; **BALLS M** ; **BRAND M** ; **DÖRING B** ; **DUPUIS J** ; **HOLZHÜTER HG** ; **KLECAK G** ; **L.EPLATTENIER H** ; **LOVELL W**. Alternative Methods in Toxicology. Mary Ann Liebert Publ, 1994, vol. 10, 243-251
- **SPIELMANN H** ; **BALLS M** ; **DUPUIS J** ; **PAPE WJW** ; **PECHOVITCH G** ; **SILVA DEO** ; **HOLZHÜTTER, HG** ; **CLOTHIER R** ; **DESOLLE P** ; **GERBERICK F**. The international EU/COLIPA in vitro phototoxicity validation study: Results of phase II (blind trial). Part 1: The 3T3 NRU phototoxicity test. *Toxicology in Vitro*, 1998, vol. 12, 305-327